# EUROPEAN PATENT SPECIFICATION

(11) **EP 4 203 868 B1**
(45) Date of publication and mention of the grant of the patent: **25.03.2026**
(21) Application number: 21862661.2
(22) Date of filing: 25.08.2021
(51) Int. Cl.: A61F 5/41

(54) **METHODS OF SELECTING A PENILE CONSTRICTION DEVICE AND MEASUREMENT DEVICE**
VERFAHREN ZUR AUSWAHL EINER VORRICHTUNG ZUR PENISVERENGUNG UND EINER MESSVORRICHTUNG
MÉTHODES DE SÉLECTION D'UN DISPOSITIF DE CONSTRICTION PÉNIENNE ET D'UN DISPOSITIF DE MESURE

(30) Priority: 26.08.2020 US 202063070724 P
(43) Date of publication of application: 05.07.2023
(73) Proprietor: Giddy Holdings, Inc., Austin, TX 78702 (US)
(72) Inventor: JACOBSON, Brett, Austin, Texas 78702 (US)
(74) Representative: Mewburn Ellis LLP
(86) International application number: PCT/US2021/047556
(87) International publication number: WO 2022/046911

(56) References cited:
- US-A- 1 225 341
- US-A- 5 125 890
- US-A- 5 234 402
- US-A1- 2003 083 598
- US-A1- 2005 277 907
- US-A1- 2017 224 357
- US-B1- 6 319 194
- US-B2- 9 962 281

## Description

### BACKGROUND OF THE INVENTION

Erectile dysfunction (ED), also known as impotence, is a type of sexual dysfunction characterized by the inability to develop or maintain an erection of the penis sufficient for sexual activity. Erectile dysfunction may have psychological consequences as it can be tied to relationship difficulties and self-image.

Some studies regarding erectile dysfunction have reported an average prevalence of 52%, which tends to increase with age. Some studies have further shown that approximately 40% and 70% of 40-year-old men and 70-year-old men are affected, respectively, whereby age is the variable most strongly associated with ED. ED has been further shown to be caused by or increase with many different physiological and psychological health conditions and diseases. US5234402A proposes an apparatus and method for augmenting male potency cinctures a vacuum induced engorged condition of a male sex organ while also protecting abdominal and scrotal tissue during application of vacuum force to the user.

### SUMMARY OF THE INVENTION

The invention is defined by the appended set of claims. Document US 5 234 402 A is regarded as the closest prior art and discloses the use of colors for the purpose of identifying to a user a given penile constriction device having specific materials characteristics.

The present invention relates to devices and methods related to alleviating erectile dysfunction and is defined in the appended claims.

In the present disclosure 1 inch is equivalent to 2.54 cm.

Erection is generally enabled in men through, in part, increased blood flow into the penis through arteries on the ventral side of the penis and decreased blood flow out from the penis through the veins on the dorsal side of the penis. Currently available ED devices attempt to alleviate ED in one of two ways - each with their own deficiencies.

The first type of ED device is a rigid, circular ring (e.g., made of metal, wood, glass, etc.). As the penis is shaped like an oval (wider than it is tall) and these devices are based on a circle, trying to fit an oval in a circle results in additional pressure being put on the sides in an effort to achieve minimal pressure on the top. This additional pressure on the side of the penis creates a distortion of the penis and an encumbrance of the arteries and corpora cavernosa. This works against the natural physiology of an erection. This pressure from rigid devices may be detrimental to the health of the tissue, arteries, and veins in the penis. Additionally, this pressure may hinder the ability of the user to employ Kegel exercises or other activities to their maximum effectiveness to increase inward blood flow and maintain the erection. Further, this uniform pressure may also limit natural inward blood flow into the penis, limiting the erection. Moreover, these rigid ED devices are less effective on a flaccid penis or an individual with severe ED, making these devices not ideal for initiating an erection. The second type of ED device is typically soft and malleable. However, these softer ED devices are ineffective in providing the appropriate constriction to either initiate or maintain a sufficient erection. These devices typically apply uniform pressure around the circumference of the penis to slow the outwards blood flow from the penis. However, the uniform pressure from these devices may be detrimental to the health of the tissue, arteries, and veins in the penis. Additionally, this uniform pressure may hinder the ability for the user to employ Kegel exercises or other activities to their maximum effectiveness to increase inward blood flow and maintain the erection. Further, this uniform pressure may also limit natural inward blood flow into the penis, limiting the erection. Further, vacuum pumps, pills, injections, and surgeries, are costly, often ineffective and induce adverse side effects.

In addition, currently available ED device configurations do not account for the particular shape (e.g., the devices are shaped like a perfect circle) or the specific anatomical regions and characteristics therein (e.g., location, size, and function of the superficial and deep dorsal veins, dorsal arteries, dorsal nerves, cavernous arteries, urethra, and corpus spongiosum) of the penis, which is not shaped like a circle. Nor do currently available ED devices provide adequate features on the exterior of the device to directly stimulate the partner's sexual experience, which may have negative psychological impact on the user and exacerbate any ED issues. Finally, none of the currently available ED devices offer instructions or guidance on selecting the properly sized device for the specific user.

Provided herein are ED methods, devices, and systems configured to more effectively prevent outwards blood flow from the penis to the body. Further provided herein are methods, devices, and systems that more effectively promote inward blood flow into the penis. Further provided herein are methods, devices, and systems that aid initiation and maintenance of an erection. Further provided herein are methods, devices, and systems that more directly and effectively stimulate the partner's sexual experience. Further provided herein are methods, devices, and systems that are configured to reduce physical ED symptoms and/or eliminate ED exacerbating performance stress. Further provided herein are methods, devices, and systems that enable improved family planning and/or sperm donation.

A first aspect provided herein are penile constriction devices comprising an arched rod comprising: a first arm comprising a first coupling end and a first converging end having a first terminus; a second arm comprising a second coupling end and a second converging end having a second terminus disconnected from the first terminus, wherein the first converging end and the second converging end converge to form a constriction region; and a bridge connecting the first coupling end to the second coupling end, the bridge comprising a compression region rising inferiorly from an inferior side of the bridge.

Optionally, in this or any other embodiment, the device has a constriction gap width, wherein the constriction gap width is measured as a minimum distance between the first converging end and the second converging end within the constriction region. Optionally, in this or any other embodiment, the device has a constriction gap width of about 0 inches to about 1.25 inches. Optionally, in this or any other embodiment, the device has a constriction gap width of at least about 0 inches. Optionally, in this or any other embodiment, the device has a constriction gap width of at most about 1.25 inches. Optionally, in this or any other embodiment, the device has a constriction gap width of about 0 inches to about 0.125 inches, about 0 inches to about 0.375 inches, about 0 inches to about 0.5 inches, about 0 inches to about 0.625 inches, about 0 inches to about 0.75 inches, about 0 inches to about 0.875 inches, about 0 inches to about 1 inch, about 0 inches to about 1.125 inches, about 0 inches to about 1.25 inches, about 0.125 inches to about 0.375 inches, about 0.125 inches to about 0.5 inches, about 0.125 inches to about 0.625 inches, about 0.125 inches to about 0.75 inches, about 0.125 inches to about 0.875 inches, about 0.125 inches to about 1 inch, about 0.125 inches to about 1.125 inches, about 0.125 inches to about 1.25 inches, about 0.375 inches to about 0.5 inches, about 0.375 inches to about 0.625 inches, about 0.375 inches to about 0.75 inches, about 0.375 inches to about 0.875 inches, about 0.375 inches to about 1 inch, about 0.375 inches to about 1.125 inches, about 0.375 inches to about 1.25 inches, about 0.5 inches to about 0.625 inches, about 0.5 inches to about 0.75 inches, about 0.5 inches to about 0.875 inches, about 0.5 inches to about 1 inch, about 0.5 inches to about 1.125 inches, about 0.5 inches to about 1.25 inches, about 0.625 inches to about 0.75 inches, about 0.625 inches to about 0.875 inches, about 0.625 inches to about 1 inch, about 0.625 inches to about 1.125 inches, about 0.625 inches to about 1.25 inches, about 0.75 inches to about 0.875 inches, about 0.75 inches to about 1 inch, about 0.75 inches to about 1.125 inches, about 0.75 inches to about 1.25 inches, about 0.875 inches to about 1 inch, about 0.875 inches to about 1.125 inches, about 0.875 inches to about 1.25 inches, about 1 inch to about 1.125 inches, about 1 inch to about 1.25 inches, or about 1.125 inches to about 1.25 inches. Optionally, in this or any other embodiment, the device has a constriction gap width of about 0 inches, about 0.125 inches, about 0.375 inches, about 0.5 inches, about 0.625 inches, about 0.75 inches, about 0.875 inches, about 1 inch, about 1.125 inches, or about 1.25 inches. Optionally, in this or any other embodiment, the device has a constriction gap width of at least about 0 inches, about 0.125 inches, about 0.375 inches, about 0.5 inches, about 0.625 inches, about 0.75 inches, about 0.875 inches, about 1 inch, about 1.125 inches, or about 1.25 inches. Optionally, in this or any other embodiment, the device has a constriction gap width of at most about 0.125 inches, about 0.375 inches, about 0.5 inches, about 0.625 inches, about 0.75 inches, about 0.875 inches, about 1 inch, about 1.125 inches, or about 1.25 inches.

Optionally, in this or any other embodiment, the device has a constriction height, wherein the constriction height is measured as a normal distance between a constriction point and the inferior side of the bridge, and wherein the constriction point is defined as the midpoint of the constriction gap width. Optionally, in this or any other embodiment, the device has a constriction height of about 0.5 inches to about 2.5 inches. Optionally, in this or any other embodiment, the device has a constriction height of at least about 0.5 inches. Optionally, in this or any other embodiment, the device has a constriction height of at most about 2.5 inches. Optionally, in this or any other embodiment, the device has a constriction height of about 0.5 inches to about 0.75 inches, about 0.5 inches to about 1 inch, about 0.5 inches to about 1.25 inches, about 0.5 inches to about 1.5 inches, about 0.5 inches to about 1.75 inches, about 0.5 inches to about 2 inches, about 0.5 inches to about 2.25 inches, about 0.5 inches to about 2.5 inches, about 0.75 inches to about 1 inch, about 0.75 inches to about 1.25 inches, about 0.75 inches to about 1.5 inches, about 0.75 inches to about 1.75 inches, about 0.75 inches to about 2 inches, about 0.75 inches to about 2.25 inches, about 0.75 inches to about 2.5 inches, about 1 inch to about 1.25 inches, about 1 inch to about 1.5 inches, about 1 inch to about 1.75 inches, about 1 inch to about 2 inches, about 1 inch to about 2.25 inches, about 1 inch to about 2.5 inches, about 1.25 inches to about 1.5 inches, about 1.25 inches to about 1.75 inches, about 1.25 inches to about 2 inches, about 1.25 inches to about 2.25 inches, about 1.25 inches to about 2.5 inches, about 1.5 inches to about 1.75 inches, about 1.5 inches to about 2 inches, about 1.5 inches to about 2.25 inches, about 1.5 inches to about 2.5 inches, about 1.75 inches to about 2 inches, about 1.75 inches to about 2.25 inches, about 1.75 inches to about 2.5 inches, about 2 inches to about 2.25 inches, about 2 inches to about 2.5 inches, or about 2.25 inches to about 2.5 inches. Optionally, in this or any other embodiment, the device has a constriction height of about 0.5 inches, about 0.75 inches, about 1 inch, about 1.25 inches, about 1.5 inches, about 1.75 inches, about 2 inches, about 2.25 inches, or about 2.5 inches. Optionally, in this or any other embodiment, the device has a constriction height of at least about 0.5 inches, about 0.75 inches, about 1 inch, about 1.25 inches, about 1.5 inches, about 1.75 inches, about 2 inches, about 2.25 inches, or about 2.5 inches. Optionally, in this or any other embodiment, the device has a constriction height of at most about 0.5 inches, about 0.75 inches, about 1 inch, about 1.25 inches, about 1.5 inches, about 1.75 inches, about 2 inches, about 2.25 inches, or about 2.5 inches.

Optionally, in this or any other embodiment, the device has a maximum inner width, wherein the maximum inner width is measured as a maximum normal distance between a mesial vertex of the surface of the first arm and a mesial vertex of the surface of the second arm, and wherein the maximum inner width is superior to the constriction gap. Optionally, in this or any other embodiment, the device has a maximum inner width of about 0.5 inches to about 3 inches. Optionally, in this or any other embodiment, the device has a maximum inner width of at least about 0.5 inches. Optionally, in this or any other embodiment, the device has a maximum inner width of at most about 3 inches. Optionally, in this or any other embodiment, the device has a maximum inner width of about 0.5 inches to about 0.75 inches, about 0.5 inches to about 1 inch, about 0.5 inches to about 1.25 inches, about 0.5 inches to about 1.5 inches, about 0.5 inches to about 1.75 inches, about 0.5 inches to about 2 inches, about 0.5 inches to about 2.25 inches, about 0.5 inches to about 2.5 inches, about 0.5 inches to about 2.75 inches, about 0.5 inches to about 3 inches, about 0.75 inches to about 1 inch, about 0.75 inches to about 1.25 inches, about 0.75 inches to about 1.5 inches, about 0.75 inches to about 1.75 inches, about 0.75 inches to about 2 inches, about 0.75 inches to about 2.25 inches, about 0.75 inches to about 2.5 inches, about 0.75 inches to about 2.75 inches, about 0.75 inches to about 3 inches, about 1 inch to about 1.25 inches, about 1 inch to about 1.5 inches, about 1 inch to about 1.75 inches, about 1 inch to about 2 inches, about 1 inch to about 2.25 inches, about 1 inch to about 2.5 inches, about 1 inch to about 2.75 inches, about 1 inch to about 3 inches, about 1.25 inches to about 1.5 inches, about 1.25 inches to about 1.75 inches, about 1.25 inches to about 2 inches, about 1.25 inches to about 2.25 inches, about 1.25 inches to about 2.5 inches, about 1.25 inches to about 2.75 inches, about 1.25 inches to about 3 inches, about 1.5 inches to about 1.75 inches, about 1.5 inches to about 2 inches, about 1.5 inches to about 2.25 inches, about 1.5 inches to about 2.5 inches, about 1.5 inches to about 2.75 inches, about 1.5 inches to about 3 inches, about 1.75 inches to about 2 inches, about 1.75 inches to about 2.25 inches, about 1.75 inches to about 2.5 inches, about 1.75 inches to about 2.75 inches, about 1.75 inches to about 3 inches, about 2 inches to about 2.25 inches, about 2 inches to about 2.5 inches, about 2 inches to about 2.75 inches, about 2 inches to about 3 inches, about 2.25 inches to about 2.5 inches, about 2.25 inches to about 2.75 inches, about 2.25 inches to about 3 inches, about 2.5 inches to about 2.75 inches, about 2.5 inches to about 3 inches, or about 2.75 inches to about 3 inches. Optionally, in this or any other embodiment, the device has a maximum inner width of about 0.5 inches, about 0.75 inches, about 1 inch, about 1.25 inches, about 1.5 inches, about 1.75 inches, about 2 inches, about 2.25 inches, about 2.5 inches, about 2.75 inches, or about 3 inches. Optionally, in this or any other embodiment, the device has a maximum inner width of at least about 0.5 inches, about 0.75 inches, about 1 inch, about 1.25 inches, about 1.5 inches, about 1.75 inches, about 2 inches, about 2.25 inches, about 2.5 inches, about 2.75 inches, or about 3 inches. Optionally, in this or any other embodiment, the device has a maximum inner width of at most about 0.5 inches, about 0.75 inches, about 1 inch, about 1.25 inches, about 1.5 inches, about 1.75 inches, about 2 inches, about 2.25 inches, about 2.5 inches, about 2.75 inches, or about 3 inches.

Optionally, in this or any other embodiment, the device has an inferior height, wherein the inferior height is measured as a normal distance between a center point, and the inferior side of the bridge, wherein the center point comprises the midpoint of the maximum inner width. Optionally, in this or any other embodiment, the device has an inferior height of about 0.25 inches to about 1 inch. Optionally, in this or any other embodiment, the device has an inferior height of at least about 0.25 inches. Optionally, in this or any other embodiment, the device has an inferior height of at most about 1 inch. Optionally, in this or any other embodiment, the device has an inferior height of about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.6 inches, about 0.25 inches to about 0.7 inches, about 0.25 inches to about 0.8 inches, about 0.25 inches to about 0.9 inches, about 0.25 inches to about 1 inch, about 0.3 inches to about 0.35 inches, about 0.3 inches to about 0.4 inches, about 0.3 inches to about 0.45 inches, about 0.3 inches to about 0.5 inches, about 0.3 inches to about 0.6 inches, about 0.3 inches to about 0.7 inches, about 0.3 inches to about 0.8 inches, about 0.3 inches to about 0.9 inches, about 0.3 inches to about 1 inch, about 0.35 inches to about 0.4 inches, about 0.35 inches to about 0.45 inches, about 0.35 inches to about 0.5 inches, about 0.35 inches to about 0.6 inches, about 0.35 inches to about 0.7 inches, about 0.35 inches to about 0.8 inches, about 0.35 inches to about 0.9 inches, about 0.35 inches to about 1 inch, about 0.4 inches to about 0.45 inches, about 0.4 inches to about 0.5 inches, about 0.4 inches to about 0.6 inches, about 0.4 inches to about 0.7 inches, about 0.4 inches to about 0.8 inches, about 0.4 inches to about 0.9 inches, about 0.4 inches to about 1 inch, about 0.45 inches to about 0.5 inches, about 0.45 inches to about 0.6 inches, about 0.45 inches to about 0.7 inches, about 0.45 inches to about 0.8 inches, about 0.45 inches to about 0.9 inches, about 0.45 inches to about 1 inch, about 0.5 inches to about 0.6 inches, about 0.5 inches to about 0.7 inches, about 0.5 inches to about 0.8 inches, about 0.5 inches to about 0.9 inches, about 0.5 inches to about 1 inch, about 0.6 inches to about 0.7 inches, about 0.6 inches to about 0.8 inches, about 0.6 inches to about 0.9 inches, about 0.6 inches to about 1 inch, about 0.7 inches to about 0.8 inches, about 0.7 inches to about 0.9 inches, about 0.7 inches to about 1 inch, about 0.8 inches to about 0.9 inches, about 0.8 inches to about 1 inch, or about 0.9 inches to about 1 inch. Optionally, in this or any other embodiment, the device has an inferior height of about 0.25 inches, about 0.3 inches, about 0.35 inches, about 0.4 inches, about 0.45 inches, about 0.5 inches, about 0.6 inches, about 0.7 inches, about 0.8 inches, about 0.9 inches, or about 1 inch. Optionally, in this or any other embodiment, the device has an inferior height of at least about 0.25 inches, about 0.3 inches, about 0.35 inches, about 0.4 inches, about 0.45 inches, about 0.5 inches, about 0.6 inches, about 0.7 inches, about 0.8 inches, about 0.9 inches, or about 1 inch. Optionally, in this or any other embodiment, the device has an inferior height of at most about 0.25 inches, about 0.3 inches, about 0.35 inches, about 0.4 inches, about 0.45 inches, about 0.5 inches, about 0.6 inches, about 0.7 inches, about 0.8 inches, about 0.9 inches, or about 1 inch.

Optionally, in this or any other embodiment, a ratio between the constriction gap width and the maximum inner width is about 0.3:1 to about 0.9:1. Optionally, in this or any other embodiment, a ratio between the constriction gap width and the maximum inner width is at least about 0.2:1. Optionally, in this or any other embodiment, a ratio between the constriction gap width and the maximum inner width is at most about 0.9:1. Optionally, in this or any other embodiment, a ratio between the constriction gap width and the maximum inner width is about 0.2:1 to about 0.25:1, about 0.2:1 to about 0.3:1, about 0.2:1 to about 0.35:1, about 0.2:1 to about 0.4:1, about 0.2:1 to about 0.5:1, about 0.2:1 to about 0.6:1, about 0.2:1 to about 0.7:1, about 0.2:1 to about 0.8:1, about 0.2:1 to about 0.9:1, about 0.25:1 to about 0.3:1, about 0.25:1 to about 0.35:1, about 0.25:1 to about 0.4:1, about 0.25:1 to about 0.5:1, about 0.25:1 to about 0.6:1, about 0.25:1 to about 0.7:1, about 0.25:1 to about 0.8:1, about 0.25:1 to about 0.9:1, about 0.3:1 to about 0.35:1, about 0.3:1 to about 0.4:1, about 0.3:1 to about 0.5:1, about 0.3:1 to about 0.6:1, about 0.3:1 to about 0.7:1, about 0.3:1 to about 0.8:1, about 0.3:1 to about 0.9:1, about 0.35:1 to about 0.4:1, about 0.35:1 to about 0.5:1, about 0.35:1 to about 0.6:1, about 0.35:1 to about 0.7:1, about 0.35:1 to about 0.8:1, about 0.35:1 to about 0.9:1, about 0.4:1 to about 0.5:1, about 0.4:1 to about 0.6:1, about 0.4:1 to about 0.7:1, about 0.4:1 to about 0.8:1, about 0.4:1 to about 0.9:1, about 0.5:1 to about 0.6:1, about 0.5:1 to about 0.7:1, about 0.5:1 to about 0.8:1, about 0.5:1 to about 0.9:1, about 0.6:1 to about 0.7:1, about 0.6:1 to about 0.8:1, about 0.6:1 to about 0.9:1, about 0.7:1 to about 0.8:1, about 0.7:1 to about 0.9:1, or about 0.8:1 to about 0.9:1. Optionally, in this or any other embodiment, a ratio between the constriction gap width and the maximum inner width is about 0.2:1, about 0.25:1, about 0.3:1, about 0.35:1, about 0.4:1, about 0.5:1, about 0.6:1, about 0.7:1, about 0.8:1, or about 0.9:1. Optionally, in this or any other embodiment, a ratio between the constriction gap width and the maximum inner width is at least about 0.2:1, about 0.25:1, about 0.3:1, about 0.35:1, about 0.4:1, about 0.5:1, about 0.6:1, about 0.7:1, about 0.8:1, or about 0.9:1. Optionally, in this or any other embodiment, a ratio between the constriction gap width and the maximum inner width is at most about 0.2:1, about 0.25:1, about 0.3:1, about 0.35:1, about 0.4:1, about 0.5:1, about 0.6:1, about 0.7:1, about 0.8:1, or about 0.9:1.

Optionally, in this or any other embodiment, a ratio between the constriction height and the maximum inner width is about 0.7:1 to about 0.99:1. Optionally, in this or any other embodiment, a ratio between the constriction height and the maximum inner width is at least about 0.7:1. Optionally, in this or any other embodiment, a ratio between the constriction height and the maximum inner width is at most about 0.99:1. Optionally, in this or any other embodiment, a ratio between the constriction height and the maximum inner width is about 0.7:1 to about 0.725:1, about 0.7:1 to about 0.75:1, about 0.7:1 to about 0.775:1, about 0.7:1 to about 0.8:1, about 0.7:1 to about 0.825:1, about 0.7:1 to about 0.85:1, about 0.7:1 to about 0.875:1, about 0.7:1 to about 0.9:1, about 0.7:1 to about 0.925:1, about 0.7:1 to about 0.95:1, about 0.7:1 to about 0.99:1, about 0.725:1 to about 0.75:1, about 0.725:1 to about 0.775:1, about 0.725:1 to about 0.8:1, about 0.725:1 to about 0.825:1, about 0.725:1 to about 0.85:1, about 0.725:1 to about 0.875:1, about 0.725:1 to about 0.9:1, about 0.725:1 to about 0.925:1, about 0.725:1 to about 0.95:1, about 0.725:1 to about 0.99:1, about 0.75:1 to about 0.775:1, about 0.75:1 to about 0.8:1, about 0.75:1 to about 0.825:1, about 0.75:1 to about 0.85:1, about 0.75:1 to about 0.875:1, about 0.75:1 to about 0.9:1, about 0.75:1 to about 0.925:1, about 0.75:1 to about 0.95:1, about 0.75:1 to about 0.99:1, about 0.775:1 to about 0.8:1, about 0.775:1 to about 0.825:1, about 0.775:1 to about 0.85:1, about 0.775:1 to about 0.875:1, about 0.775:1 to about 0.9:1, about 0.775:1 to about 0.925:1, about 0.775:1 to about 0.95:1, about 0.775:1 to about 0.99:1, about 0.8:1 to about 0.825:1, about 0.8:1 to about 0.85:1, about 0.8:1 to about 0.875:1, about 0.8:1 to about 0.9:1, about 0.8:1 to about 0.925:1, about 0.8:1 to about 0.95:1, about 0.8:1 to about 0.99:1, about 0.825:1 to about 0.85:1, about 0.825:1 to about 0.875:1, about 0.825:1 to about 0.9:1, about 0.825:1 to about 0.925:1, about 0.825:1 to about 0.95:1, about 0.825:1 to about 0.99:1, about 0.85:1 to about 0.875:1, about 0.85:1 to about 0.9:1, about 0.85:1 to about 0.925:1, about 0.85:1 to about 0.95:1, about 0.85:1 to about 0.99:1, about 0.875:1 to about 0.9:1, about 0.875:1 to about 0.925:1, about 0.875:1 to about 0.95:1, about 0.875:1 to about 0.99:1, about 0.9:1 to about 0.925:1, about 0.9:1 to about 0.95:1, about 0.9:1 to about 0.99:1, about 0.925:1 to about 0.95:1, about 0.925:1 to about 0.99:1, or about 0.95:1 to about 0.99:1. Optionally, in this or any other embodiment, a ratio between the constriction height and the maximum inner width is about 0.7:1, about 0.725:1, about 0.75:1, about 0.775:1, about 0.8:1, about 0.825:1, about 0.85:1, about 0.875:1, about 0.9:1, about 0.925:1, about 0.95:1, or about 0.99:1. Optionally, in this or any other embodiment, a ratio between the constriction height and the maximum inner width is at least about 0.7:1, about 0.725:1, about 0.75:1, about 0.775:1, about 0.8:1, about 0.825:1, about 0.85:1, about 0.875:1, about 0.9:1, about 0.925:1, about 0.95:1, or about 0.99:1. Optionally, in this or any other embodiment, a ratio between the constriction height and the maximum inner width is at most about 0.7:1, about 0.725:1, about 0.75:1, about 0.775:1, about 0.8:1, about 0.825:1, about 0.85:1, about 0.875:1, about 0.9:1, about 0.925:1, about 0.95:1, or about 0.99:1.

Optionally, in this or any other embodiment, a ratio between the inferior height and the maximum inner width is about 0.15:1 to about 0.6:1. Optionally, in this or any other embodiment, a ratio between the inferior height and the maximum inner width is at least about 0.15:1. Optionally, in this or any other embodiment, a ratio between the inferior height and the maximum inner width is at most about 0.6:1. Optionally, in this or any other embodiment, a ratio between the inferior height and the maximum inner width is about 0.15:1 to about 0.2:1, about 0.15:1 to about 0.25:1, about 0.15:1 to about 0.3:1, about 0.15:1 to about 0.35:1, about 0.15:1 to about 0.4:1, about 0.15:1 to about 0.45:1, about 0.15:1 to about 0.5:1, about 0.15:1 to about 0.55:1, about 0.15:1 to about 0.6:1, about 0.2:1 to about 0.25:1, about 0.2:1 to about 0.3:1, about 0.2:1 to about 0.35:1, about 0.2:1 to about 0.4:1, about 0.2:1 to about 0.45:1, about 0.2:1 to about 0.5:1, about 0.2:1 to about 0.55:1, about 0.2:1 to about 0.6:1, about 0.25:1 to about 0.3:1, about 0.25:1 to about 0.35:1, about 0.25:1 to about 0.4:1, about 0.25:1 to about 0.45:1, about 0.25:1 to about 0.5:1, about 0.25:1 to about 0.55:1, about 0.25:1 to about 0.6:1, about 0.3:1 to about 0.35:1, about 0.3:1 to about 0.4:1, about 0.3:1 to about 0.45:1, about 0.3:1 to about 0.5:1, about 0.3:1 to about 0.55:1, about 0.3:1 to about 0.6:1, about 0.35:1 to about 0.4:1, about 0.35:1 to about 0.45:1, about 0.35:1 to about 0.5:1, about 0.35:1 to about 0.55:1, about 0.35:1 to about 0.6:1, about 0.4:1 to about 0.45:1, about 0.4:1 to about 0.5:1, about 0.4:1 to about 0.55:1, about 0.4:1 to about 0.6:1, about 0.45:1 to about 0.5:1, about 0.45:1 to about 0.55:1, about 0.45:1 to about 0.6:1, about 0.5:1 to about 0.55:1, about 0.5:1 to about 0.6:1, or about 0.55:1 to about 0.6:1. Optionally, in this or any other embodiment, a ratio between the inferior height and the maximum inner width is about 0.15:1, about 0.2:1, about 0.25:1, about 0.3:1, about 0.35:1, about 0.4:1, about 0.45:1, about 0.5:1, about 0.55:1, or about 0.6:1. Optionally, in this or any other embodiment, a ratio between the inferior height and the maximum inner width is at least about 0.15:1, about 0.2:1, about 0.25:1, about 0.3:1, about 0.35:1, about 0.4:1, about 0.45:1, about 0.5:1, about 0.55:1, or about 0.6:1. Optionally, in this or any other embodiment, a ratio between the inferior height and the maximum inner width is at most about 0.15:1, about 0.2:1, about 0.25:1, about 0.3:1, about 0.35:1, about 0.4:1, about 0.45:1, about 0.5:1, about 0.55:1, or about 0.6:1.

Optionally, in this or any other embodiment, the arched rod has a thickness, wherein the thickness is measured as a maximum normal cross-sectional width of at least a portion of at least one of the first arm, the second arm, and the bridge. Optionally, in this or any other embodiment, the device has a thickness of about 0.125 inches to about 0.8 inches. Optionally, in this or any other embodiment, the device has a thickness of at least about 0.125 inches. Optionally, in this or any other embodiment, the device has a thickness of at most about 0.8 inches. Optionally, in this or any other embodiment, the device has a thickness of about 0.125 inches to about 0.15 inches, about 0.125 inches to about 0.2 inches, about 0.125 inches to about 0.25 inches, about 0.125 inches to about 0.3 inches, about 0.125 inches to about 0.35 inches, about 0.125 inches to about 0.4 inches, about 0.125 inches to about 0.45 inches, about 0.125 inches to about 0.5 inches, about 0.125 inches to about 0.6 inches, about 0.125 inches to about 0.7 inches, about 0.125 inches to about 0.8 inches, about 0.15 inches to about 0.2 inches, about 0.15 inches to about 0.25 inches, about 0.15 inches to about 0.3 inches, about 0.15 inches to about 0.35 inches, about 0.15 inches to about 0.4 inches, about 0.15 inches to about 0.45 inches, about 0.15 inches to about 0.5 inches, about 0.15 inches to about 0.6 inches, about 0.15 inches to about 0.7 inches, about 0.15 inches to about 0.8 inches, about 0.2 inches to about 0.25 inches, about 0.2 inches to about 0.3 inches, about 0.2 inches to about 0.35 inches, about 0.2 inches to about 0.4 inches, about 0.2 inches to about 0.45 inches, about 0.2 inches to about 0.5 inches, about 0.2 inches to about 0.6 inches, about 0.2 inches to about 0.7 inches, about 0.2 inches to about 0.8 inches, about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.6 inches, about 0.25 inches to about 0.7 inches, about 0.25 inches to about 0.8 inches, about 0.3 inches to about 0.35 inches, about 0.3 inches to about 0.4 inches, about 0.3 inches to about 0.45 inches, about 0.3 inches to about 0.5 inches, about 0.3 inches to about 0.6 inches, about 0.3 inches to about 0.7 inches, about 0.3 inches to about 0.8 inches, about 0.35 inches to about 0.4 inches, about 0.35 inches to about 0.45 inches, about 0.35 inches to about 0.5 inches, about 0.35 inches to about 0.6 inches, about 0.35 inches to about 0.7 inches, about 0.35 inches to about 0.8 inches, about 0.4 inches to about 0.45 inches, about 0.4 inches to about 0.5 inches, about 0.4 inches to about 0.6 inches, about 0.4 inches to about 0.7 inches, about 0.4 inches to about 0.8 inches, about 0.45 inches to about 0.5 inches, about 0.45 inches to about 0.6 inches, about 0.45 inches to about 0.7 inches, about 0.45 inches to about 0.8 inches, about 0.5 inches to about 0.6 inches, about 0.5 inches to about 0.7 inches, about 0.5 inches to about 0.8 inches, about 0.6 inches to about 0.7 inches, about 0.6 inches to about 0.8 inches, or about 0.7 inches to about 0.8 inches. Optionally, in this or any other embodiment, the device has a thickness of about 0.125 inches, about 0.15 inches, about 0.2 inches, about 0.25 inches, about 0.3 inches, about 0.35 inches, about 0.4 inches, about 0.45 inches, about 0.5 inches, about 0.6 inches, about 0.7 inches, or about 0.8 inches. Optionally, in this or any other embodiment, the device has a thickness of at least about 0.125 inches, about 0.15 inches, about 0.2 inches, about 0.25 inches, about 0.3 inches, about 0.35 inches, about 0.4 inches, about 0.45 inches, about 0.5 inches, about 0.6 inches, about 0.7 inches, or about 0.8 inches. Optionally, in this or any other embodiment, the device has a thickness of at most about 0.125 inches, about 0.15 inches, about 0.2 inches, about 0.25 inches, about 0.3 inches, about 0.35 inches, about 0.4 inches, about 0.45 inches, about 0.5 inches, about 0.6 inches, about 0.7 inches, or about 0.8 inches.

Optionally, in this or any other embodiment, has a first termination distance and a second termination distance, wherein the first termination distance may be measured as the maximum normal distance between the constriction gap width and the first terminus, and wherein the second termination distance may be measured as the maximum normal distance between the constriction gap width and the second terminus. Optionally, in this or any other embodiment, at least one of the first termination distance and the second termination distance is about 0.02 inches to about 0.08 inches. Optionally, in this or any other embodiment, at least one of the first termination distance and the second termination distance is at least about 0.02 inches. Optionally, in this or any other example, at least one of the first termination distance and the second termination distance is at most about 0.08 inches. Optionally, in this or any other example, at least one of the first termination distance and the second termination distance is about 0.02 inches to about 0.025 inches, about 0.02 inches to about 0.03 inches, about 0.02 inches to about 0.035 inches, about 0.02 inches to about 0.04 inches, about 0.02 inches to about 0.045 inches, about 0.02 inches to about 0.05 inches, about 0.02 inches to about 0.055 inches, about 0.02 inches to about 0.06 inches, about 0.02 inches to about 0.07 inches, about 0.02 inches to about 0.08 inches, about 0.025 inches to about 0.03 inches, about 0.025 inches to about 0.035 inches, about 0.025 inches to about 0.04 inches, about 0.025 inches to about 0.045 inches, about 0.025 inches to about 0.05 inches, about 0.025 inches to about 0.055 inches, about 0.025 inches to about 0.06 inches, about 0.025 inches to about 0.07 inches, about 0.025 inches to about 0.08 inches, about 0.03 inches to about 0.035 inches, about 0.03 inches to about 0.04 inches, about 0.03 inches to about 0.045 inches, about 0.03 inches to about 0.05 inches, about 0.03 inches to about 0.055 inches, about 0.03 inches to about 0.06 inches, about 0.03 inches to about 0.07 inches, about 0.03 inches to about 0.08 inches, about 0.035 inches to about 0.04 inches, about 0.035 inches to about 0.045 inches, about 0.035 inches to about 0.05 inches, about 0.035 inches to about 0.055 inches, about 0.035 inches to about 0.06 inches, about 0.035 inches to about 0.07 inches, about 0.035 inches to about 0.08 inches, about 0.04 inches to about 0.045 inches, about 0.04 inches to about 0.05 inches, about 0.04 inches to about 0.055 inches, about 0.04 inches to about 0.06 inches, about 0.04 inches to about 0.07 inches, about 0.04 inches to about 0.08 inches, about 0.045 inches to about 0.05 inches, about 0.045 inches to about 0.055 inches, about 0.045 inches to about 0.06 inches, about 0.045 inches to about 0.07 inches, about 0.045 inches to about 0.08 inches, about 0.05 inches to about 0.055 inches, about 0.05 inches to about 0.06 inches, about 0.05 inches to about 0.07 inches, about 0.05 inches to about 0.08 inches, about 0.055 inches to about 0.06 inches, about 0.055 inches to about 0.07 inches, about 0.055 inches to about 0.08 inches, about 0.06 inches to about 0.07 inches, about 0.06 inches to about 0.08 inches, or about 0.07 inches to about 0.08 inches. Optionally, in this or any other embodiment, at least one of the first termination distance and the second termination distance is about 0.02 inches, about 0.025 inches, about 0.03 inches, about 0.035 inches, about 0.04 inches, about 0.045 inches, about 0.05 inches, about 0.055 inches, about 0.06 inches, about 0.07 inches, or about 0.08 inches. Optionally, in this or any other embodiment, at least one of the first termination distance and the second termination distance is at least about 0.02 inches, about 0.025 inches, about 0.03 inches, about 0.035 inches, about 0.04 inches, about 0.045 inches, about 0.05 inches, about 0.055 inches, about 0.06 inches, about 0.07 inches, or about 0.08 inches. Optionally, in this or any other embodiment, at least one of the first termination distance and the second termination distance is at most about 0.02 inches, about 0.025 inches, about 0.03 inches, about 0.035 inches, about 0.04 inches, about 0.045 inches, about 0.05 inches, about 0.055 inches, about 0.06 inches, about 0.07 inches, or about 0.08 inches.

Optionally, in this or any other embodiment, the first terminus diverges from the constriction region to form a first elbow. Optionally, in this or any other embodiment, the second terminus diverges from the constriction region to form a second elbow. Optionally, in this or any other embodiment, the first elbow and the second elbow are configured to removably affix to a band. Optionally, in this or any other embodiment, the first arm and the second arm are configured to removably affix to a band.

Optionally, in this or any other embodiment, the compression region comprises: a first dorsal vein protrusion comprising: a first dorsal apex; and a first dorsal height measured as a first minimum normal distance between the first dorsal apex and a first lateral edge of the first dorsal vein protrusion where the first dorsal vein protrusion rises from the inferior side of the bridge. Optionally, in this or any other embodiment, the compression region comprises a second dorsal vein protrusion comprising: a second dorsal apex; and a second dorsal height measured as a second minimum normal distance between the second dorsal apex and a second lateral edge of the second dorsal vein protrusion where the second dorsal vein protrusion rises from the inferior side of the bridge. Optionally, in this or any other embodiment, the compression region comprises: a dorsal vein valley between the first dorsal vein apex and the second dorsal vein apex.

Optionally, in this or any other embodiment, the first dorsal vein protrusion, the dorsal vein valley, and the second dorsal vein protrusion are arranged sequentially within the compression region in a direction along the bridge from the first arm to the second arm.

Optionally, in this or any other embodiment, the device comprises a dorsal vein valley distance between the first dorsal apex and the second dorsal apex. Optionally, in this or any other embodiment, the dorsal vein valley distance is about 0.15 inches to about 0.75 inches. Optionally, in this or any other embodiment, the dorsal vein valley distance is at least about 0.15 inches. Optionally, in this or any other embodiment, the dorsal vein valley distance is at most about 0.75 inches. Optionally, in this or any other embodiment, the dorsal vein valley distance is about 0.15 inches to about 0.2 inches, about 0.15 inches to about 0.25 inches, about 0.15 inches to about 0.3 inches, about 0.15 inches to about 0.35 inches, about 0.15 inches to about 0.4 inches, about 0.15 inches to about 0.45 inches, about 0.15 inches to about 0.5 inches, about 0.15 inches to about 0.55 inches, about 0.15 inches to about 0.6 inches, about 0.15 inches to about 0.65 inches, about 0.15 inches to about 0.75 inches, about 0.2 inches to about 0.25 inches, about 0.2 inches to about 0.3 inches, about 0.2 inches to about 0.35 inches, about 0.2 inches to about 0.4 inches, about 0.2 inches to about 0.45 inches, about 0.2 inches to about 0.5 inches, about 0.2 inches to about 0.55 inches, about 0.2 inches to about 0.6 inches, about 0.2 inches to about 0.65 inches, about 0.2 inches to about 0.75 inches, about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.55 inches, about 0.25 inches to about 0.6 inches, about 0.25 inches to about 0.65 inches, about 0.25 inches to about 0.75 inches, about 0.3 inches to about 0.35 inches, about 0.3 inches to about 0.4 inches, about 0.3 inches to about 0.45 inches, about 0.3 inches to about 0.5 inches, about 0.3 inches to about 0.55 inches, about 0.3 inches to about 0.6 inches, about 0.3 inches to about 0.65 inches, about 0.3 inches to about 0.75 inches, about 0.35 inches to about 0.4 inches, about 0.35 inches to about 0.45 inches, about 0.35 inches to about 0.5 inches, about 0.35 inches to about 0.55 inches, about 0.35 inches to about 0.6 inches, about 0.35 inches to about 0.65 inches, about 0.35 inches to about 0.75 inches, about 0.4 inches to about 0.45 inches, about 0.4 inches to about 0.5 inches, about 0.4 inches to about 0.55 inches, about 0.4 inches to about 0.6 inches, about 0.4 inches to about 0.65 inches, about 0.4 inches to about 0.75 inches, about 0.45 inches to about 0.5 inches, about 0.45 inches to about 0.55 inches, about 0.45 inches to about 0.6 inches, about 0.45 inches to about 0.65 inches, about 0.45 inches to about 0.75 inches, about 0.5 inches to about 0.55 inches, about 0.5 inches to about 0.6 inches, about 0.5 inches to about 0.65 inches, about 0.5 inches to about 0.75 inches, about 0.55 inches to about 0.6 inches, about 0.55 inches to about 0.65 inches, about 0.55 inches to about 0.75 inches, about 0.6 inches to about 0.65 inches, about 0.6 inches to about 0.75 inches, or about 0.65 inches to about 0.75 inches. Optionally, in this or any other embodiment, the dorsal vein valley distance is about 0.15 inches, about 0.2 inches, about 0.25 inches, about 0.3 inches, about 0.35 inches, about 0.4 inches, about 0.45 inches, about 0.5 inches, about 0.55 inches, about 0.6 inches, about 0.65 inches, or about 0.75 inches. Optionally, in this or any other embodiment, the dorsal vein valley distance is at least about 0.15 inches, about 0.2 inches, about 0.25 inches, about 0.3 inches, about 0.35 inches, about 0.4 inches, about 0.45 inches, about 0.5 inches, about 0.55 inches, about 0.6 inches, about 0.65 inches, or about 0.75 inches. Optionally, in this or any other embodiment, the dorsal vein valley distance is at most about 0.15 inches, about 0.2 inches, about 0.25 inches, about 0.3 inches, about 0.35 inches, about 0.4 inches, about 0.45 inches, about 0.5 inches, about 0.55 inches, about 0.6 inches, about 0.65 inches, or about 0.75 inches.

Optionally, in this or any other embodiment, the device comprises a compression region width between the first lateral edge and the second lateral edge. Optionally, in this or any other embodiment, the compression region width is about 0.2 inches to about 0.8 inches. Optionally, in this or any other embodiment, the compression region width is at least about 0.2 inches. Optionally, in this or any other embodiment, the compression region width is at most about 0.8 inches. Optionally, in this or any other embodiment, the compression region width is about 0.2 inches to about 0.25 inches, about 0.2 inches to about 0.3 inches, about 0.2 inches to about 0.35 inches, about 0.2 inches to about 0.4 inches, about 0.2 inches to about 0.45 inches, about 0.2 inches to about 0.5 inches, about 0.2 inches to about 0.55 inches, about 0.2 inches to about 0.6 inches, about 0.2 inches to about 0.7 inches, about 0.2 inches to about 0.8 inches, about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.55 inches, about 0.25 inches to about 0.6 inches, about 0.25 inches to about 0.7 inches, about 0.25 inches to about 0.8 inches, about 0.3 inches to about 0.35 inches, about 0.3 inches to about 0.4 inches, about 0.3 inches to about 0.45 inches, about 0.3 inches to about 0.5 inches, about 0.3 inches to about 0.55 inches, about 0.3 inches to about 0.6 inches, about 0.3 inches to about 0.7 inches, about 0.3 inches to about 0.8 inches, about 0.35 inches to about 0.4 inches, about 0.35 inches to about 0.45 inches, about 0.35 inches to about 0.5 inches, about 0.35 inches to about 0.55 inches, about 0.35 inches to about 0.6 inches, about 0.35 inches to about 0.7 inches, about 0.35 inches to about 0.8 inches, about 0.4 inches to about 0.45 inches, about 0.4 inches to about 0.5 inches, about 0.4 inches to about 0.55 inches, about 0.4 inches to about 0.6 inches, about 0.4 inches to about 0.7 inches, about 0.4 inches to about 0.8 inches, about 0.45 inches to about 0.5 inches, about 0.45 inches to about 0.55 inches, about 0.45 inches to about 0.6 inches, about 0.45 inches to about 0.7 inches, about 0.45 inches to about 0.8 inches, about 0.5 inches to about 0.55 inches, about 0.5 inches to about 0.6 inches, about 0.5 inches to about 0.7 inches, about 0.5 inches to about 0.8 inches, about 0.55 inches to about 0.6 inches, about 0.55 inches to about 0.7 inches, about 0.55 inches to about 0.8 inches, about 0.6 inches to about 0.7 inches, about 0.6 inches to about 0.8 inches, or about 0.7 inches to about 0.8 inches. Optionally, in this or any other embodiment, the compression region width is about 0.2 inches, about 0.25 inches, about 0.3 inches, about 0.35 inches, about 0.4 inches, about 0.45 inches, about 0.5 inches, about 0.55 inches, about 0.6 inches, about 0.7 inches, or about 0.8 inches. Optionally, in this or any other embodiment, the compression region width is at least about 0.2 inches, about 0.25 inches, about 0.3 inches, about 0.35 inches, about 0.4 inches, about 0.45 inches, about 0.5 inches, about 0.55 inches, about 0.6 inches, about 0.7 inches, or about 0.8 inches. Optionally, in this or any other embodiment, the compression region width is at most about 0.2 inches, about 0.25 inches, about 0.3 inches, about 0.35 inches, about 0.4 inches, about 0.45 inches, about 0.5 inches, about 0.55 inches, about 0.6 inches, about 0.7 inches, or about 0.8 inches.

Optionally, in this or any other embodiment, the compression region within the dorsal vein valley at a center of the dorsal vein valley is superior to or has a same height relative to the first lateral edge as the first minimum normal distance or relative to the second lateral edge as the second minimum normal distance, or both. Optionally, in this or any other embodiment, the compression region is superior to or has a same height relative to the first lateral edge as the first minimum normal distance or relative to the second lateral edge as the second minimum normal distance, or both. Optionally, in this or any other embodiment, the compression region at the center of the dorsal vein valley is inferior to the first lateral edge, the second lateral edge, or both. Optionally, in this or any other embodiment, the compression region is inferior to the first lateral edge, the second lateral edge, or both.

Optionally, in this or any other embodiment, the device further comprises a plurality of friction protrusions on a superior side of the bridge. Optionally, in this or any other embodiment, the plurality of friction protrusions comprises about 2 protrusions to about 60 protrusions. Optionally, in this or any other embodiment, the plurality of friction protrusions comprises at least about 2 protrusions. Optionally, in this or any other embodiment, the plurality of friction protrusions comprises at most about 60 protrusions. Optionally, in this or any other embodiment, the plurality of friction protrusions comprises about 2 protrusions to about 4 protrusions, about 2 protrusions to about 6 protrusions, about 2 protrusions to about 8 protrusions, about 2 protrusions to about 10 protrusions, about 2 protrusions to about 15 protrusions, about 2 protrusions to about 20 protrusions, about 2 protrusions to about 25 protrusions, about 2 protrusions to about 30 protrusions, about 2 protrusions to about 40 protrusions, about 2 protrusions to about 50 protrusions, about 2 protrusions to about 60 protrusions, about 4 protrusions to about 6 protrusions, about 4 protrusions to about 8 protrusions, about 4 protrusions to about 10 protrusions, about 4 protrusions to about 15 protrusions, about 4 protrusions to about 20 protrusions, about 4 protrusions to about 25 protrusions, about 4 protrusions to about 30 protrusions, about 4 protrusions to about 40 protrusions, about 4 protrusions to about 50 protrusions, about 4 protrusions to about 60 protrusions, about 6 protrusions to about 8 protrusions, about 6 protrusions to about 10 protrusions, about 6 protrusions to about 15 protrusions, about 6 protrusions to about 20 protrusions, about 6 protrusions to about 25 protrusions, about 6 protrusions to about 30 protrusions, about 6 protrusions to about 40 protrusions, about 6 protrusions to about 50 protrusions, about 6 protrusions to about 60 protrusions, about 8 protrusions to about 10 protrusions, about 8 protrusions to about 15 protrusions, about 8 protrusions to about 20 protrusions, about 8 protrusions to about 25 protrusions, about 8 protrusions to about 30 protrusions, about 8 protrusions to about 40 protrusions, about 8 protrusions to about 50 protrusions, about 8 protrusions to about 60 protrusions, about 10 protrusions to about 15 protrusions, about 10 protrusions to about 20 protrusions, about 10 protrusions to about 25 protrusions, about 10 protrusions to about 30 protrusions, about 10 protrusions to about 40 protrusions, about 10 protrusions to about 50 protrusions, about 10 protrusions to about 60 protrusions, about 15 protrusions to about 20 protrusions, about 15 protrusions to about 25 protrusions, about 15 protrusions to about 30 protrusions, about 15 protrusions to about 40 protrusions, about 15 protrusions to about 50 protrusions, about 15 protrusions to about 60 protrusions, about 20 protrusions to about 25 protrusions, about 20 protrusions to about 30 protrusions, about 20 protrusions to about 40 protrusions, about 20 protrusions to about 50 protrusions, about 20 protrusions to about 60 protrusions, about 25 protrusions to about 30 protrusions, about 25 protrusions to about 40 protrusions, about 25 protrusions to about 50 protrusions, about 25 protrusions to about 60 protrusions, about 30 protrusions to about 40 protrusions, about 30 protrusions to about 50 protrusions, about 30 protrusions to about 60 protrusions, about 40 protrusions to about 50 protrusions, about 40 protrusions to about 60 protrusions, or about 50 protrusions to about 60 protrusions. Optionally, in this or any other embodiment, the plurality of friction protrusions comprises about 2 protrusions, about 4 protrusions, about 6 protrusions, about 8 protrusions, about 10 protrusions, about 15 protrusions, about 20 protrusions, about 25 protrusions, about 30 protrusions, about 40 protrusions, about 50 protrusions, or about 60 protrusions. Optionally, in this or any other embodiment, the plurality of friction protrusions comprises at most about 2 protrusions, about 4 protrusions, about 6 protrusions, about 8 protrusions, about 10 protrusions, about 15 protrusions, about 20 protrusions, about 25 protrusions, about 30 protrusions, about 40 protrusions, about 50 protrusions, or about 60 protrusions. Optionally, in this or any other embodiment, the plurality of friction protrusions comprises at least about 2 protrusions, about 4 protrusions, about 6 protrusions, about 8 protrusions, about 10 protrusions, about 15 protrusions, about 20 protrusions, about 25 protrusions, about 30 protrusions, about 40 protrusions, about 50 protrusions, or about 60 protrusions.

Optionally, in this or any other embodiment, the friction protrusions have a height of about 0.01 inches to about 0.06 inches. Optionally, in this or any other embodiment, the friction protrusions have a height of about 0.01 inches to about 0.02 inches, about 0.01 inches to about 0.03 inches, about 0.01 inches to about 0.04 inches, about 0.01 inches to about 0.05 inches, about 0.01 inches to about 0.06 inches, about 0.02 inches to about 0.03 inches, about 0.02 inches to about 0.04 inches, about 0.02 inches to about 0.05 inches, about 0.02 inches to about 0.06 inches, about 0.03 inches to about 0.04 inches, about 0.03 inches to about 0.05 inches, about 0.03 inches to about 0.06 inches, about 0.04 inches to about 0.05 inches, about 0.04 inches to about 0.06 inches, or about 0.05 inches to about 0.06 inches. Optionally, in this or any other embodiment, the friction protrusions have a height of about 0.01 inches, about 0.02 inches, about 0.03 inches, about 0.04 inches, about 0.05 inches, or about 0.06 inches. Optionally, in this or any other embodiment, the friction protrusions have a height of at least about 0.01 inches, about 0.02 inches, about 0.03 inches, about 0.04 inches, or about 0.05 inches. Optionally, in this or any other embodiment, the friction protrusions have a height of at most about 0.02 inches, about 0.03 inches, about 0.04 inches, about 0.05 inches, or about 0.06 inches.

Optionally, in this or any other embodiment, the friction protrusions have a width of about 0.01 inches to about 0.06 inches. Optionally, in this or any other embodiment, the friction protrusions have a width of about 0.01 inches to about 0.02 inches, about 0.01 inches to about 0.03 inches, about 0.01 inches to about 0.04 inches, about 0.01 inches to about 0.05 inches, about 0.01 inches to about 0.06 inches, about 0.02 inches to about 0.03 inches, about 0.02 inches to about 0.04 inches, about 0.02 inches to about 0.05 inches, about 0.02 inches to about 0.06 inches, about 0.03 inches to about 0.04 inches, about 0.03 inches to about 0.05 inches, about 0.03 inches to about 0.06 inches, about 0.04 inches to about 0.05 inches, about 0.04 inches to about 0.06 inches, or about 0.05 inches to about 0.06 inches. Optionally, in this or any other embodiment, the friction protrusions have a width of about 0.01 inches, about 0.02 inches, about 0.03 inches, about 0.04 inches, about 0.05 inches, or about 0.06 inches. Optionally, in this or any other embodiment, the friction protrusions have a width of at least about 0.01 inches, about 0.02 inches, about 0.03 inches, about 0.04 inches, or about 0.05 inches. Optionally, in this or any other embodiment, the friction protrusions have a width of at most about 0.02 inches, about 0.03 inches, about 0.04 inches, about 0.05 inches, or about 0.06 inches.

Optionally, in this or any other embodiment, the arched rod comprises a cover substantially surrounding a frame, wherein a frame modulus of elasticity of the frame is higher than a cover modulus of elasticity of the cover.

Another aspect provided herein is a penile constriction system comprising a band and a penile constriction device comprising an arched rod comprising: a first arm comprising a first coupling end and a first converging end having a first terminus; a second arm comprising a second coupling end and a second converging end having a second terminus disconnected from the first terminus, wherein the first converging end and the second converging end converge to form a constriction region; and a bridge connecting the first coupling end to the second coupling end, the bridge comprising a compression region rising inferiorly from an inferior side of the bridge.

Optionally, in this or any other embodiment, the device of the penile constriction system has a constriction gap width, wherein the constriction gap width is measured as a minimum distance between the first converging end and the second converging end within the constriction region. Optionally, in this or any other embodiment, the device of the penile constriction system has a constriction gap width of about 0 inches to about 1.25 inches. Optionally, in this or any other embodiment, the device of the penile constriction system has a constriction gap width of at least about 0 inches. Optionally, in this or any other embodiment, the device of the penile constriction system has a constriction gap width of at most about 1.25 inches. Optionally, in this or any other embodiment, the device of the penile constriction system has a constriction gap width of about 0 inches to about 0.125 inches, about 0 inches to about 0.375 inches, about 0 inches to about 0.5 inches, about 0 inches to about 0.625 inches, about 0 inches to about 0.75 inches, about 0 inches to about 0.875 inches, about 0 inches to about 1 inch, about 0 inches to about 1.125 inches, about 0 inches to about 1.25 inches, about 0.125 inches to about 0.375 inches, about 0.125 inches to about 0.5 inches, about 0.125 inches to about 0.625 inches, about 0.125 inches to about 0.75 inches, about 0.125 inches to about 0.875 inches, about 0.125 inches to about 1 inch, about 0.125 inches to about 1.125 inches, about 0.125 inches to about 1.25 inches, about 0.375 inches to about 0.5 inches, about 0.375 inches to about 0.625 inches, about 0.375 inches to about 0.75 inches, about 0.375 inches to about 0.875 inches, about 0.375 inches to about 1 inch, about 0.375 inches to about 1.125 inches, about 0.375 inches to about 1.25 inches, about 0.5 inches to about 0.625 inches, about 0.5 inches to about 0.75 inches, about 0.5 inches to about 0.875 inches, about 0.5 inches to about 1 inch, about 0.5 inches to about 1.125 inches, about 0.5 inches to about 1.25 inches, about 0.625 inches to about 0.75 inches, about 0.625 inches to about 0.875 inches, about 0.625 inches to about 1 inch, about 0.625 inches to about 1.125 inches, about 0.625 inches to about 1.25 inches, about 0.75 inches to about 0.875 inches, about 0.75 inches to about 1 inch, about 0.75 inches to about 1.125 inches, about 0.75 inches to about 1.25 inches, about 0.875 inches to about 1 inch, about 0.875 inches to about 1.125 inches, about 0.875 inches to about 1.25 inches, about 1 inch to about 1.125 inches, about 1 inch to about 1.25 inches, or about 1.125 inches to about 1.25 inches. Optionally, in this or any other embodiment, the device of the penile constriction system has a constriction gap width of about 0 inches, about 0.125 inches, about 0.375 inches, about 0.5 inches, about 0.625 inches, about 0.75 inches, about 0.875 inches, about 1 inch, about 1.125 inches, or about 1.25 inches. Optionally, in this or any other embodiment, the device of the penile constriction system has a constriction gap width of at least about 0 inches, about 0.125 inches, about 0.375 inches, about 0.5 inches, about 0.625 inches, about 0.75 inches, about 0.875 inches, about 1 inch, about 1.125 inches, or about 1.25 inches. Optionally, in this or any other embodiment, the device of the penile constriction system has a constriction gap width of at most about 0.125 inches, about 0.375 inches, about 0.5 inches, about 0.625 inches, about 0.75 inches, about 0.875 inches, about 1 inch, about 1.125 inches, or about 1.25 inches.

Optionally, in this or any other embodiment, the device of the penile constriction system has a constriction height, wherein the constriction height is measured as a normal distance between a constriction point and the inferior side of the bridge, and wherein the constriction point is defined as the midpoint of the constriction gap width. Optionally, in this or any other embodiment, the device of the penile constriction system has a constriction height of about 0.5 inches to about 2.5 inches. Optionally, in this or any other embodiment, the device of the penile constriction system has a constriction height of at least about 0.5 inches. Optionally, in this or any other embodiment, the device of the penile constriction system has a constriction height of at most about 2.5 inches. Optionally, in this or any other embodiment, the device of the penile constriction system has a constriction height of about 0.5 inches to about 0.75 inches, about 0.5 inches to about 1 inch, about 0.5 inches to about 1.25 inches, about 0.5 inches to about 1.5 inches, about 0.5 inches to about 1.75 inches, about 0.5 inches to about 2 inches, about 0.5 inches to about 2.25 inches, about 0.5 inches to about 2.5 inches, about 0.75 inches to about 1 inch, about 0.75 inches to about 1.25 inches, about 0.75 inches to about 1.5 inches, about 0.75 inches to about 1.75 inches, about 0.75 inches to about 2 inches, about 0.75 inches to about 2.25 inches, about 0.75 inches to about 2.5 inches, about 1 inch to about 1.25 inches, about 1 inch to about 1.5 inches, about 1 inch to about 1.75 inches, about 1 inch to about 2 inches, about 1 inch to about 2.25 inches, about 1 inch to about 2.5 inches, about 1.25 inches to about 1.5 inches, about 1.25 inches to about 1.75 inches, about 1.25 inches to about 2 inches, about 1.25 inches to about 2.25 inches, about 1.25 inches to about 2.5 inches, about 1.5 inches to about 1.75 inches, about 1.5 inches to about 2 inches, about 1.5 inches to about 2.25 inches, about 1.5 inches to about 2.5 inches, about 1.75 inches to about 2 inches, about 1.75 inches to about 2.25 inches, about 1.75 inches to about 2.5 inches, about 2 inches to about 2.25 inches, about 2 inches to about 2.5 inches, or about 2.25 inches to about 2.5 inches. Optionally, in this or any other embodiment, the device of the penile constriction system has a constriction height of about 0.5 inches, about 0.75 inches, about 1 inch, about 1.25 inches, about 1.5 inches, about 1.75 inches, about 2 inches, about 2.25 inches, or about 2.5 inches. Optionally, in this or any other embodiment, the device of the penile constriction system has a constriction height of at least about 0.5 inches, about 0.75 inches, about 1 inch, about 1.25 inches, about 1.5 inches, about 1.75 inches, about 2 inches, about 2.25 inches, or about 2.5 inches. Optionally, in this or any other embodiment, the device of the penile constriction system has a constriction height of at most about 0.5 inches, about 0.75 inches, about 1 inch, about 1.25 inches, about 1.5 inches, about 1.75 inches, about 2 inches, about 2.25 inches, or about 2.5 inches.

Optionally, in this or any other embodiment, the device of the penile constriction system has a maximum inner width, wherein the maximum inner width is measured as a maximum normal distance between a mesial vertex of the surface of the first arm and a mesial vertex of the surface of the second arm, and wherein the maximum inner width is superior to the constriction gap. Optionally, in this or any other embodiment, the device of the penile constriction system has a maximum inner width of about 0.5 inches to about 3 inches. Optionally, in this or any other embodiment, the device of the penile constriction system has a maximum inner width of at least about 0.5 inches. Optionally, in this or any other embodiment, the device of the penile constriction system has a maximum inner width of at most about 3 inches. Optionally, in this or any other embodiment, the device of the penile constriction system has a maximum inner width of about 0.5 inches to about 0.75 inches, about 0.5 inches to about 1 inch, about 0.5 inches to about 1.25 inches, about 0.5 inches to about 1.5 inches, about 0.5 inches to about 1.75 inches, about 0.5 inches to about 2 inches, about 0.5 inches to about 2.25 inches, about 0.5 inches to about 2.5 inches, about 0.5 inches to about 2.75 inches, about 0.5 inches to about 3 inches, about 0.75 inches to about 1 inch, about 0.75 inches to about 1.25 inches, about 0.75 inches to about 1.5 inches, about 0.75 inches to about 1.75 inches, about 0.75 inches to about 2 inches, about 0.75 inches to about 2.25 inches, about 0.75 inches to about 2.5 inches, about 0.75 inches to about 2.75 inches, about 0.75 inches to about 3 inches, about 1 inch to about 1.25 inches, about 1 inch to about 1.5 inches, about 1 inch to about 1.75 inches, about 1 inch to about 2 inches, about 1 inch to about 2.25 inches, about 1 inch to about 2.5 inches, about 1 inch to about 2.75 inches, about 1 inch to about 3 inches, about 1.25 inches to about 1.5 inches, about 1.25 inches to about 1.75 inches, about 1.25 inches to about 2 inches, about 1.25 inches to about 2.25 inches, about 1.25 inches to about 2.5 inches, about 1.25 inches to about 2.75 inches, about 1.25 inches to about 3 inches, about 1.5 inches to about 1.75 inches, about 1.5 inches to about 2 inches, about 1.5 inches to about 2.25 inches, about 1.5 inches to about 2.5 inches, about 1.5 inches to about 2.75 inches, about 1.5 inches to about 3 inches, about 1.75 inches to about 2 inches, about 1.75 inches to about 2.25 inches, about 1.75 inches to about 2.5 inches, about 1.75 inches to about 2.75 inches, about 1.75 inches to about 3 inches, about 2 inches to about 2.25 inches, about 2 inches to about 2.5 inches, about 2 inches to about 2.75 inches, about 2 inches to about 3 inches, about 2.25 inches to about 2.5 inches, about 2.25 inches to about 2.75 inches, about 2.25 inches to about 3 inches, about 2.5 inches to about 2.75 inches, about 2.5 inches to about 3 inches, or about 2.75 inches to about 3 inches. Optionally, in this or any other embodiment, the device of the penile constriction system has a maximum inner width of about 0.5 inches, about 0.75 inches, about 1 inch, about 1.25 inches, about 1.5 inches, about 1.75 inches, about 2 inches, about 2.25 inches, about 2.5 inches, about 2.75 inches, or about 3 inches. Optionally, in this or any other embodiment, the device of the penile constriction system has a maximum inner width of at least about 0.5 inches, about 0.75 inches, about 1 inch, about 1.25 inches, about 1.5 inches, about 1.75 inches, about 2 inches, about 2.25 inches, about 2.5 inches, about 2.75 inches, or about 3 inches. Optionally, in this or any other embodiment, the device of the penile constriction system has a maximum inner width of at most about 0.5 inches, about 0.75 inches, about 1 inch, about 1.25 inches, about 1.5 inches, about 1.75 inches, about 2 inches, about 2.25 inches, about 2.5 inches, about 2.75 inches, or about 3 inches.

Optionally, in this or any other embodiment, the device of the penile constriction system has an inferior height, wherein the inferior height is measured as a normal distance between a center point, and the inferior side of the bridge, wherein the center point comprises the midpoint of the maximum inner width. Optionally, in this or any other embodiment, the device of the penile constriction system has an inferior height of about 0.25 inches to about 1 inch. Optionally, in this or any other embodiment, the device of the penile constriction system has an inferior height of at least about 0.25 inches. Optionally, in this or any other embodiment, the device of the penile constriction system has an inferior height of at most about 1 inch. Optionally, in this or any other embodiment, the device of the penile constriction system has an inferior height of about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.6 inches, about 0.25 inches to about 0.7 inches, about 0.25 inches to about 0.8 inches, about 0.25 inches to about 0.9 inches, about 0.25 inches to about 1 inch, about 0.3 inches to about 0.35 inches, about 0.3 inches to about 0.4 inches, about 0.3 inches to about 0.45 inches, about 0.3 inches to about 0.5 inches, about 0.3 inches to about 0.6 inches, about 0.3 inches to about 0.7 inches, about 0.3 inches to about 0.8 inches, about 0.3 inches to about 0.9 inches, about 0.3 inches to about 1 inch, about 0.35 inches to about 0.4 inches, about 0.35 inches to about 0.45 inches, about 0.35 inches to about 0.5 inches, about 0.35 inches to about 0.6 inches, about 0.35 inches to about 0.7 inches, about 0.35 inches to about 0.8 inches, about 0.35 inches to about 0.9 inches, about 0.35 inches to about 1 inch, about 0.4 inches to about 0.45 inches, about 0.4 inches to about 0.5 inches, about 0.4 inches to about 0.6 inches, about 0.4 inches to about 0.7 inches, about 0.4 inches to about 0.8 inches, about 0.4 inches to about 0.9 inches, about 0.4 inches to about 1 inch, about 0.45 inches to about 0.5 inches, about 0.45 inches to about 0.6 inches, about 0.45 inches to about 0.7 inches, about 0.45 inches to about 0.8 inches, about 0.45 inches to about 0.9 inches, about 0.45 inches to about 1 inch, about 0.5 inches to about 0.6 inches, about 0.5 inches to about 0.7 inches, about 0.5 inches to about 0.8 inches, about 0.5 inches to about 0.9 inches, about 0.5 inches to about 1 inch, about 0.6 inches to about 0.7 inches, about 0.6 inches to about 0.8 inches, about 0.6 inches to about 0.9 inches, about 0.6 inches to about 1 inch, about 0.7 inches to about 0.8 inches, about 0.7 inches to about 0.9 inches, about 0.7 inches to about 1 inch, about 0.8 inches to about 0.9 inches, about 0.8 inches to about 1 inch, or about 0.9 inches to about 1 inch. Optionally, in this or any other embodiment, the device of the penile constriction system has an inferior height of about 0.25 inches, about 0.3 inches, about 0.35 inches, about 0.4 inches, about 0.45 inches, about 0.5 inches, about 0.6 inches, about 0.7 inches, about 0.8 inches, about 0.9 inches, or about 1 inch. Optionally, in this or any other embodiment, the device of the penile constriction system has an inferior height of at least about 0.25 inches, about 0.3 inches, about 0.35 inches, about 0.4 inches, about 0.45 inches, about 0.5 inches, about 0.6 inches, about 0.7 inches, about 0.8 inches, about 0.9 inches, or about 1 inch. Optionally, in this or any other embodiment, the device of the penile constriction system has an inferior height of at most about 0.25 inches, about 0.3 inches, about 0.35 inches, about 0.4 inches, about 0.45 inches, about 0.5 inches, about 0.6 inches, about 0.7 inches, about 0.8 inches, about 0.9 inches, or about 1 inch.

Optionally, in this or any other embodiment, a ratio between the constriction gap width and the maximum inner width is about 0.3:1 to about 0.9:1. Optionally, in this or any other embodiment, a ratio between the constriction gap width and the maximum inner width is at least about 0.2:1. Optionally, in this or any other embodiment, a ratio between the constriction gap width and the maximum inner width is at most about 0.9:1. Optionally, in this or any other embodiment, a ratio between the constriction gap width and the maximum inner width is about 0.2:1 to about 0.25:1, about 0.2:1 to about 0.3:1, about 0.2:1 to about 0.35:1, about 0.2:1 to about 0.4:1, about 0.2:1 to about 0.5:1, about 0.2:1 to about 0.6:1, about 0.2:1 to about 0.7:1, about 0.2:1 to about 0.8:1, about 0.2:1 to about 0.9:1, about 0.25:1 to about 0.3:1, about 0.25:1 to about 0.35:1, about 0.25:1 to about 0.4:1, about 0.25:1 to about 0.5:1, about 0.25:1 to about 0.6:1, about 0.25:1 to about 0.7:1, about 0.25:1 to about 0.8:1, about 0.25:1 to about 0.9:1, about 0.3:1 to about 0.35:1, about 0.3:1 to about 0.4:1, about 0.3:1 to about 0.5:1, about 0.3:1 to about 0.6:1, about 0.3:1 to about 0.7:1, about 0.3:1 to about 0.8:1, about 0.3:1 to about 0.9:1, about 0.35:1 to about 0.4:1, about 0.35:1 to about 0.5:1, about 0.35:1 to about 0.6:1, about 0.35:1 to about 0.7:1, about 0.35:1 to about 0.8:1, about 0.35:1 to about 0.9:1, about 0.4:1 to about 0.5:1, about 0.4:1 to about 0.6:1, about 0.4:1 to about 0.7:1, about 0.4:1 to about 0.8:1, about 0.4:1 to about 0.9:1, about 0.5:1 to about 0.6:1, about 0.5:1 to about 0.7:1, about 0.5:1 to about 0.8:1, about 0.5:1 to about 0.9:1, about 0.6:1 to about 0.7:1, about 0.6:1 to about 0.8:1, about 0.6:1 to about 0.9:1, about 0.7:1 to about 0.8:1, about 0.7:1 to about 0.9:1, or about 0.8:1 to about 0.9:1. Optionally, in this or any other embodiment, a ratio between the constriction gap width and the maximum inner width is about 0.2:1, about 0.25:1, about 0.3:1, about 0.35:1, about 0.4:1, about 0.5:1, about 0.6:1, about 0.7:1, about 0.8:1, or about 0.9:1. Optionally, in this or any other embodiment, a ratio between the constriction gap width and the maximum inner width is at least about 0.2:1, about 0.25:1, about 0.3:1, about 0.35:1, about 0.4:1, about 0.5:1, about 0.6:1, about 0.7:1, about 0.8:1, or about 0.9:1. Optionally, in this or any other embodiment, a ratio between the constriction gap width and the maximum inner width is at most about 0.2:1, about 0.25:1, about 0.3:1, about 0.35:1, about 0.4:1, about 0.5:1, about 0.6:1, about 0.7:1, about 0.8:1, or about 0.9:1.

Optionally, in this or any other embodiment, a ratio between the constriction height and the maximum inner width is about 0.7:1 to about 0.99:1. Optionally, in this or any other embodiment, a ratio between the constriction height and the maximum inner width is at least about 0.7:1. Optionally, in this or any other embodiment, a ratio between the constriction height and the maximum inner width is at most about 0.99:1. Optionally, in this or any other embodiment, a ratio between the constriction height and the maximum inner width is about 0.7:1 to about 0.725:1, about 0.7:1 to about 0.75:1, about 0.7:1 to about 0.775:1, about 0.7:1 to about 0.8:1, about 0.7:1 to about 0.825:1, about 0.7:1 to about 0.85:1, about 0.7:1 to about 0.875:1, about 0.7:1 to about 0.9:1, about 0.7:1 to about 0.925:1, about 0.7:1 to about 0.95:1, about 0.7:1 to about 0.99:1, about 0.725:1 to about 0.75:1, about 0.725:1 to about 0.775:1, about 0.725:1 to about 0.8:1, about 0.725:1 to about 0.825:1, about 0.725:1 to about 0.85:1, about 0.725:1 to about 0.875:1, about 0.725:1 to about 0.9:1, about 0.725:1 to about 0.925:1, about 0.725:1 to about 0.95:1, about 0.725:1 to about 0.99:1, about 0.75:1 to about 0.775:1, about 0.75:1 to about 0.8:1, about 0.75:1 to about 0.825:1, about 0.75:1 to about 0.85:1, about 0.75:1 to about 0.875:1, about 0.75:1 to about 0.9:1, about 0.75:1 to about 0.925:1, about 0.75:1 to about 0.95:1, about 0.75:1 to about 0.99:1, about 0.775:1 to about 0.8:1, about 0.775:1 to about 0.825:1, about 0.775:1 to about 0.85:1, about 0.775:1 to about 0.875:1, about 0.775:1 to about 0.9:1, about 0.775:1 to about 0.925:1, about 0.775:1 to about 0.95:1, about 0.775:1 to about 0.99:1, about 0.8:1 to about 0.825:1, about 0.8:1 to about 0.85:1, about 0.8:1 to about 0.875:1, about 0.8:1 to about 0.9:1, about 0.8:1 to about 0.925:1, about 0.8:1 to about 0.95:1, about 0.8:1 to about 0.99:1, about 0.825:1 to about 0.85:1, about 0.825:1 to about 0.875:1, about 0.825:1 to about 0.9:1, about 0.825:1 to about 0.925:1, about 0.825:1 to about 0.95:1, about 0.825:1 to about 0.99:1, about 0.85:1 to about 0.875:1, about 0.85:1 to about 0.9:1, about 0.85:1 to about 0.925:1, about 0.85:1 to about 0.95:1, about 0.85:1 to about 0.99:1, about 0.875:1 to about 0.9:1, about 0.875:1 to about 0.925:1, about 0.875:1 to about 0.95:1, about 0.875:1 to about 0.99:1, about 0.9:1 to about 0.925:1, about 0.9:1 to about 0.95:1, about 0.9:1 to about 0.99:1, about 0.925:1 to about 0.95:1, about 0.925:1 to about 0.99:1, or about 0.95:1 to about 0.99:1. Optionally, in this or any other embodiment, a ratio between the constriction height and the maximum inner width is about 0.7:1, about 0.725:1, about 0.75:1, about 0.775:1, about 0.8:1, about 0.825:1, about 0.85:1, about 0.875:1, about 0.9:1, about 0.925:1, about 0.95:1, or about 0.99:1. Optionally, in this or any other embodiment, a ratio between the constriction height and the maximum inner width is at least about 0.7:1, about 0.725:1, about 0.75:1, about 0.775:1, about 0.8:1, about 0.825:1, about 0.85:1, about 0.875:1, about 0.9:1, about 0.925:1, about 0.95:1, or about 0.99:1. Optionally, in this or any other embodiment, a ratio between the constriction height and the maximum inner width is at most about 0.7:1, about 0.725:1, about 0.75:1, about 0.775:1, about 0.8:1, about 0.825:1, about 0.85:1, about 0.875:1, about 0.9:1, about 0.925:1, about 0.95:1, or about 0.99:1.

Optionally, in this or any other embodiment, a ratio between the inferior height and the maximum inner width is about 0.15:1 to about 0.6:1. Optionally, in this or any other embodiment, a ratio between the inferior height and the maximum inner width is at least about 0.15:1. Optionally, in this or any other embodiment, a ratio between the inferior height and the maximum inner width is at most about 0.6:1. Optionally, in this or any other embodiment, a ratio between the inferior height and the maximum inner width is about 0.15:1 to about 0.2:1, about 0.15:1 to about 0.25:1, about 0.15:1 to about 0.3:1, about 0.15:1 to about 0.35:1, about 0.15:1 to about 0.4:1, about 0.15:1 to about 0.45:1, about 0.15:1 to about 0.5:1, about 0.15:1 to about 0.55:1, about 0.15:1 to about 0.6:1, about 0.2:1 to about 0.25:1, about 0.2:1 to about 0.3:1, about 0.2:1 to about 0.35:1, about 0.2:1 to about 0.4:1, about 0.2:1 to about 0.45:1, about 0.2:1 to about 0.5:1, about 0.2:1 to about 0.55:1, about 0.2:1 to about 0.6:1, about 0.25:1 to about 0.3:1, about 0.25:1 to about 0.35:1, about 0.25:1 to about 0.4:1, about 0.25:1 to about 0.45:1, about 0.25:1 to about 0.5:1, about 0.25:1 to about 0.55:1, about 0.25:1 to about 0.6:1, about 0.3:1 to about 0.35:1, about 0.3:1 to about 0.4:1, about 0.3:1 to about 0.45:1, about 0.3:1 to about 0.5:1, about 0.3:1 to about 0.55:1, about 0.3:1 to about 0.6:1, about 0.35:1 to about 0.4:1, about 0.35:1 to about 0.45:1, about 0.35:1 to about 0.5:1, about 0.35:1 to about 0.55:1, about 0.35:1 to about 0.6:1, about 0.4:1 to about 0.45:1, about 0.4:1 to about 0.5:1, about 0.4:1 to about 0.55:1, about 0.4:1 to about 0.6:1, about 0.45:1 to about 0.5:1, about 0.45:1 to about 0.55:1, about 0.45:1 to about 0.6:1, about 0.5:1 to about 0.55:1, about 0.5:1 to about 0.6:1, or about 0.55:1 to about 0.6:1. Optionally, in this or any other embodiment, a ratio between the inferior height and the maximum inner width is about 0.15:1, about 0.2:1, about 0.25:1, about 0.3:1, about 0.35:1, about 0.4:1, about 0.45:1, about 0.5:1, about 0.55:1, or about 0.6:1. Optionally, in this or any other embodiment, a ratio between the inferior height and the maximum inner width is at least about 0.15:1, about 0.2:1, about 0.25:1, about 0.3:1, about 0.35:1, about 0.4:1, about 0.45:1, about 0.5:1, about 0.55:1, or about 0.6:1. Optionally, in this or any other embodiment, a ratio between the inferior height and the maximum inner width is at most 0.15:1, about 0.2:1, about 0.25:1, about 0.3:1, about 0.35:1, about 0.4:1, about 0.45:1, about 0.5:1, about 0.55:1, or about 0.6:1.

Optionally, in this or any other embodiment, the arched rod has a thickness, wherein the thickness is measured as a maximum normal cross-sectional width of at least a portion of at least one of the first arm, the second arm, and the bridge. Optionally, in this or any other embodiment, the device of the penile constriction system has a thickness of about 0.125 inches to about 0.8 inches. Optionally, in this or any other embodiment, the device of the penile constriction system has a thickness of at least about 0.125 inches. Optionally, in this or any other embodiment, the device of the penile constriction system has a thickness of at most about 0.8 inches. Optionally, in this or any other embodiment, the device of the penile constriction system has a thickness of about 0.125 inches to about 0.15 inches, about 0.125 inches to about 0.2 inches, about 0.125 inches to about 0.25 inches, about 0.125 inches to about 0.3 inches, about 0.125 inches to about 0.35 inches, about 0.125 inches to about 0.4 inches, about 0.125 inches to about 0.45 inches, about 0.125 inches to about 0.5 inches, about 0.125 inches to about 0.6 inches, about 0.125 inches to about 0.7 inches, about 0.125 inches to about 0.8 inches, about 0.15 inches to about 0.2 inches, about 0.15 inches to about 0.25 inches, about 0.15 inches to about 0.3 inches, about 0.15 inches to about 0.35 inches, about 0.15 inches to about 0.4 inches, about 0.15 inches to about 0.45 inches, about 0.15 inches to about 0.5 inches, about 0.15 inches to about 0.6 inches, about 0.15 inches to about 0.7 inches, about 0.15 inches to about 0.8 inches, about 0.2 inches to about 0.25 inches, about 0.2 inches to about 0.3 inches, about 0.2 inches to about 0.35 inches, about 0.2 inches to about 0.4 inches, about 0.2 inches to about 0.45 inches, about 0.2 inches to about 0.5 inches, about 0.2 inches to about 0.6 inches, about 0.2 inches to about 0.7 inches, about 0.2 inches to about 0.8 inches, about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.6 inches, about 0.25 inches to about 0.7 inches, about 0.25 inches to about 0.8 inches, about 0.3 inches to about 0.35 inches, about 0.3 inches to about 0.4 inches, about 0.3 inches to about 0.45 inches, about 0.3 inches to about 0.5 inches, about 0.3 inches to about 0.6 inches, about 0.3 inches to about 0.7 inches, about 0.3 inches to about 0.8 inches, about 0.35 inches to about 0.4 inches, about 0.35 inches to about 0.45 inches, about 0.35 inches to about 0.5 inches, about 0.35 inches to about 0.6 inches, about 0.35 inches to about 0.7 inches, about 0.35 inches to about 0.8 inches, about 0.4 inches to about 0.45 inches, about 0.4 inches to about 0.5 inches, about 0.4 inches to about 0.6 inches, about 0.4 inches to about 0.7 inches, about 0.4 inches to about 0.8 inches, about 0.45 inches to about 0.5 inches, about 0.45 inches to about 0.6 inches, about 0.45 inches to about 0.7 inches, about 0.45 inches to about 0.8 inches, about 0.5 inches to about 0.6 inches, about 0.5 inches to about 0.7 inches, about 0.5 inches to about 0.8 inches, about 0.6 inches to about 0.7 inches, about 0.6 inches to about 0.8 inches, or about 0.7 inches to about 0.8 inches. Optionally, in this or any other embodiment, the device of the penile constriction system has a thickness of about 0.125 inches, about 0.15 inches, about 0.2 inches, about 0.25 inches, about 0.3 inches, about 0.35 inches, about 0.4 inches, about 0.45 inches, about 0.5 inches, about 0.6 inches, about 0.7 inches, or about 0.8 inches. Optionally, in this or any other embodiment, the device of the penile constriction system has a thickness of at least about 0.125 inches, about 0.15 inches, about 0.2 inches, about 0.25 inches, about 0.3 inches, about 0.35 inches, about 0.4 inches, about 0.45 inches, about 0.5 inches, about 0.6 inches, about 0.7 inches, or about 0.8 inches. Optionally, in this or any other embodiment, the device of the penile constriction system has a thickness of at most about 0.125 inches, about 0.15 inches, about 0.2 inches, about 0.25 inches, about 0.3 inches, about 0.35 inches, about 0.4 inches, about 0.45 inches, about 0.5 inches, about 0.6 inches, about 0.7 inches, or about 0.8 inches.

Optionally, in this or any other embodiment, the first terminus diverges from the constriction region to form a first elbow, and wherein the second terminus diverges from the constriction region to form a second elbow. Optionally, in this or any other embodiment, the first elbow and the second elbow are configured to removably affix to a band.

Optionally, in this or any other embodiment, the compression region comprises: a first dorsal vein protrusion comprising: a first dorsal apex; and a first dorsal height measured as a first minimum normal distance between the first dorsal apex and a first lateral edge of the first dorsal vein protrusion where the first dorsal vein protrusion rises from the inferior side of the bridge; a second dorsal vein protrusion comprising: a second dorsal apex; and a second dorsal height measured as a second minimum normal distance between the second dorsal apex and a second lateral edge of the second dorsal vein protrusion where the second dorsal vein protrusion rises from the inferior side of the bridge; and a dorsal vein valley between the first dorsal vein apex and the second dorsal vein apex.

Optionally, in this or any other embodiment, the first dorsal vein protrusion, the dorsal vein valley, and the second dorsal vein protrusion are arranged sequentially within the compression region in a direction along the bridge from the first arm to the second arm.

Optionally, in this or any other embodiment, the device of the penile constriction system comprises a dorsal vein valley distance between the first dorsal apex and the second dorsal apex. Optionally, in this or any other embodiment, the dorsal vein valley distance is about 0.15 inches to about 0.75 inches. Optionally, in this or any other embodiment, the dorsal vein valley distance is at least about 0.15 inches. Optionally, in this or any other embodiment, the dorsal vein valley distance is at most about 0.75 inches. Optionally, in this or any other embodiment, the dorsal vein valley distance is about 0.15 inches to about 0.2 inches, about 0.15 inches to about 0.25 inches, about 0.15 inches to about 0.3 inches, about 0.15 inches to about 0.35 inches, about 0.15 inches to about 0.4 inches, about 0.15 inches to about 0.45 inches, about 0.15 inches to about 0.5 inches, about 0.15 inches to about 0.55 inches, about 0.15 inches to about 0.6 inches, about 0.15 inches to about 0.65 inches, about 0.15 inches to about 0.75 inches, about 0.2 inches to about 0.25 inches, about 0.2 inches to about 0.3 inches, about 0.2 inches to about 0.35 inches, about 0.2 inches to about 0.4 inches, about 0.2 inches to about 0.45 inches, about 0.2 inches to about 0.5 inches, about 0.2 inches to about 0.55 inches, about 0.2 inches to about 0.6 inches, about 0.2 inches to about 0.65 inches, about 0.2 inches to about 0.75 inches, about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.55 inches, about 0.25 inches to about 0.6 inches, about 0.25 inches to about 0.65 inches, about 0.25 inches to about 0.75 inches, about 0.3 inches to about 0.35 inches, about 0.3 inches to about 0.4 inches, about 0.3 inches to about 0.45 inches, about 0.3 inches to about 0.5 inches, about 0.3 inches to about 0.55 inches, about 0.3 inches to about 0.6 inches, about 0.3 inches to about 0.65 inches, about 0.3 inches to about 0.75 inches, about 0.35 inches to about 0.4 inches, about 0.35 inches to about 0.45 inches, about 0.35 inches to about 0.5 inches, about 0.35 inches to about 0.55 inches, about 0.35 inches to about 0.6 inches, about 0.35 inches to about 0.65 inches, about 0.35 inches to about 0.75 inches, about 0.4 inches to about 0.45 inches, about 0.4 inches to about 0.5 inches, about 0.4 inches to about 0.55 inches, about 0.4 inches to about 0.6 inches, about 0.4 inches to about 0.65 inches, about 0.4 inches to about 0.75 inches, about 0.45 inches to about 0.5 inches, about 0.45 inches to about 0.55 inches, about 0.45 inches to about 0.6 inches, about 0.45 inches to about 0.65 inches, about 0.45 inches to about 0.75 inches, about 0.5 inches to about 0.55 inches, about 0.5 inches to about 0.6 inches, about 0.5 inches to about 0.65 inches, about 0.5 inches to about 0.75 inches, about 0.55 inches to about 0.6 inches, about 0.55 inches to about 0.65 inches, about 0.55 inches to about 0.75 inches, about 0.6 inches to about 0.65 inches, about 0.6 inches to about 0.75 inches, or about 0.65 inches to about 0.75 inches. Optionally, in this or any other embodiment, the dorsal vein valley distance is about 0.15 inches, about 0.2 inches, about 0.25 inches, about 0.3 inches, about 0.35 inches, about 0.4 inches, about 0.45 inches, about 0.5 inches, about 0.55 inches, about 0.6 inches, about 0.65 inches, or about 0.75 inches. Optionally, in this or any other embodiment, the dorsal vein valley distance is at least about 0.15 inches, about 0.2 inches, about 0.25 inches, about 0.3 inches, about 0.35 inches, about 0.4 inches, about 0.45 inches, about 0.5 inches, about 0.55 inches, about 0.6 inches, about 0.65 inches, or about 0.75 inches. Optionally, in this or any other embodiment, the dorsal vein valley distance is at most about 0.15 inches, about 0.2 inches, about 0.25 inches, about 0.3 inches, about 0.35 inches, about 0.4 inches, about 0.45 inches, about 0.5 inches, about 0.55 inches, about 0.6 inches, about 0.65 inches, or about 0.75 inches.

Optionally, in this or any other embodiment, the device of the penile constriction system comprises a compression region width between the first lateral edge and the second lateral edge. Optionally, in this or any other embodiment, the compression region width is about 0.2 inches to about 0.8 inches. Optionally, in this or any other embodiment, the compression region width is at least about 0.2 inches. Optionally, in this or any other embodiment, the compression region width is at most about 0.8 inches. Optionally, in this or any other embodiment, the compression region width is about 0.2 inches to about 0.25 inches, about 0.2 inches to about 0.3 inches, about 0.2 inches to about 0.35 inches, about 0.2 inches to about 0.4 inches, about 0.2 inches to about 0.45 inches, about 0.2 inches to about 0.5 inches, about 0.2 inches to about 0.55 inches, about 0.2 inches to about 0.6 inches, about 0.2 inches to about 0.7 inches, about 0.2 inches to about 0.8 inches, about 0.25 inches to about 0.3 inches, about 0.25 inches to about 0.35 inches, about 0.25 inches to about 0.4 inches, about 0.25 inches to about 0.45 inches, about 0.25 inches to about 0.5 inches, about 0.25 inches to about 0.55 inches, about 0.25 inches to about 0.6 inches, about 0.25 inches to about 0.7 inches, about 0.25 inches to about 0.8 inches, about 0.3 inches to about 0.35 inches, about 0.3 inches to about 0.4 inches, about 0.3 inches to about 0.45 inches, about 0.3 inches to about 0.5 inches, about 0.3 inches to about 0.55 inches, about 0.3 inches to about 0.6 inches, about 0.3 inches to about 0.7 inches, about 0.3 inches to about 0.8 inches, about 0.35 inches to about 0.4 inches, about 0.35 inches to about 0.45 inches, about 0.35 inches to about 0.5 inches, about 0.35 inches to about 0.55 inches, about 0.35 inches to about 0.6 inches, about 0.35 inches to about 0.7 inches, about 0.35 inches to about 0.8 inches, about 0.4 inches to about 0.45 inches, about 0.4 inches to about 0.5 inches, about 0.4 inches to about 0.55 inches, about 0.4 inches to about 0.6 inches, about 0.4 inches to about 0.7 inches, about 0.4 inches to about 0.8 inches, about 0.45 inches to about 0.5 inches, about 0.45 inches to about 0.55 inches, about 0.45 inches to about 0.6 inches, about 0.45 inches to about 0.7 inches, about 0.45 inches to about 0.8 inches, about 0.5 inches to about 0.55 inches, about 0.5 inches to about 0.6 inches, about 0.5 inches to about 0.7 inches, about 0.5 inches to about 0.8 inches, about 0.55 inches to about 0.6 inches, about 0.55 inches to about 0.7 inches, about 0.55 inches to about 0.8 inches, about 0.6 inches to about 0.7 inches, about 0.6 inches to about 0.8 inches, or about 0.7 inches to about 0.8 inches. Optionally, in this or any other embodiment, the compression region width is about 0.2 inches, about 0.25 inches, about 0.3 inches, about 0.35 inches, about 0.4 inches, about 0.45 inches, about 0.5 inches, about 0.55 inches, about 0.6 inches, about 0.7 inches, or about 0.8 inches. Optionally, in this or any other embodiment, the compression region width is at least about 0.2 inches, about 0.25 inches, about 0.3 inches, about 0.35 inches, about 0.4 inches, about 0.45 inches, about 0.5 inches, about 0.55 inches, about 0.6 inches, about 0.7 inches, or about 0.8 inches. Optionally, in this or any other embodiment, the compression region width is at most about 0.2 inches, about 0.25 inches, about 0.3 inches, about 0.35 inches, about 0.4 inches, about 0.45 inches, about 0.5 inches, about 0.55 inches, about 0.6 inches, about 0.7 inches, or about 0.8 inches.

Optionally, in this or any other embodiment, the compression region within the dorsal vein valley at a center of the dorsal vein valley is superior to or has a same height relative to the first lateral edge as the first minimum normal distance or relative to the second lateral edge as the second minimum normal distance, or both. Optionally, in this or any other embodiment, the compression region at the center of the dorsal vein valley is inferior to the first lateral edge, the second lateral edge, or both.

Optionally, in this or any other embodiment, the device of the penile constriction system further comprises a plurality of friction protrusions on a superior side of the bridge. Optionally, in this or any other embodiment, the plurality of friction protrusions comprises about 2 protrusions to about 60 protrusions. Optionally, in this or any other embodiment, the plurality of friction protrusions comprises at least about 2 protrusions. Optionally, in this or any other embodiment, the plurality of friction protrusions comprises at most about 60 protrusions. Optionally, in this or any other embodiment, the plurality of friction protrusions comprises about 2 protrusions to about 4 protrusions, about 2 protrusions to about 6 protrusions, about 2 protrusions to about 8 protrusions, about 2 protrusions to about 10 protrusions, about 2 protrusions to about 15 protrusions, about 2 protrusions to about 20 protrusions, about 2 protrusions to about 25 protrusions, about 2 protrusions to about 30 protrusions, about 2 protrusions to about 40 protrusions, about 2 protrusions to about 50 protrusions, about 2 protrusions to about 60 protrusions, about 4 protrusions to about 6 protrusions, about 4 protrusions to about 8 protrusions, about 4 protrusions to about 10 protrusions, about 4 protrusions to about 15 protrusions, about 4 protrusions to about 20 protrusions, about 4 protrusions to about 25 protrusions, about 4 protrusions to about 30 protrusions, about 4 protrusions to about 40 protrusions, about 4 protrusions to about 50 protrusions, about 4 protrusions to about 60 protrusions, about 6 protrusions to about 8 protrusions, about 6 protrusions to about 10 protrusions, about 6 protrusions to about 15 protrusions, about 6 protrusions to about 20 protrusions, about 6 protrusions to about 25 protrusions, about 6 protrusions to about 30 protrusions, about 6 protrusions to about 40 protrusions, about 6 protrusions to about 50 protrusions, about 6 protrusions to about 60 protrusions, about 8 protrusions to about 10 protrusions, about 8 protrusions to about 15 protrusions, about 8 protrusions to about 20 protrusions, about 8 protrusions to about 25 protrusions, about 8 protrusions to about 30 protrusions, about 8 protrusions to about 40 protrusions, about 8 protrusions to about 50 protrusions, about 8 protrusions to about 60 protrusions, about 10 protrusions to about 15 protrusions, about 10 protrusions to about 20 protrusions, about 10 protrusions to about 25 protrusions, about 10 protrusions to about 30 protrusions, about 10 protrusions to about 40 protrusions, about 10 protrusions to about 50 protrusions, about 10 protrusions to about 60 protrusions, about 15 protrusions to about 20 protrusions, about 15 protrusions to about 25 protrusions, about 15 protrusions to about 30 protrusions, about 15 protrusions to about 40 protrusions, about 15 protrusions to about 50 protrusions, about 15 protrusions to about 60 protrusions, about 20 protrusions to about 25 protrusions, about 20 protrusions to about 30 protrusions, about 20 protrusions to about 40 protrusions, about 20 protrusions to about 50 protrusions, about 20 protrusions to about 60 protrusions, about 25 protrusions to about 30 protrusions, about 25 protrusions to about 40 protrusions, about 25 protrusions to about 50 protrusions, about 25 protrusions to about 60 protrusions, about 30 protrusions to about 40 protrusions, about 30 protrusions to about 50 protrusions, about 30 protrusions to about 60 protrusions, about 40 protrusions to about 50 protrusions, about 40 protrusions to about 60 protrusions, or about 50 protrusions to about 60 protrusions. Optionally, in this or any other embodiment, the plurality of friction protrusions comprises about 2 protrusions, about 4 protrusions, about 6 protrusions, about 8 protrusions, about 10 protrusions, about 15 protrusions, about 20 protrusions, about 25 protrusions, about 30 protrusions, about 40 protrusions, about 50 protrusions, or about 60 protrusions. Optionally, in this or any other embodiment, the plurality of friction protrusions comprises at most about 2 protrusions, about 4 protrusions, about 6 protrusions, about 8 protrusions, about 10 protrusions, about 15 protrusions, about 20 protrusions, about 25 protrusions, about 30 protrusions, about 40 protrusions, about 50 protrusions, or about 60 protrusions. Optionally, in this or any other embodiment, the plurality of friction protrusions comprises at least about 2 protrusions, about 4 protrusions, about 6 protrusions, about 8 protrusions, about 10 protrusions, about 15 protrusions, about 20 protrusions, about 25 protrusions, about 30 protrusions, about 40 protrusions, about 50 protrusions, or about 60 protrusions.

Optionally, in this or any other embodiment, the friction protrusions have a height of about 0.01 inches to about 0.06 inches. Optionally, in this or any other embodiment, the friction protrusions have a height of about 0.01 inches to about 0.02 inches, about 0.01 inches to about 0.03 inches, about 0.01 inches to about 0.04 inches, about 0.01 inches to about 0.05 inches, about 0.01 inches to about 0.06 inches, about 0.02 inches to about 0.03 inches, about 0.02 inches to about 0.04 inches, about 0.02 inches to about 0.05 inches, about 0.02 inches to about 0.06 inches, about 0.03 inches to about 0.04 inches, about 0.03 inches to about 0.05 inches, about 0.03 inches to about 0.06 inches, about 0.04 inches to about 0.05 inches, about 0.04 inches to about 0.06 inches, or about 0.05 inches to about 0.06 inches. Optionally, in this or any other embodiment, the friction protrusions have a height of about 0.01 inches, about 0.02 inches, about 0.03 inches, about 0.04 inches, about 0.05 inches, or about 0.06 inches. Optionally, in this or any other embodiment, the friction protrusions have a height of at least about 0.01 inches, about 0.02 inches, about 0.03 inches, about 0.04 inches, or about 0.05 inches. Optionally, in this or any other embodiment, the friction protrusions have a height of at most about 0.02 inches, about 0.03 inches, about 0.04 inches, about 0.05 inches, or about 0.06 inches.

Optionally, in this or any other embodiment, the friction protrusions have a width of about 0.01 inches to about 0.06 inches. Optionally, in this or any other embodiment, the friction protrusions have a width of about 0.01 inches to about 0.02 inches, about 0.01 inches to about 0.03 inches, about 0.01 inches to about 0.04 inches, about 0.01 inches to about 0.05 inches, about 0.01 inches to about 0.06 inches, about 0.02 inches to about 0.03 inches, about 0.02 inches to about 0.04 inches, about 0.02 inches to about 0.05 inches, about 0.02 inches to about 0.06 inches, about 0.03 inches to about 0.04 inches, about 0.03 inches to about 0.05 inches, about 0.03 inches to about 0.06 inches, about 0.04 inches to about 0.05 inches, about 0.04 inches to about 0.06 inches, or about 0.05 inches to about 0.06 inches. Optionally, in this or any other embodiment, the friction protrusions have a width of about 0.01 inches, about 0.02 inches, about 0.03 inches, about 0.04 inches, about 0.05 inches, or about 0.06 inches. Optionally, in this or any other embodiment, the friction protrusions have a width of at least about 0.01 inches, about 0.02 inches, about 0.03 inches, about 0.04 inches, or about 0.05 inches. Optionally, in this or any other embodiment, the friction protrusions have a width of at most about 0.02 inches, about 0.03 inches, about 0.04 inches, about 0.05 inches, or about 0.06 inches.

Optionally, in this or any other embodiment, the arched rod comprises a cover substantially surrounding a frame, wherein a frame modulus of elasticity of the frame is higher than a cover modulus of elasticity of the cover.

Another aspect provided herein is a method of treating an ailment in a patient, the method comprising: wearing the penile constriction device to promote intercourse and/or masturbation; maintaining an erection; and engaging in sexual intercourse, masturbation, or both with the worn penile constriction device, wherein engaging in sexual intercourse, masturbation, or both with the worn penile constriction device treats the ailment by facilitating ejaculation and/or orgasm, releasing endorphins, oxytocin, and dopamine, and reducing cortisol levels, facilitating sexual activity, or any combination thereof. In some examples, the ailment comprises anxiety, depression, post-traumatic stress disorder, low self-esteem, seasonal affective disorder (SAD), low testosterone, prostate cancer, hypertension, coronary artery disease, stress, ED, prostate cancer, hypertension, coronary artery disease, diabetes, heart disease, high blood pressure, low blood pressure, coronary artery disease (CAD), COPD, hypertension, headache, migraines, obesity, or any combination thereof. Further provided herein are methods, devices, and systems that enable improved family planning and/or sperm donation. In some examples, the methods, devices, and systems herein enable improved family planning and/or sperm donation.

Another aspect provided herein is a method of reducing the required intake of a medication, the method comprising: wearing the penile constriction device to promote intercourse; maintaining an erection; and engaging in sexual intercourse, masturbation, or both with the worn penile constriction device, wherein engaging in sexual intercourse, masturbation, or both with the worn penile constriction device sexual intercourse, masturbation, or both reduces the required intake of a medication by facilitating ejaculation and/or orgasm, releasing endorphins, oxytocin, serotonin, and dopamine, and reducing cortisol levels, facilitating sexual activity, or any combination thereof. In some examples, the medication comprises an anti-anxiety medication, an anti-depression medication, a pain medication, an opioid, a heart medication, a blood-pressure medication, a cholesterol medication, or any combination thereof.

The invention provided herein is a method of selecting a penile constriction device for an individual, the method comprising: providing a measurement device having color coded demarcations, each demarcation indicating a size of the penile constriction device; measuring the individual's penis using the measurement device; and selecting the penile constriction device based on the demarcation.

Another aspect of the invention provided herein is a measurement device for sizing a penile constriction device comprising color-coded demarcations, each demarcation indicating a size of the penile constriction device.

Another aspect provided herein is a penile constriction device comprising one or more tactile size indications to indicate size. In some embodiments, the tactile size indication is concave relative to a surface of the device. In some embodiments, the tactile size indication is convex relative to a surface of the device.

Another aspect provided herein is a method of improving the efficacy of a penile constriction device, the method comprising: wearing the penile constriction device to promote sexual intercourse, masturbation, or both; determining a performance issue during the wearing of the penile constriction device; determining a remedy based on a troubleshooting guide; performing the remedy; and wearing the penile constriction device to promote sexual intercourse, masturbation, or both. In some examples, the performance issue comprises no erection, partial erection, rapid loss of erection, delayed loss of erection, pulling of the skin of the scrotum, discomfort or pain while donning or wearing the penile constriction device, discomfort or pain during intercourse, discomfort/pain during ejaculation, or any combination thereof. In some examples, the remedy comprises adding lubrication, replacing a band of the penile constriction device, using a smaller size of the penile constriction device, using a larger size of the penile constriction device, or any combination thereof.

Another aspect provided herein is a kit comprising: a penile constriction device; and a troubleshooting guide.

These and other examples are described in further detail in the following description related to the appended drawing figures.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:
**FIG.** 1 shows a perspective view of a non-limiting example of a first penile constriction device, per an embodiment herein;
**FIG. 2A** shows a front view of a non-limiting example of the penile constriction device of **FIG. 1****;**
**FIG. 2B** shows a side cross-sectional view of a non-limiting example of the penile constriction device of **FIG. 1****;**
**FIG. 2C** shows an alternative front view of a non-limiting example of the penile constriction device of **FIG. 1****;**
**FIG. 3A** shows a detailed front view of the compression region of a non-limiting example of the penile constriction device of **FIG. 1****;**
**FIG. 3B** shows a detailed bottom view of the compression region of a non-limiting example of the penile constriction device of **FIG. 1****;**
**FIG. 4** shows a top view of the friction protrusions of a non-limiting example of the penile constriction device of **FIG. 1****;**
**FIG. 5** shows a side cross-sectioned view of the friction protrusions and compression region of the penile constriction device of **FIG. 1****;**
**FIG. 6** shows a front view of a non-limiting example of a second penile constriction device, per an embodiment here;
**FIG. 7** shows a front view of a non-limiting example of a third penile constriction device, per an embodiment herein;
**FIG. 8** shows a front view of a non-limiting example of a fourth penile constriction device, per an embodiment herein;
**FIG. 9** shows a front view of a non-limiting example of a fifth penile constriction device, per an embodiment herein;
**FIG. 10A** shows a front cross-sectioned view of a non-limiting example of a penile constriction device comprising a frame and a cover, per an embodiment herein;
**FIG. 10B** shows a perspective view of a non-limiting example of the frame of **FIG. 10A****;**
**FIG. 10C** shows a front view of a non-limiting example of the frame of **FIG. 10A****;**
**FIG. 11A** shows a front cross-sectioned view of an alternative non-limiting example of a penile constriction device comprising a frame and a cover, per an embodiment herein;
**FIG. 11B** shows a perspective view of the manufacturing marks of penile constriction device of **FIG. 11A****;**
**FIG. 11C** shows a front view of the manufacturing marks of penile constriction device of **FIG. 11A****;**
**FIG. 11D** shows a back view of the manufacturing marks of penile constriction device of **FIG. 11A****;**
**FIG. 11E** shows a top view of the manufacturing marks of penile constriction device of **FIG. 11A****;**
**FIG. 11F** shows a bottom view of the manufacturing marks of penile constriction device of **FIG. 11A****;**
**FIG. 11G** shows a side view of the manufacturing marks of penile constriction device of **FIG. 11A****;**
**FIG. 11H** shows a side cross-sectioned view of the manufacturing marks of penile constriction device of **FIG. 11A****;**
**FIG. 11J** shows a perspective view of the frame of a non-limiting example of the frame of **FIG. 11A****;**
**FIG. 11K** shows a front view of the frame of a non-limiting example of the frame of **FIG. 11A****;**
**FIG. 11L** shows a front view of the frame of a non-limiting example of the frame of **FIG. 11A****;**
**FIG. 12A** shows a front cross-sectioned view of another alternative non-limiting example of a penile constriction device comprising a frame and a cover, per an embodiment herein;
**FIG. 12B** shows a perspective view of the manufacturing marks of penile constriction device of **FIG. 12A****;**
**FIG. 12C** shows a front view of the manufacturing marks of penile constriction device of **FIG. 12A****;**
**FIG. 12D** shows a back view of the manufacturing marks of penile constriction device of **FIG. 12A****;**
**FIG. 12E** shows a top view of the manufacturing marks of penile constriction device of **FIG. 12A****;**
**FIG. 12F** shows a bottom view of the manufacturing marks of penile constriction device of **FIG. 12A****;**
**FIG. 12G** shows a side view of the manufacturing marks of penile constriction device of **FIG. 12A****;**
**FIG. 12H** shows a side cross-sectioned view of the manufacturing marks of penile constriction device of **FIG. 12A****;**
**FIG. 12J** shows a perspective view of the frame of a non-limiting example of the frame of **FIG. 12A****;**
**FIG. 12K** shows a front view of the frame of a non-limiting example of the frame of **FIG. 12A****;**
**FIG. 12L** shows a front view of the frame of a non-limiting example of the frame of **FIG. 12A****;**
**FIG. 13** shows a front cross-sectioned view of the veins and arteries in a human penis;
**FIG. 14** shows a front cross-sectioned view of a non-limiting example of a penile constriction device on a human penis, per an embodiment herein;
**FIG. 15A** shows a front view of a non-limiting example of a penile constriction system comprising an exemplary penile constriction device and a band, per an embodiment herein;
**FIG. 15B** shows a front view of a non-limiting example of a penile constriction system comprising an exemplary penile constriction device and a high tension band, per an embodiment herein;
**FIG. 16A** shows a perspective view of a non-limiting example of a penile constriction device on a flaccid human penis, per an embodiment herein;
**FIG. 16B** shows a perspective view of a non-limiting example of a penile constriction device and a band on a flaccid human penis, per an embodiment herein;
**FIG. 16C** shows a perspective view of a non-limiting example of a penile constriction device and a band on an erect human penis, per an embodiment herein;
**FIG. 17** shows a front view of a penile constriction device in the prior art;
**FIG. 18** shows a front cross-sectioned view of the prior art penile constriction device in **FIG. 17** on a human penis;
**FIG. 19** shows a front view of a non-limiting example of a penile constriction device having friction protrusions but no first dorsal vein protrusion or second dorsal vein protrusion; per an embodiment herein;
**FIG. 20** shows a front view of a non-limiting example of a penile constriction device having the first dorsal vein protrusion and second dorsal vein protrusion, but no friction protrusions; per an embodiment herein;
**FIG. 21** shows a front view of a non-limiting example of a penile constriction device having no first dorsal vein protrusion, second dorsal vein protrusion, or friction protrusions; per an embodiment herein; and
**FIG. 22** shows a front view of a non-limiting example of a penile constriction device having a manufacturing mark; per an embodiment herein;
**FIG. 23** shows a top view image of the penile constriction device of **FIG. 22****;** per an embodiment herein;
**FIG. 24** shows a right side cross-sectioned view image of the penile constriction device of **FIG. 22****;** per an embodiment herein;
**FIG. 25** shows a first exemplary exercise guide; per an example herein;
**FIG. 26** shows a second exemplary exercise guide; per an example herein;
**FIG. 27** shows a third exemplary exercise guide; per an example herein;
**FIG. 28** shows a fourth exemplary exercise guide; per an example herein;
**FIG. 29** shows a fifth exemplary exercise guide; per an example herein;
**FIG. 30** shows a sixth exemplary exercise guide; per an example herein;
**FIG. 31** shows a first page of an exemplary instruction booklet; per an example herein;
**FIG. 32** shows a second page of an exemplary instruction booklet; per an example herein;
**FIG. 33** shows a third page of an exemplary instruction booklet; per an example herein;
**FIG. 34** shows a fourth page of an exemplary instruction booklet; per an example herein;
**FIG. 35** shows a fifth page of an exemplary instruction booklet; per an example herein;
**FIG. 36** shows a sixth page of an exemplary instruction booklet; per an example herein;
**FIG. 37** shows a seventh page of an exemplary instruction booklet; per an example herein;
**FIG. 38** shows an eighth page of an exemplary instruction booklet; per an example herein;
**FIG. 39** shows a ninth page of an exemplary instruction booklet; per an example herein;
**FIG. 40** shows a tenth page of an exemplary instruction booklet; per an example herein;
**FIG. 41** shows an eleventh page of an exemplary instruction booklet; per an example herein;
**FIG. 42** shows a twelfth page of an exemplary instruction booklet; per an example herein;
**FIG. 43** shows a thirteenth page of an exemplary instruction booklet; per an example herein;
**FIG. 44** shows a fourteenth page of an exemplary instruction booklet; per an example herein;
**FIG. 45** shows a fifteenth page of an exemplary instruction booklet; per an example herein;
**FIG. 46** shows a sixteenth page of an exemplary instruction booklet; per an example herein.
**FIG. 47** shows a perspective image of a non-limiting example of a first size of a primary band of the penile constriction device herein, per an embodiment herein;
**FIG. 48** shows a perspective view image of a non-limiting example of a third size of a primary band of the penile constriction device herein, per an embodiment herein;
**FIG. 49** shows a perspective view illustration of a non-limiting example of a primary band of the penile constriction device herein, per an embodiment herein;
**FIG. 50A** shows a top view illustration of a non-limiting example of a primary band of the penile constriction device herein, per an embodiment herein;
**FIG. 50B** shows a front view illustration of a non-limiting example of a primary band of the penile constriction device herein, per an embodiment herein;
**FIG. 51** shows a perspective view illustration of a non-limiting example of a secondary band of the penile constriction device herein, per an embodiment herein; and
**FIG. 52** shows a front view illustration of a non-limiting example of a secondary band of the penile constriction device herein, per an embodiment herein.

### DETAILED DESCRIPTION OF THE INVENTION

Specific embodiments of the disclosed system, method, and device will now be described with reference to the drawings. Nothing in this detailed description is intended to imply that any particular component, feature, or step is essential to the invention.

### Penile Constriction Devices

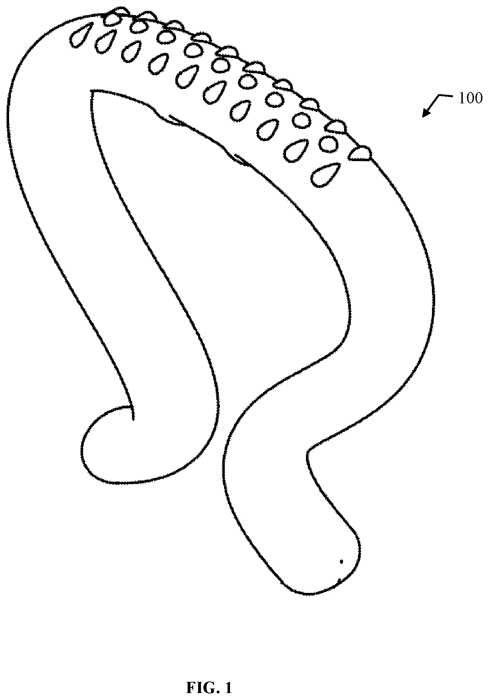

Provided herein, per **FIGS. 1-12****,** are exemplary embodiments of a penile constriction device. **FIG. 1** shows a perspective view of a non-limiting example of a first penile constriction device **100.**

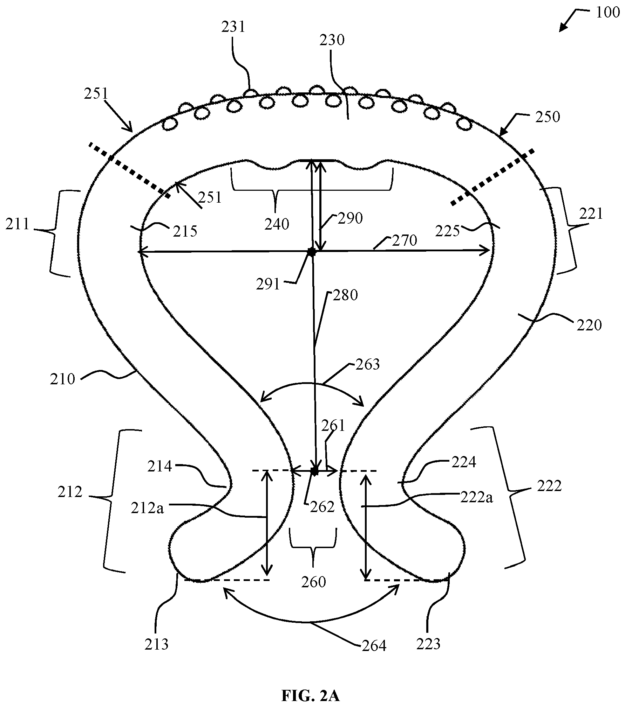

**FIG. 2A** shows a front view of a non-limiting example of the penile constriction device of **FIG. 1****.** The device **100** comprises an arched rod **250** comprising a first arm **210,** a second arm **210,** and a bridge **230.** The first arm **210** comprises at least one of a first coupling end **211** and a first converging end **212.** The first converging end **212** comprises a first terminus **213.** Further, the second arm **220** comprises at least one of a second coupling end **221** and a second converging end **222.** The second converging **222** end has a second terminus **223.** Optionally, in this and other embodiments, the second terminus **223** is disconnected from the first terminus **213.** In other embodiments, the second terminus may be connected with the first terminus. In the present embodiment, the first converging end **212** and the second converging end **222** converge to form a constriction region **260.** The bridge **230** is generally disposed between the first arm **210** and the second arm **220.** Additionally, the bridge **230** connects the first coupling end **211** to the second coupling end **221.** Optionally, in this and other embodiments, the bridge **230** comprises a compression region **240.** The compression region **240** rises inferiorly from an inferior side of the bridge **230.** Optionally, in some embodiments, the device does not comprise the compression region. The bridge **230** further comprises a plurality of friction protrusions **231** on a superior side of the bridge **230.** Optionally, in other embodiments, the device does not comprise a plurality of friction protrusions

**FIG. 2A** portrays the arched rod **250,** which optionally may comprise a curvilinear rod. Optionally, in this or any other embodiment, at least one of the first arm **210** and the second arm **220** is S-shaped. Alternatively, in this or any other embodiment, at least one of the first arm **210** and the second arm **220** is optionally C-shaped, J-shaped, L-shaped, U-shaped, or V-shaped. Optionally, in this or any other embodiment, the arched rod **250** has the shape of the Greek capital letter omega (Ω). Optionally, in this or any other embodiment, the arched rod **250** is U-shaped. In some embodiments, the first terminus **213** extends beyond the second terminus **223.** In some embodiments, the second terminus **223** extends beyond the first terminus **213.** In some embodiments, the first terminus **213** and the second terminus **223** have the same shape. In some embodiments, the first terminus **213** and the second terminus **223** have the different shapes. As shown in **FIG. 2A****,** the first terminus **213** and the second terminus **223** can be rounded. Alternatively, in some embodiments, at least one of the first terminus **213** and the second terminus **223** are tapered, pointed, twisted, pinched, flat, or have any other geometrical shape. In some embodiments, the arms and terminus connect so the penile constriction device is an overall oval shape.

The first arm **210** and the second arm **220** extend from opposing ends of the bridge **230.** Optionally, in this or any other embodiment, at least one of the first arm **210** and the second arm **220** extends radially and transversely from the bridge **230.** Optionally, in this or any other embodiment, at least one of the first arm **210** and the second arm **220** extend downward relative to the superior side of the bridge **230.** Optionally, in this or any other embodiment, at least one of the first arm **210** and the second arm **220** extend radially and transversely downward relative to the superior side of the bridge **230.** Optionally, in this or any other embodiment, at least one of the first arm **210** below the first coupling end **211** and the second arm **220** extends radially and mesially below the second coupling end **221.** Optionally, in this or any other embodiment, at least one of the first arm **210** below the first coupling end **211** and the second arm **220** extends radially and medially below the second coupling end **221.** Optionally, in this or any other embodiment, at least one of the first arm **210** below the first coupling end **211** and the second arm **220** extends downward towards the constriction region **260** below the second coupling end **221.** Optionally, in this or any other embodiment, at least one of the first arm **210** below the first coupling end **211** and the second arm **220** below the second coupling end **221** extend radially, mesially, and downward towards the constriction region **260.** Optionally, in this or any other embodiment, at least one of the first arm **210** below the first coupling end **211** and the second arm **220** below the second coupling end **221** extend radially, medially and downward towards the constriction region **260.**

Optionally, in this or any other embodiment, the first arm **210** and the second arm **220** extend symmetrically from opposing ends of the bridge **230.** Optionally, in this or any other embodiment, at least one of the first arm **210** and the second arm **220** are symmetric about a center plane of the bridge **230.** Optionally, in this or any other embodiment, the arched rod **250** is symmetric about one or more planes. Optionally, in this or any other embodiment, the arched rod **250** is symmetric about two planes. Optionally, in this or any other embodiment, the arched rod **250** is symmetric about two perpendicular planes. Optionally, in this or any other embodiment, the arched rod **250** comprises a curvilinear rod. Optionally, in this or any other embodiment, the first arm **210** and the second arm **220** are asymmetric about the bridge **230.** Optionally, in this or any other embodiment, at least one of the first arm **210** and the second arm **220** are asymmetric about a center plane of the bridge **230.** Optionally, in this or any other embodiment, the arched rod **250** is asymmetric about one or more planes.

Optionally, in this or any other embodiment, at least one of the first arm and the second arm **220** extends radially and transversely below the constriction region **260.** Optionally, in this or any other embodiment, at least one of the first arm and the second arm **220** extends downward towards the first terminus **213** and the second terminus **223,** respectively, below the constriction region **260.** Optionally, in this or any other embodiment, at least one of the first arm and the second arm **220** extends radially, transversely, and downward towards the first terminus **213** and the second terminus **223,** respectively, below the constriction region **260.**

Optionally, in this and other embodiments, the penile constriction device may portray a mesial divergence of at least one of the first arm **210** and the second arm **220** from the bridge **230.** A first mesial divergence **215** may optionally form within the first coupling end **211** of the first arm **210** and a second mesial divergence **225** may optionally form within the second coupling end **221** of the second arm **220,** respectively. The transverse divergence of at least one of the first arm **210** and the second arm **220** from the constriction region **260** may forms a first elbow **214** within the first converging end **212** of the first arm **210** and a second elbow **224** within the second converging end **222** of the second arm **220,** respectively.

Per **FIG. 2A****,** the first coupling end **211,** the second coupling end **221,** the first converging end **212,** the second converging end **222,** the first terminus **213,** the second terminus **223,** the first mesial bend **215,** the second mesial bend **225,** the first elbow **214,** and the second elbow **224** is inferior to the superior side of the bridge **230.** Optionally, in this or any other embodiment, at least one of the first coupling end **211,** the second coupling end **221,** the first converging end **212,** the second converging end **222,** the first terminus **213,** the second terminus **223,** the first mesial bend **215,** the second mesial bend **225,** the first elbow **214,** and the second elbow **224** may be inferior to the inferior side of the bridge **230.** Optionally, in this or any other embodiment, at least one of the first coupling end **211,** the second coupling end **221,** the first converging end **212,** the second converging end **222,** the first terminus **213,** the second terminus **223,** the first mesial bend **215,** the second mesial bend **225,** the first elbow **214,** and the second elbow **224** is inferior to the superior side of the bridge **230.** Optionally, in this or any other embodiment, at least a portion of the first coupling end **211** is superior to at least a portion of the second coupling end **221.** Optionally, in this or any other embodiment, at least a portion of the first coupling end **211** is inferior to at least a portion of the second coupling end **221.** Optionally, in this or any other embodiment, the first coupling end **211** is neither inferior, nor superior to the second coupling end **221.** Optionally, in this or any other embodiment, at least a portion of the first converging end **212** is superior to at least a portion of the second converging end **222.** Optionally, in this or any other embodiment, at least a portion of the first converging end **212** is inferior to at least a portion of the second converging end **222.** Optionally, in this or any other embodiment, the first converging end **212** is neither superior nor inferior to the second converging end **222.** Optionally, in this or any other embodiment, the first terminus **213** is superior to the second terminus **223.** Optionally, in this or any other embodiment, the first terminus **213** is inferior to the second terminus **223.** Optionally, in this or any other embodiment, the first terminus **213** is neither inferior nor superior to the second terminus **223.** Optionally, in this or any other embodiment, at least a portion of the first mesial bend **215** is superior to at least a portion of the second mesial bend **225.** Optionally, in this or any other embodiment, at least a portion of the first mesial bend **215** is inferior to at least a portion of the second mesial bend **225.** Optionally, in this or any other embodiment, the entirety of the first mesial bend **215** is neither inferior nor superior to the entirety of the second mesial bend **225.** Optionally, in this or any other embodiment, at least a portion of the first elbow **214** is superior to at least a portion of the second elbow **224.** Optionally, in this or any other embodiment, at least a portion of the first elbow **214** is inferior to at least a portion of the second elbow **224.** Optionally, in this or any other embodiment, the entirety of the first elbow **214** is neither inferior nor superior to the entirety of the second elbow **224.**

Optionally, in this or any other embodiment, a demarcation between the first arm **210** and the bridge **230** may comprise the termination of the first mesial bend **215.** Optionally, in this or any other embodiment, a demarcation between the first arm **210** and the bridge **230** comprises the location of the friction protrusion **231** closest to the first arm **210.** Optionally, in this or any other embodiment, a demarcation between the second arm **220** and the bridge **230** comprises the termination of the second mesial bend **225.** Optionally, in this or any other embodiment, a demarcation between the second arm **220** and the bridge **230** comprises the location of the friction protrusion **231** closest to the second arm **220.**

As seen in **FIG. 2A****,** at least a portion of the first arm **210** may be tangent to a portion of the bridge **230.** Optionally, in this or any other embodiment, at least a portion of the first coupling end **211** is tangent to a portion of the bridge **230.** Optionally, in this or any other embodiment, at least a portion of the first arm **210** is tangent to a portion of the inferior side of bridge **230.** Optionally, in this or any other embodiment, at least a portion of the first coupling end **211** is tangent to a portion of the inferior side of bridge **230.** Optionally, in this or any other embodiment, at least a portion of the first arm **210** is tangent to a portion of the superior side of bridge **230.** Optionally, in this or any other embodiment, at least a portion of the first coupling end **211** is tangent to a portion of the superior side of bridge **230.** Optionally, in this or any other embodiment, at least a portion of the second arm **220** is tangent to a portion of the bridge **230.** Optionally, in this or any other embodiment, at least a portion of the second coupling end **221** is tangent to a portion of the bridge **230.** Optionally, in this or any other embodiment, at least a portion of the second arm **220** is tangent to a portion of the inferior side of bridge **230.** Optionally, in this or any other embodiment, at least a portion of the second coupling end **221** is tangent to a portion of the inferior side of bridge **230.** Optionally, in this or any other embodiment, at least a portion of the second arm **220** is tangent to a portion of the superior side of bridge **230.** Optionally, in this or any other embodiment, at least a portion of the second coupling end **221** is tangent to a portion of the superior side of bridge **230.**

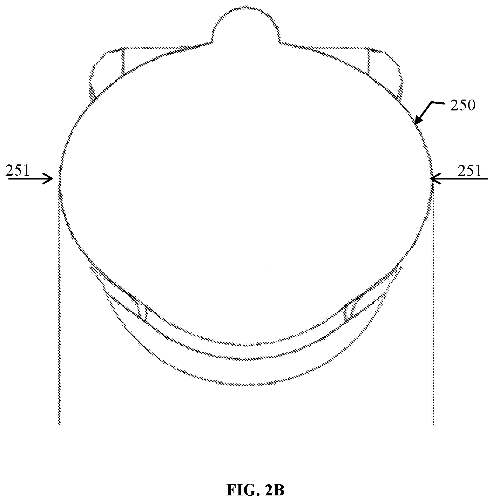

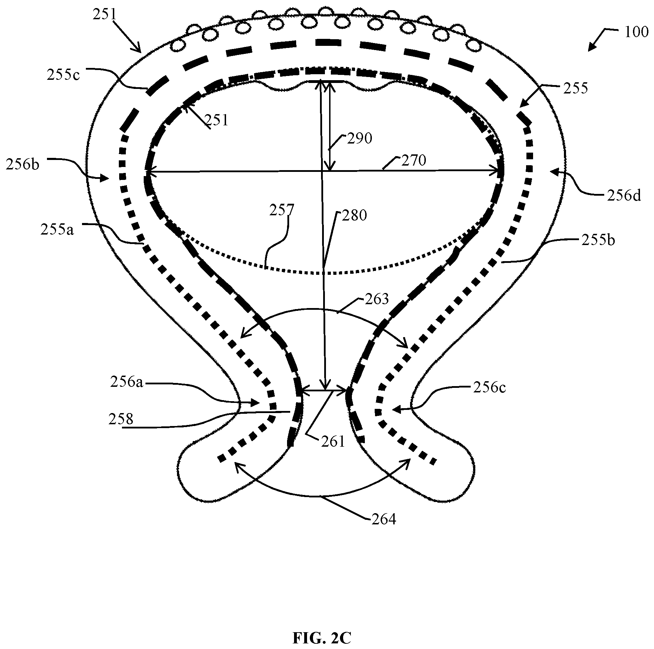

The first terminus **213** and the second terminus **223,** per **FIG. 2A****,** comprise a rounded shape. Optionally, in this or any other embodiment, at least one of the first terminus **213** and the second terminus **223** comprise a pointed shape, a tapered shape, a twisted shape, a blunt shape, a polygonal shape, a bulge shape, or any combination thereof. Per **FIG. 2****,** the first terminus **213** and the second terminus **223** may be equivalent. Optionally, in any other embodiment, the first terminus **213** and the second terminus **223** are not equivalent.

The penile constriction device **100** in **FIG. 2A** has a constriction gap width **261** wherein the constriction gap width **261** is measured as a minimum distance between the first converging end **212** and the second converging end **222** within the constriction region **260.** Optionally, in this or any other embodiment, the constriction gap width **261** is measured as a minimum distance between a mesial vertex of the surface of the first elbow **214** and a mesial vertex of the surface of the second elbow **224.** Optionally, in this or any other embodiment, the constriction gap width **261** is measured as a minimum distance between the first arm **210** and the second arm **220.** Optionally, in this or any other embodiment, the constriction gap distance **261** is measured as a minimum normal distance between the first elbow **214** and the second elbow **214** within the constriction region **260.** Optionally, in this or any other embodiment, the constriction gap width **261** is normal to at least one of a mesial vertex of the surface of the first converging end **212** a mesial vertex of the surface of the second converging end **212,** the first arm **210,** and the second arm **220.**

The penile constriction device **100 in** **FIG. 2A** has a constriction gap distance **261** of greater than 0 inches. Optionally, in this or any other embodiment, the constriction gap distance **261** is negligible or equal to zero, wherein the first arm **210** and the second arm **220** are in temporary and divisible contact. Optionally, in other embodiments, the penile constriction device may have no constriction gap distance (or no constriction region) because the first elbow and the second elbow are permanently physically connected. Optionally, in other embodiments, the penile constriction device may have no constriction gap distance (or no constriction region) because the first arm and the second are permanently physically connected. Optionally, in this or any other embodiment, a recess is formed between the first arm **210,** the second arm **220,** the bridge **230,** and the constriction region **260.** Optionally, in this or any other embodiment, the recess is configured to fit around a penis. Optionally, in this or any other embodiment, the constriction region **260** is superior to at least one of the first terminus **213** and the second terminus **223.** Optionally, in this or any other embodiment, the constriction region **260** is inferior to at least one of the first terminus **213** and the second terminus **223.**

The penile constriction device **100 in** **FIG. 2A** has a constriction height **280,** wherein the constriction height **280** may be measured as a normal distance between a constriction point **262** and the constriction region **240.** Optionally, in this or any other embodiment, the constriction height **280** is measured as a normal distance between the constriction point **262** and the inferior side of the bridge **230.** Optionally, in this or any other embodiment, the constriction height **280** is measured as a normal distance between the constriction point **262** and the inferior side of the bridge **230.** Optionally, in this or any other embodiment, the constriction height **280** is measured as a normal distance between the first elbow **214** and the inferior side of the bridge **230.** Optionally, in this or any other embodiment, the constriction height **280** is measured as a normal distance between the second elbow **224** and the inferior side of the bridge **230.** Optionally, in this or any other embodiment, the constriction height **280** is measured as a normal distance between the most mesial point of the first converging end **212** and the inferior side of the bridge **230.** Optionally, in this or any other embodiment, the constriction height **280** is measured as a normal distance between the most mesial point of the second converging end **222** and the inferior side of the bridge **230.** Optionally, in this or any other embodiment, the constriction height **280** is measured as a normal distance between the first elbow **214** and the compression region **240.** Optionally, in this or any other embodiment, the constriction height **280** is measured as a normal distance between the second elbow **224** and the compression region **240.** Optionally, in this or any other embodiment, the constriction height **280** is measured as a normal distance between the most mesial point of the first converging end **212** and the compression region **240.** Optionally, in this or any other embodiment, the constriction height **280** is measured as a normal distance between the most mesial point of the second converging end **222** and the compression region **240.** Optionally, in this or any other embodiment, the constriction height **280** is measured as a normal distance between the first elbow **214** and the most inferior point of the compression region **240.** Optionally, in this or any other embodiment, the constriction height **280** is measured as a normal distance between the second elbow **224** and the most inferior point of the compression region **240.** Optionally, in this or any other embodiment, the constriction height **280** is measured as a normal distance between the most mesial point of the first converging end **212** and the most inferior point of the compression region **240.** Optionally, in this or any other embodiment, the constriction height **280** is measured as a normal distance between the most mesial point of the second converging end **222** and the most inferior point of the compression region **240.** Optionally, in this or any other embodiment, the constriction height **280** is measured as a normal distance between the first elbow **214** and the most superior point of the compression region **240.** Optionally, in this or any other embodiment, the constriction height **280** is measured as a normal distance between the second elbow **224** and the most superior point of the compression region **240.** Optionally, in this or any other embodiment, the constriction height **280** is measured as a normal distance between the most mesial point of the first converging end **212** and the most superior point of the compression region **240.** Optionally, in this or any other embodiment, the constriction height **280** is measured as a normal distance between the most mesial point of the second converging end **222** and the most superior point of the compression region **240.** Per **FIG. 2****,** the constriction height **280** is perpendicular to at least one of the constriction gap width **261** and the inferior side of the bridge **230.** Alternatively, the constriction height **280** is oblique to at least one of the constriction gap width **261** and the inferior side of the bridge **230.**

The penile constriction device **100** in **FIG. 2A** has a constriction point **262,** wherein the constriction point **262** may be defined as the midpoint of the constriction gap width **261.** Optionally, in this or any other embodiment, the constriction point **262** is defined as a minimally equidistant point from the first converging end **212** and the second converging end **222.** Optionally, in this or any other embodiment, the constriction point **262** is defined as a minimally equidistant point from the first arm **210** and the second arm **220.** Optionally, in this or any other embodiment, the constriction point **262** is defined as a minimally equidistant point from the first elbow **214** and the second elbow **224.**

The penile constriction device **100** in **FIG. 2A****,** has a maximum inner width **270,** wherein the maximum inner width **270** may be measured as a maximum normal distance between a mesial vertex of the surface of the first arm **210** and a mesial vertex of the surface of the second arm **220.** Optionally, in this or any other embodiment, the maximum inner width **270** is measured as a maximum normal distance between a mesial vertex of the surface of the first converging end **212** and a mesial vertex of the surface of the second converging end **222.** Optionally, in this or any other embodiment, the maximum inner width **270** is measured as major axis of an ellipse formed by the first coupling end **211,** the bridge **230,** and the second coupling end **221.** Optionally, in this or any other embodiment, the maximum inner width **270** and the constriction gap width **261** are parallel. Optionally, in this or any other embodiment, the maximum inner width **270** and the constriction gap width **261** are oblique. Optionally, in this or any other embodiment, the maximum inner width **270** and the constriction gap width **261** are coplanar. Optionally, in this or any other embodiment, the maximum inner width **270** and the constriction gap width **261** are non-coplanar. Optionally, in this or any other embodiment, the maximum inner width **270** is superior to the constriction gap **260.**

The penile constriction device **100 in** **FIG. 2A** has a center point **291** defined as the midpoint of a maximum normal distance between a mesial vertex of the surface of the first arm **210** and a mesial vertex of the surface of the second arm **220.** Optionally, in this or any other embodiment, the center point **291** is defined as the midpoint of the maximum inner width **270.** Optionally, in this or any other embodiment, the center point **291** is defined as a point minimally equidistant from a mesial vertex of the surface of the first arm **210** and a mesial vertex of the surface of the second arm **220.** Optionally, in this or any other embodiment, the center point **291** is defined as point minimally equidistant from the first converging end **211** and the second converging end **221.** Optionally, in this or any other embodiment, the center point **291** is defined as the center of an ellipse formed by the first coupling end **211,** the bridge **230,** and the second coupling end **221.**

The device **100,** per **FIG. 2A****,** has an inferior height **290,** wherein the maximum inferior height **290** may be measured as a normal distance between the center point **291** and the compression region **240.** Optionally, in this or any other embodiment, the inferior height **290** is measured as a normal distance between the center point **291** and the inferior side of the bridge **230.** Optionally, in this or any other embodiment, the inferior height **290** is parallel to the constriction height **280.** Optionally, in this or any other embodiment, the inferior height **290** and the constriction height **280** are collinear. Optionally, in this or any other embodiment, the inferior height **290** is perpendicular to at least one of the constriction gap width **261** and the maximum inner width **270.** Optionally, in this or any other embodiment, the inferior height **290** is coplanar with at least one of the constriction gap width **261,** the maximum inner width **270,** the constriction height **280,** and the constriction point **262.**

The device **100,** per **FIG. 2A****,** has a thickness **251,** wherein the thickness **251** may be measured as a maximum normal cross-sectional width of at least a portion of the first arched rod **250.** Optionally, in this or any other embodiment, the thickness **251** of the arched rod **250** is measured as a maximum outer distance between two opposing points of its normal cross-section. Optionally, in this or any other embodiment, the thickness **251** is measured as a maximum normal cross-sectional width of at least a portion of at least one of the first arm **210** and the second arm **220.** Optionally, in this or any other embodiment, the thickness **251** of the first arm **210,** the second arm **220,** and the bridge **230,** is measured as a maximum outer distance between two opposing points on the surfaces of their respective normal cross-sections. Optionally, in this or any other embodiment, the thickness **251** of at least two of the first arm **210,** the second arm **220,** and the bridge **230** are the same. Optionally, in this or any other embodiment, the thickness **251** of at least two of the first arm **210,** the second arm **220,** and the bridge **230** are unequal. Optionally, in this or any other embodiment, at least one of the first arm **210,** the second arm **220,** and the bridge **230** have a uniform thickness **251.** Optionally, in this or any other embodiment, at least one of the first arm **210,** the second arm **220,** and the bridge **230** have a non-uniform thickness **251,** wherein the thickness **251** varies along its span.

The device **100,** per **FIG. 2A****,** has a first termination distance **212a** and a second termination distance **222a,** wherein the first termination distance **212a** may be measured as the maximum normal distance between the constriction gap width **261** and the first terminus **213,** and wherein the second termination distance **222a** may be measured as the maximum normal distance between the constriction gap width **261** and the second terminus **223.** Optionally, in this or any other embodiment, the first termination distance **212a** is measured as the maximum normal distance between the first elbow **214** and the first terminus **213.** Optionally, in this or any other embodiment, the first termination distance **212a** is measured as the maximum normal distance between the second elbow **224** and the second terminus **223.** Optionally, in this or any other embodiment, at least one of the first termination distance **212a** and the second termination distance **222a** is about 0.2 inches to about 0.8 inches. Optionally, in this or any other embodiment, at least one of the first termination distance **212a** and the second termination distance **222a** is at least about 0.2 inches. Optionally, in this or any other embodiment, at least one of the first termination distance **212a** and the second termination distance **222a** is at most about 0.8 inches. Optionally, in this or any other embodiment, at least one of the first termination distance **212a** and the second termination distance **222a** is a constant and does not scale with the maximum inner width **270,** the constriction height **280,** the inferior height **290,** or the constriction gap width **261.**

As seen in **FIG. 2A****,** the first arm **210** and the second arm **220** may be generally parallel at a point superior to the constriction region **260.** Optionally, in this or any other embodiment, the first arm **210** and the second arm **220** are not parallel at a point superior to the constriction region **260.** Optionally, in this or any other embodiment, the first arm **210** and the second arm **220** are separated at a point superior to the constriction region **260** by a constriction angle **263.** Optionally, in this or any other embodiment, the constriction angle **263** is measured as a normal interior angle between the first arm **210** and the second arm **220** at a point superior to the constriction region **260.**

As further seen in **FIG. 2A****,** the first arm **210** and the second arm **220** may be generally parallel at a point inferior to the constriction region **260.** Optionally, in this or any other embodiment, the first arm **210** and the second arm **220** are not parallel at a point inferior to the constriction region **260.** Optionally, in this or any other embodiment, the first arm **210** and the second arm **220** are separated at a point inferior to the constriction region **260** by a termination angle **264.** Optionally, in this or any other embodiment, the termination angle **264** is measured as a normal interior angle between the first arm **210** and the second arm **220** at a point inferior to the constriction region **260.**

Optionally, in this or any other embodiment, the first arm **210** and the second arm **220** have a first arm length and a second arm length, respectively. Optionally, in this or any other embodiment, the first arm **210** has a first length measured a maximal span between the first coupling end **211** and the first terminus **213.** Optionally, in this or any other embodiment, the second arm **220** has a second arm length measured as a maximal span between the second coupling end **221** and the second terminus **223.** Optionally, in this or any other embodiment, the bridge **230** has a bridge length measured as a maximal span of the bridge **230** between the first arm **210** and the second arm **220.** Optionally, in this or any other embodiment, the bridge **230** has a bridge length measured as a maximal span of the bridge **230** between the first coupling end **211** and the second coupling end **221.** Optionally, in this or any other embodiment, the first arm length is equal to the second arm length. Optionally, in this or any other embodiment, the first arm length is greater or less than the second arm length. Optionally, in this or any other embodiment, at least one of the first arm length and the second arm length is greater or less than the bridge length. Optionally, in this or any other embodiment, at least one of the first arm length and the second arm length is equal to the bridge length.

Optionally, in this or any other embodiment, the first mesial divergence **215,** the second mesial divergence **225,** the first elbow **214,** and the second elbow **224** have a first mesial radius, a second mesial radius, a first elbow radius, and a second elbow radius, respectively. Optionally, in this or any other embodiment, the first mesial radius is equal to or greater than at least one of the second mesial radius, the first elbow radius, and the second elbow radius. Optionally, in this or any other embodiment, the second mesial radius is equal to or greater than at least one of the first mesial radius, the first elbow radius, and the second elbow radius. Optionally, in this or any other embodiment, the first elbow radius is equal to or greater than at least one of the first mesial radius, the second mesial radius, and the second elbow radius. Optionally, in this or any other embodiment, the second elbow radius is equal to or greater than at least one of the first mesial radius, the second mesial radius, and the first elbow radius.

**FIG. 2B** shows a side cross-sectional view of a non-limiting example of the penile constriction device of **FIG. 1**. Optionally, in this or any other embodiment, at least one of the first arm **210,** the second arm **220,** and the bridge **230** has a uniform normal cross sectional shape. Optionally, in this or any other embodiment, at least one of the first arm **210,** the second arm **220,** and the bridge **230** have a uniform normal cross sectional shape. Optionally, in this or any other embodiment, at least one of the first arm **210,** the second arm **220,** and the bridge **230** have a non-uniform normal cross sectional shape. Optionally, in this or any other embodiment, at least two of the first arm **210,** the second arm **220,** and the bridge **230** have the same normal cross sectional shape. Optionally, in this or any other embodiment, at least two of the first arm 210, the second arm **220,** and the bridge **230** have dissimilar normal cross sectional shapes. Per **FIG. 2B**, the bridge **230** may have a normal cross section comprising an ellipse. Optionally, in this or any other embodiment, at least one of the first arm **210,** the second arm **220,** and the bridge **230** have a normal cross section comprising a circle, an oval, an ellipse, a polygon, an irregular shape, a hollow shape, or any combination thereof. Optionally, in this or any other embodiment, at least one of the first arm **210,** the second arm **220,** and the bridge **230** have a normal cross section comprising a c-shape, an x-shape, a T-shape, or an H-shape.

**FIG. 2C** shows an alternative front view of a non-limiting example of the penile constriction device of **FIG. 1**. Optionally, in this or any other embodiment, the arched rod **250** comprises an arched axis **255** comprising a curve defined by the centroids of the normal cross sections of the arched rod **250** along its span. Optionally, in this or any other embodiment, the first arm **210,** the second arm **220,** and the bridge **230** comprise a first arched axis **255a,** a second arched axis **255b,** and a third arched axis **255c,** respectively. Optionally, in this or any other embodiment, the first arched axis **255a** and the second arched axis **255b** are coincident, tangent, or both. Optionally, in this or any other embodiment, the second arched axis **255b** and the third arched axis **255c** are coincident, tangent, or both. Optionally, in this or any other embodiment, at least one of the first arched axis **255a,** the second arched axis **255b,** and the third arched axis **255c** comprises a planar curve which is completely coincident with a single plane. Optionally, in this or any other embodiment, at least two of the first arched axis **255a,** the second arched axis **255b,** and the third arched axis **255c** are coplanar.

Per **FIG. 2C****,** the first arched axis **255a** may comprise at least one of a first transverse bend **256a** and a first mesial bend **256b**. Optionally, in this or any other embodiment, the third arched axis **255c** comprises the first mesial bend **256b**. Optionally, in this or any other embodiment, the first transverse bend **256a** comprises the first elbow **214.** Optionally, in this or any other embodiment, the first mesial bend **256b** comprises the first mesial divergence **215.** Optionally, in this or any other embodiment, the first mesial bend **256c** is superior to the first transverse bend **256a**. Optionally, in this or any other embodiment, the second arched axis **255b** comprises at least one of a second transverse bend **256c** and a second mesial bend **256d.** Optionally, in this or any other embodiment, the third arched axis **255c** comprises the second mesial bend **256d.** Optionally, in this or any other embodiment, the second transverse bend **256c** comprises the second elbow **224.** Optionally, in this or any other embodiment, the second mesial bend **256d** comprises the second mesial divergence **225.** Optionally, in this or any other embodiment, the second mesial bend **256c** is superior to the second transverse bend **256d.** Optionally, in this or any other embodiment, a demarcation between the first arched axis **255a** and the bridge **255c** comprises the termination of the first mesial bend **256b.** Optionally, in this or any other embodiment, a demarcation between the first arched axis **255a** and the bridge **255c** comprises the location of the friction protrusion **231** closest to the first arched axis **255a**. Optionally, in this or any other embodiment, a demarcation between the second arched axis **255b** and the bridge **255c** comprises the termination of the second mesial bend **256d.** Optionally, in this or any other embodiment, a demarcation between the second arched axis **255b** and the bridge **255c** comprises the location of the friction protrusion **331** closest to the second arched axis **255b**. Optionally, in this or any other embodiment, the radius of the first transverse bend **256a** is equal to or greater than the radius of the first mesial bend **256b**. Optionally, in this or any other embodiment, the radius of the first transverse bend **256a** is less than the radius of the first mesial bend **256b**. Optionally, in this or any other embodiment, the radius of the second transverse bend **256c** is equal to or greater than the radius of the second mesial bend **256d.** Optionally, in this or any other embodiment, the radius of the second transverse bend **256c** is less than the radius of the second mesial bend **256d.** Optionally, in this or any other embodiment, the radius of the first transverse bend **256a** is equal to or greater than the radius of the second transverse bend **256c**. Optionally, in this or any other embodiment, the radius of the first transverse bend **256a** is less than the radius of the second transverse bend **256c**. Optionally, in this or any other embodiment, the radius of the first mesial bend **256b** is equal to or greater than the radius of the second mesial bend **256d.** Optionally, in this or any other embodiment, the radius of the first mesial bend **256b** is less than the radius of the second mesial bend **256d.**

Per **FIG. 2C**, at least a portion of the first arched axis **255a**, the second arched axis **255b**, and the third arched axis **255c** may form a portion of an ellipse **257.** Optionally, in this or any other embodiment, the ellipse **257** has a major axis and a minor axis, wherein the major axis greater than the minor axis. Optionally, in this or any other embodiment, the major axis is oriented from the first arched axis **255a** to the second arched axis **255b**, along the third arched axis **255c**.

As seen in **FIG. 2C**, the arched rod **250** may comprise a mesial vertex arched axis **258.** Optionally, in this or any other embodiment, the mesial vertex arched axis **258** is defined as a single continuous line that is coincident to the surface of the arched rod **250** and closest to the center point **291.** Optionally, in this or any other embodiment, the mesial vertex arched axis **258** is substantially planar. Optionally, in this or any other embodiment, the mesial vertex arched axis **258** and the arched axis **255** are substantially coplanar. Optionally, in this or any other embodiment, the mesial vertex arched axis **258** and at least one of the first arched axis **255a**, the second arched axis **255b**, and the third arched axis **255c** are substantially coplanar.

Per **FIG. 2C****,** the constriction gap width **261** of the arched rod **250** may be measured as a minimum separation of the mesial vertex arched axis **255** within the constriction region **260.** Optionally, in this or any other embodiment, the first arm length, the second arm length, and the bridge a length are measured as an arc length of the first arched axis **255a,** the second arched axis **255b,** and the third arched axis **255c,** respectively. Optionally, in this or any other embodiment, the inferior height **290** is measured as a half the minor axis of the ellipse formed by the first coupling end **211,** the bridge **230,** and the second coupling end **221.** Optionally, in this or any other embodiment, the constriction height **280** is measured as a maximum normal distance between the constriction point **262** and the mesial arched axis **255.** Optionally, in this or any other embodiment, the constriction height **280,** is perpendicular to the mesial vertex arched axis **258.** Optionally, in this or any other embodiment, the maximum inner width **270,** is measured as a maximum normal separation distance of the mesial vertex arched axis **270.** Optionally, in this or any other embodiment, the inferior height **290** is measured, as a normal distance between the center point **291** and the mesial vertex arched axis **258.** Optionally, in this or any other embodiment, the minor axis is twice the inferior height **290.** Optionally, in this or any other embodiment, a center point of the ellipse **257** is the center point **291.**

Per **FIG. 2C****,** the constriction angle **263** of the arched rod **250** may be measured as a normal interior angle between the first arched axis **255a** and the second arched axis **255b** at a point inferior to the constriction region **260.** Optionally, in this or any other embodiment, the first arched axis **255a** and the second arched axis **255b** are parallel at a point inferior to the constriction region **260.** Optionally, in this or any other embodiment, the first arched axis **255a** and the second arched axis **255b** are not parallel a point inferior to the constriction region **260.**

Per **FIG. 2C****,** the termination angle **264** may be measured as a normal interior angle between the first arched axis **255a** and the second arched axis **255b** at a point inferior to the constriction region **260.** Optionally, in this or any other embodiment, the first arched axis **255a** and the second arched axis **255b** are parallel at a point inferior to the constriction region **260.** Optionally, in this or any other embodiment, the first arched axis **255a** and the second arched axis **255b** are not parallel a point inferior to the constriction region **260.**

Per **FIG. 2C****,** the thickness **251** may be measured, as twice the normal distance between the mesial vertex arched axis **255** and at least one of the first arched axis **255a,** the second arched axis **255b,** and the third arched axis **255c**. Optionally, in this or any other embodiment, the thickness **251** of the first arm **210,** the second arm **220,** and the bridge **230** are measured as a maximum outer distance that is normal to the first arched axis **255a,** the second arched axis **255b,** and the third arched axis **255c,** respectively. Optionally, in this or any other embodiment, the normal cross section is defined as being normal to at least one of the first arched axis **255a,** the second arched axis **255b,** and the third arched axis **255c**. Optionally, in this or any other embodiment, the normal cross section of at least one of the first arm **210,** the second arm **220,** and the bridge **230** helically twists along the first arched axis **210,** the second arched axis **220,** and the third arched axis **230,** respectively. Optionally, in this or any other embodiment, the arched rod has a two-dimensional cavity area measured as a two-dimensional area confined by the constriction gap width, and the mesial vertex arched axis **258.**

As seen in **FIGS. 1** and **2A-C****,** at least one of the first elbow **214** and the second elbow **224,** the first mesial bend **215,** the second mesial bend **225,** the first converging end **212,** the second converging end **222,** the first terminus **213,** and the second terminus **223** may be configured to enable a user to grasp and apply a force to the device **100.** Optionally, in this or any other embodiment, at least one of the first elbow **214,** the second elbow **224,** the first mesial bend **215,** the second mesial bend **225** the first terminus **213,** and the second terminus **223** enable a user to grasp and apply a force between the first arm **210** and the second arm **220.** Optionally, in this or any other embodiment, at least one of the first elbow **214,** the second elbow **224,** the first mesial bend **215,** the second mesial bend **225** the first terminus **213,** and the second terminus **223** enable a user to grasp and apply a force to the device **100** to increase the constriction gap **261.** Optionally, in this or any other embodiment, at least one of the first elbow **214,** the second elbow **224,** the first mesial bend **215,** the second mesial bend **225,** the first terminus **213,** and the second terminus **223** enable a user to grasp and apply a force to the device **100** to increase the constriction gap **261** and install the device by passing the penis through the constriction gap **260.**

Optionally, in this or any other embodiment, the device **100** is ductile (flexible and/or elastic), wherein an applied force modifies the shape of the arched rod **250,** and wherein the arched rod **250** returns to its original shape upon cessation of the applied force. Optionally, in this or any other embodiment, at least one of the first arm **210,** the second arm **220,** and the bridge **230** is ductile. Optionally, in this or any other embodiment, the device **100** is pliable, wherein an applied force modifies the shape of the arched rod **250,** and wherein the shape of the arched rod **250** does not return to its original shape upon cessation of the applied force. Optionally, in this or any other embodiment, at least one of the first arm **210,** the second arm **220,** and the bridge **230** is pliable. Optionally, in this or any other embodiment, the arched rod **250** is ductile and pliable. Optionally, in this or any other embodiment, at least one of the first arm **210,** the second arm **220,** and the bridge **230** is ductile and pliable. Optionally, in this or any other embodiment, at least one of the first arm **210,** the second arm **220,** and the bridge **230** is ductile and not pliable. Optionally, in this or any other embodiment, at least one of the first arm **210,** the second arm **220,** and the bridge **230** is pliable and not ductile. Optionally, in this or any other embodiment, at least one of the first arm **210,** the second arm **220,** is more ductile than the bridge **230.** Optionally, in this or any other embodiment, at least one of the first arm **210,** the second arm **220,** is more pliable than the bridge **230.** Optionally, in this or any other embodiment, at least one of the first arm **210,** the second arm **220,** is less ductile than the bridge **230.** Optionally, in this or any other embodiment, at least one of the first arm **210,** the second arm **220,** is less pliable than the bridge **230.** Optionally, in this or any other embodiment, the first arm **210** is more pliable than the second arm **220.** Optionally, in this or any other embodiment, the first arm **210** is more ductile than the second arm **220.** Optionally, in this or any other embodiment, the first arm **210** is less pliable than the second arm **220.** Optionally, in this or any other embodiment, the first arm **210** is less ductile than the second arm **220.**

Optionally, in this or any other embodiment, the arched rod **250** is ductile, wherein the constriction gap **261** returns to its original size upon cessation of the applied force. Optionally, in this or any other embodiment, the ductility of the arched rod **250** enables a user to apply the device **100** to the penis by applying a tensile force between the first converging end **212** and/or the first terminus **213** and the second converging end **222** and/or the second terminus **223** to increase the constriction gap **261,** inserting the device **100** onto the penile area, and releasing the tensile force. Optionally, in this or any other embodiment, once the tensile force is released, the constriction gap **261** of the arched rod **250** is greater than the constriction gap **261** of an unstressed and/or unapplied device **100.** Optionally, in this or any other embodiment, once the tensile force is released, the ductile arched rod **250** maintains a compressive force against the penile area.

Optionally, in this or any other embodiment, a tensile force applied between the first converging end **212** and/or the first terminus **213** and the second converging end **222** and/or the second terminus **223,** increases the constriction gap **260.** Optionally, in this or any other embodiment, a compressive force applied between the first converging end **212 and the second** converging end **222** decreases the constriction gap **261.**

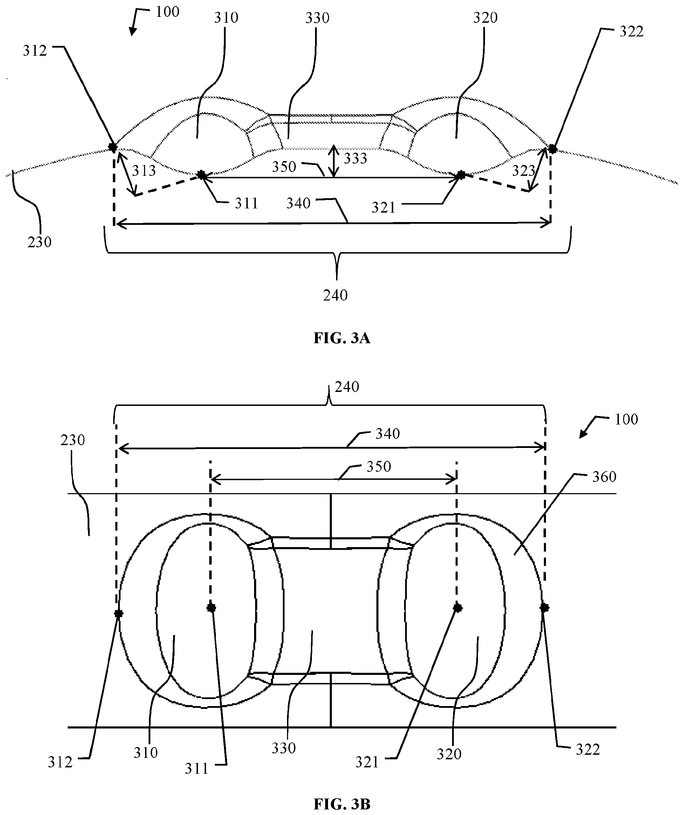

**FIG. 3A** shows a detailed front view of the compression region **240** of a non-limiting example of the penile constriction device **100** of **FIG. 1**. **FIG. 3B** shows a detailed bottom view of the compression region **240** of the penile constriction device **100** of **FIG. 1**. Provided herein is a penile constriction device **100** comprising an arched rod **250** comprising a bridge **230** having a compression region **240** rising inferiorly from an inferior side of the bridge **230.**

Per **FIGS. 3A-B**, the compression region **240** may comprise a first dorsal vein protrusion **310** and a second dorsal vein protrusion **320** rising from the inferior side of the bridge **230.** Further, the first dorsal vein protrusion **310** may comprise at least one of a first dorsal apex **311** and a first lateral edge **312.** The first lateral edge **312**, per **FIG. 3A**, may be disposed at the most transverse point along the intersection between the first dorsal vein protrusion **310** and the inferior side of the bridge **230.** Alternatively, in this or any other embodiment, the first lateral edge **312** may be disposed at the most transverse point of the compression region **240** that is closest a first arm. The first dorsal apex **311,** per **FIG. 3A**, may be disposed at the most inferior point on the first dorsal vein protrusion **310.** Alternatively, in this or any other embodiment, the first dorsal apex **311** may be disposed at an inflection point of the first dorsal vein protrusion **310.** Optionally, in this or any other embodiment, the second dorsal vein protrusion **320** comprises at least one of a second dorsal apex **321** and a second lateral edge **322.** Optionally, per **FIG. 3A**, the second lateral edge **322** may be disposed at the most transverse point along the intersection between the second dorsal vein protrusion **320** and the inferior side of the bridge **230.** Alternatively, in this or any other embodiment, the second lateral edge **322** may be disposed at the most transverse point of the compression region **240** that is closest a second arm. The second dorsal apex **321,** per **FIG. 3A****,** may be disposed at the most inferior point on the second dorsal vein protrusion **320.** Alternatively, in this or any other embodiment, the second dorsal apex **321** may be disposed at an inflection point of the second dorsal vein protrusion **320.** Optionally, in this or any other embodiment, the compression region **240** is integral with the inferior side of the bridge **230.** Optionally, in this or any other embodiment, the compression region **240** is separate and distinct from the inferior side of the bridge **230.** Optionally, in this or any other embodiment, the compression region **240** within the dorsal vein valley **330** at a center of the dorsal vein valley **330** is superior to, or has a same height relative to, the first lateral edge **312** as the first minimum normal distance or relative to the second lateral edge **322** as the second minimum normal distance, or both. Optionally, in this or any other embodiment, the compression region **240** at a center of the dorsal vein valley **330** is superior to the first lateral edge **312,** the second lateral edge **322,** or both. Optionally, in this or any other embodiment, the compression region **240** at a center of the dorsal vein valley **330** is inferior to the first lateral edge **312,** the second lateral edge **322,** or both. Optionally, in this or any other embodiment, a boundary between the first dorsal vein protrusion **310** and the dorsal vein valley **330** comprises a plane coincident with the first dorsal apex **311,** and perpendicular to the dorsal vein valley distance. Optionally, in this or any other embodiment, a boundary between the second dorsal vein protrusion **220** and the dorsal vein valley **330** comprises a plane coincident with the second dorsal apex **321,** and normal to the dorsal vein valley distance **350.** Optionally, in this or any other embodiment, the first dorsal vein protrusion **310,** the dorsal vein valley **330,** and the second dorsal vein protrusion **320** are arranged sequentially within the compression region **240** in a direction along the bridge **230** from the first arm **210** to the second arm **220.**

Per **FIG. 3A****,** at least a portion of a perimeter of the compression region **240,** where the compression region **240** meets inferior side of the bridge **230,** optionally comprises a fillet **360.** Optionally, in this or any other embodiment, the portion of a perimeter of the compression region **240** where the compression region **240** meets the inferior side of the bridge **230** comprises at least one of the intersection between the first dorsal protrusion **310** and the inferior side of the bridge **230,** the intersection between the second dorsal protrusion **320** and the inferior side of the bridge **230,** and the intersection between the dorsal vein valley **330** and the inferior side of the bridge **230.** Optionally, in this or any other embodiment, at least one of the first dorsal protrusion **310,** the second dorsal protrusion **320,** and the dorsal vein valley **330** comprise a first portion, a second portion, and a fillet **360** between the first portion and the second portion. Optionally, in this or any other embodiment, the fillet **360** comprises a concave fillet, a miter fillet, or a convex fillet. Optionally, in this or any other embodiment, the fillet **360** comprises a constant fillet. Optionally, in this or any other embodiment, the fillet **360** comprises a variable fillet. Optionally, in this or any other embodiment, the fillet **360** improves the manufacturability of the device **100.** Optionally, in this or any other embodiment, the fillet **360** increases the rigidity of at least one of the first dorsal vein protrusion **210,** the second dorsal vein protrusion **220,** and the dorsal vein valley **230.** Optionally, in this or any other embodiment, the fillet **360** prevents pinching between the compression region **240** and the inferior side of the bridge **230.**

As seen in **FIGS. 3A-B****,** at least one of the first dorsal vein protrusion **210** and the second dorsal vein protrusion **220** may comprise a cross sectional shape comprising an ellipse. Optionally, in this or any other embodiment, at least one of the first dorsal vein protrusion **210** and the second dorsal vein protrusion **220** comprise a cross sectional shape comprising a circle, an oval, a polygon, or any combination thereof. Optionally, in this or any other embodiment, the first dorsal vein protrusion **210** and the second dorsal vein protrusion **220** have equivalent cross sectional shapes. Optionally, in this or any other embodiment, the first dorsal vein protrusion **210** and the second dorsal vein protrusion **220** have cross sectional shapes with a mirrored symmetry. Optionally, in this or any other embodiment, the first dorsal vein protrusion **210** and the second dorsal vein protrusion **220** have mirrored symmetry. Optionally, in this or any other embodiment, the first dorsal vein protrusion **210** and the second dorsal vein protrusion **220** have inequivalent cross sectional shapes. Optionally, in this or any other embodiment, the first dorsal vein protrusion **210** and the second dorsal vein protrusion **220** have equivalent cross sectional shapes. Optionally, in this or any other embodiment, the dorsal vein valley **230** is generally concave in one or more directions. Optionally, in this or any other embodiment, the dorsal vein valley **230** is generally convex in one or more directions.

Per **FIGS. 3A-B****,** the compression region **240** comprises a compression region width **340** measured as a distance between the first lateral edge **312** and the second lateral edge **322.** Optionally, in this or any other embodiment, the compression region width **340** is measured as a maximum width of the compression region **240** in a dimension from the first arm **210** to the second arm **220** along the inferior surface of the bridge **230.** Optionally, in this or any other embodiment, the compression region width **340** is measured as a maximum normal width of the compression region **240** in a dimension from the first arm **210** to the second arm **220** along the inferior surface of the bridge **230.**

Per **FIGS. 3A-B****,** the dorsal vein valley **330** has a dorsal vein valley distance **350** measured as a distance between the first dorsal apex **311** and the second dorsal apex **321.** Optionally, in this or any other embodiment, the dorsal vein valley distance **350** is measured as a maximum transverse span of the dorsal vein valley **330.** Optionally, in this or any other embodiment, the dorsal vein valley distance **350** is measured as a maximum transverse and normal span of the dorsal vein valley **330**

As seen in **FIG. 3A**, the first dorsal protrusion **310** has a first dorsal height **313** measured as a first minimum normal distance between the first dorsal apex **311** and the first lateral edge **312.** Further, the second dorsal protrusion **320** may comprise a second dorsal height **323** measured as a second minimum normal distance between the second dorsal apex **321** and the second lateral edge **322.** Optionally, in this or any other embodiment, the first dorsal height **313** is measured as a minimum normal distance between the first dorsal apex **311** and a perimeter of the compression region **240.** Optionally, in this or any other embodiment, the second dorsal height **323** is measured as a minimum normal distance between the second dorsal apex **321** and a perimeter of the compression region **240.** Optionally, in this or any other embodiment, the first dorsal height **313** is measured as a maximum normal distance between the first dorsal apex **311** and a perimeter of the compression region **240.** Optionally, in this or any other embodiment, the second dorsal height **323** is measured as a maximum normal distance between the second dorsal apex **321** and a perimeter of the compression region **240.** Optionally, in this or any other embodiment, the first dorsal height **313** is measured as an average normal distance between the first dorsal apex **311** and a perimeter of the compression region **240.** Optionally, in this or any other embodiment, the second dorsal height **323** is measured as an average normal distance between the second dorsal apex **321** and a perimeter of the compression region **240.** Optionally, in this or any other embodiment, the first dorsal height **313** is greater than the second dorsal height **323.** Optionally, in this or any other embodiment, the first dorsal height **313** is less than the second dorsal height **323.** Optionally, in this or any other embodiment, the first dorsal height **313** and the second dorsal height **323** are approximately equal.

The dorsal vein valley **330,** per **FIG. 3A****,** comprises a dorsal vein valley depth **333** measured as a normal distance between the first dorsal apex **311** and the center of the dorsal vein valley **330.** Optionally, in this or any other embodiment, the dorsal vein valley depth **333** is measured as a normal distance between the second dorsal apex **321** and the center of the dorsal vein valley **330.** Optionally, in this or any other embodiment, the dorsal vein valley depth **333** is measured as a minimal normal distance between the second dorsal apex **321** and the center of the dorsal vein valley **330.** Optionally, in this or any other embodiment, the dorsal vein valley depth **333** is measured as a traverse dimension of the dorsal vein valley **330** normal to the dorsal vein valley distance **350.** Optionally, in this or any other embodiment, the dorsal vein valley **330** comprises two or more dorsal vein valley depths **333.** Optionally, in this or any other embodiment, the dorsal vein valley depth **333** of the dorsal vein valley **330** at its intersection with the first dorsal vein protrusion **210** is equal to the dorsal vein valley depth **333** of the dorsal vein valley **330** at its intersection with the center of the dorsal vein valley **330.** Optionally, in this or any other embodiment, the dorsal vein valley depth **333** of the dorsal vein valley **330** at its intersection with the first dorsal vein protrusion **210** is smaller or larger than the dorsal vein valley depth **333** of the dorsal vein valley **330** at its intersection with the center of the dorsal vein valley **330.** Optionally, in this or any other embodiment, the dorsal vein valley depth **333** of the dorsal vein valley **330** at its intersection with the second dorsal vein protrusion **220** is equal to the dorsal vein valley depth **333** of the dorsal vein valley **330** at its intersection with the center of the dorsal vein valley **330.** Optionally, in this or any other embodiment, the dorsal vein valley depth **333** of the dorsal vein valley **330** at its intersection with the second dorsal vein protrusion **220** is smaller or larger than the dorsal vein valley depth **333** of the dorsal vein valley **330** at its intersection with the center of the dorsal vein valley **330.** Optionally, in this or any other embodiment, the dorsal vein valley depth **333** of the dorsal vein valley **330** at its intersection with the first dorsal vein protrusion **210** is equal to the dorsal vein valley depth **333** of the dorsal vein valley **330** at its intersection with the second dorsal vein protrusion **220.** Optionally, in this or any other embodiment, the dorsal vein depth of the dorsal vein valley **330** at its intersection with the first dorsal vein protrusion **210** is smaller or larger than the dorsal vein valley depth **333** of the dorsal vein valley **330** at its intersection with the second dorsal vein protrusion **220.**

Optionally, in this or any other embodiment, at least one of the first dorsal height **313,** the second dorsal height **323,** the dorsal vein valley distance **350,** the compression region width **340,** and the dorsal vein valley depth are scaled with respect to at least one of the maximum inner width **270,** the constriction gap **261,** inferior height **290,** and the constriction height **280.**

Optionally, in this or any other embodiment, at least one of the first dorsal height and the second dorsal height is about 0.02 inches to about 0.08 inches. Optionally, in this or any other embodiment, at least one of the first dorsal height and the second dorsal height is at least about 0.02 inches. Optionally, in this or any other embodiment, at least one of the first dorsal height and the second dorsal height is at most about 0.08 inches. Optionally, in this or any other embodiment, a dorsal vein valley distance is about 0.15 inches to about 0.7 inches. Optionally, in this or any other embodiment, a dorsal vein valley distance is at least about 0.15 inches. Optionally, in this or any other embodiment, a dorsal vein valley distance is at most about 0.7 inches. Optionally, in this or any other embodiment, a dorsal vein valley depth is about 0.02 inches to about 0.08 inches. Optionally, in this or any other embodiment, a dorsal vein valley depth is at least about 0.02 inches. Optionally, in this or any other embodiment, a dorsal vein valley depth is at most about 0.08 inches. Optionally, in this or any other embodiment, a ratio between the dorsal vein valley distance and at least one of the first dorsal height and the second dorsal height is about 4:1 to about 16:1. Optionally, in this or any other embodiment, a ratio between the dorsal vein valley distance and at least one of the first dorsal height and the second dorsal height is at least about 4:1. Optionally, in this or any other embodiment, a ratio between the dorsal vein valley distance and at least one of the first dorsal height and the second dorsal height is at most about 16:1. Optionally, in this or any other embodiment, the compression region width is about 0.2 inches to about 0.8 inches. Optionally, in this or any other embodiment, the compression region width is at least about 0.2 inches. Optionally, in this or any other embodiment, the compression region width is at most about 0.8 inches. Optionally, in this or any other embodiment, a ratio between the compression region width and a dorsal vein valley distance is about 1.1:1 to about 2:1. Optionally, in this or any other embodiment, a ratio between the compression region width and a dorsal vein valley distance is at least about 1.1:1. Optionally, in this or any other embodiment, a ratio between the compression region width and a dorsal vein valley distance is at most about 2:1. Optionally, in this or any other embodiment, a dorsal vein depth is about 0.15 inches to about 0.65 inches. Optionally, in this or any other embodiment, a dorsal vein depth is at least about 0.15 inches. Optionally, in this or any other embodiment, a dorsal vein depth is at most about 0.65 inches. Optionally, in this or any other embodiment, a ratio between the compression region width and the maximum inner width is about 0.2:1 to about 0.7:1. Optionally, in this or any other embodiment, a ratio between the compression region width and the maximum inner width is at least about 0.2:1. Optionally, in this or any other embodiment, a ratio between the compression region width and the maximum inner width is at most about 0.7:1. Optionally, in this or any other embodiment, a dorsal vein valley distance is about 0.15 inches to about 0.75 inches. Optionally, in this or any other embodiment, a dorsal vein valley distance is at least about 0.15 inches. Optionally, in this or any other embodiment, the dorsal vein valley distance is at most about 0.75 inches. Optionally, in this or any other embodiment, the compression region width is about 0.2 inches to about 0.8 inches. Optionally, in this or any other embodiment, the compression region width is at least about 0.2 inches. Optionally, in this or any other embodiment, the compression region width is at most about 0.8 inches.

Per **FIGS. 3A-B****,** the compression region **240** may comprise the first dorsal vein protrusion **310** and the second dorsal vein protrusion **320.** Optionally, in this or any other embodiment, compression region **240** may further comprise 1, 2, 3, 4, 5, or more dorsal vein protrusions. Optionally, in this or any other embodiment, the three or more dorsal vein protrusions are arranged sequentially within the compression region **240** in a direction along the bridge **230** from the first arm **210** to the second arm **220.** Optionally, in this or any other embodiment, the three or more dorsal vein protrusions are arranged in a linear pattern, a circular pattern, a checkerboard pattern, a random pattern, or any combination thereof. Optionally, in this or any other embodiment, at least two of the three or more dorsal vein protrusions have the same cross sectional shape. Optionally, in this or any other embodiment, at least two of the three or more dorsal vein protrusions have different cross sectional shapes. Optionally, in this or any other embodiment, the dorsal height of each of the three or more dorsal vein protrusions are equal. Optionally, in this or any other embodiment, the dorsal vein height of each of the three or more dorsal vein protrusions are unequal.

Optionally, in this or any other embodiment, the compression region **240** comprises three or more dorsal vein protrusions, wherein each dorsal vein protrusion is separated from one or more other dorsal vein protrusions by a dorsal vein valley **330,** wherein the compression region **240** comprises two or more dorsal vein valleys Optionally, in this or any other embodiment, at least two of the two or more dorsal vein valleys have the same cross sectional shape. Optionally, in this or any other embodiment, at least two of the three or more dorsal vein protrusions have different cross sectional shapes. Optionally, in this or any other embodiment, the dorsal vein valley distance **350** of each of the two or more dorsal vein valleys are equal. Optionally, in this or any other embodiment, the dorsal vein valley distance **350** of each of the two or more dorsal vein valleys are unequal. Optionally, in this or any other embodiment, the dorsal vein valley depths 333 of each of the two or more dorsal vein valleys are equal. Optionally, in this or any other embodiment, the dorsal vein valley depths 333 of each of the two or more dorsal vein valleys are unequal.

Optionally, in this or any other embodiment, at least one three or more dorsal protrusions has a dorsal height of about 0.02 inches to about 0.08 inches. Optionally, in this or any other embodiment, at least one three or more dorsal protrusions has a dorsal height of least about 0.02 inches. Optionally, in this or any other embodiment, at least one three or more dorsal protrusions has a dorsal height of at most about 0.08 inches. Optionally, in this or any other embodiment, a dorsal vein valley distance of at least one of the dorsal vein valleys is about 0.15 inches to about 0.7 inches. Optionally, in this or any other embodiment, the dorsal vein valley distance of at least one of the dorsal vein valleys is at least about 0.15 inches. Optionally, in this or any other embodiment, the dorsal vein valley distance of at least one of the dorsal vein valleys is at most about 0.7 inches. Optionally, in this or any other embodiment, a dorsal vein valley depth of at least one of the dorsal vein valleys is about 0.02 inches to about 0.08 inches. Optionally, in this or any other embodiment, the dorsal vein valley depth of at least one of the dorsal vein valleys is at least about 0.02 inches. Optionally, in this or any other embodiment, the dorsal vein valley depth of at least one of the dorsal vein valleys is at most about 0.08 inches.

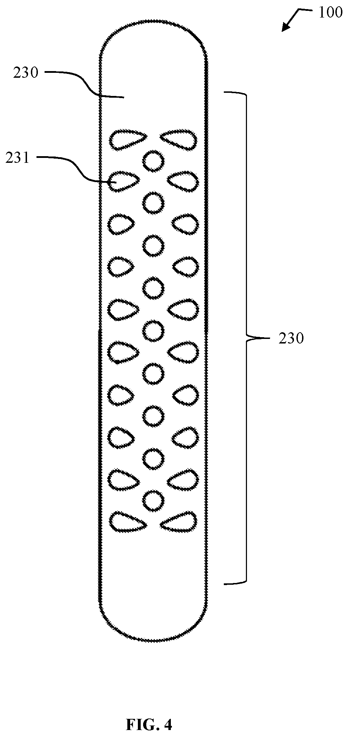

**FIG. 4** shows a top view of the friction protrusions of the penile constriction device **100** of **FIG. 1**. Optionally, in this or any other embodiment, the penile constriction device **100** comprises a plurality of friction protrusions **231** on a superior side of the bridge **230.**

In one exemplary embodiment, per **FIG. 4****,** the bridge **230** comprises 29 friction protrusions **231** in a staggered rectilinear array comprising 3 columns and 19 rows, wherein each column is arranged in a direction from the first arm **210** to the second arm **220.** Optionally, in this or any other embodiment, the plurality of friction protrusions is arranged in a circular array, a polygonal array, a curvilinear array, or any combination thereof. Optionally, in this or any other embodiment, the friction protrusions **251** comprise a cross sectional shape comprising a circle, a half circle, an oval, an ellipse, a teardrop, a polygon, an irregular shape, a hollow shape, or any combination thereof. Optionally, in this or any other embodiment, at least two of the friction protrusions **251** comprise the same cross sectional shape. Optionally, in this or any other embodiment, at least two of the friction protrusions **251** comprise different cross sectional shapes. In this exemplary embodiment, at least one of the shape and the size of the friction protrusions **231** are configured to be easily producible by a molding process or an over-molding process. Optionally, in this or any other embodiment, the plurality of friction protrusions **231** comprises about 2 protrusions to about 60 protrusions. Alternatively, in this or any other embodiment, the plurality of friction protrusions **231** comprises at least about 2 protrusions. Alternatively, in this or any other embodiment, the plurality of friction protrusions **231** comprises at most about 60 protrusions.

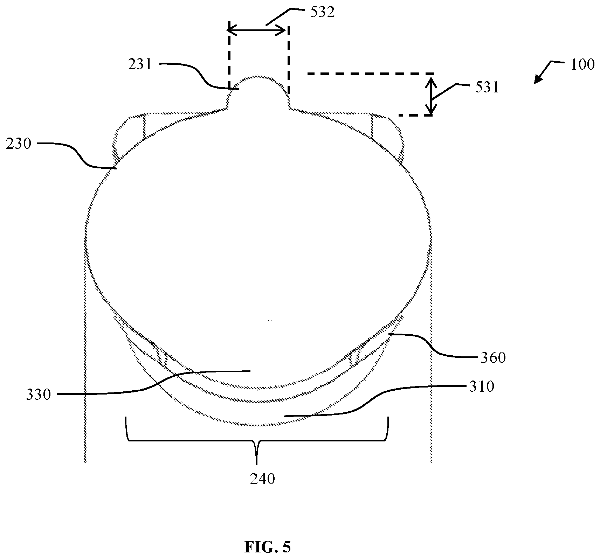

**FIG. 5** shows a side cross-sectioned view of the friction protrusions and compression region of the penile constriction device **100** of **FIG. 1**. Per **FIG**. **5**, the friction protrusion height **531** is measured as a maximal normal distance between an apex of the friction protrusion **251** and the superior side of the bridge **230.** Optionally, in this or any other embodiment, the friction protrusion height **531** is measured as a maximum normal distance between the apex of the friction protrusion **251** and its intersection with the superior side of the bridge **230.** Optionally, in this or any other embodiment, the friction protrusion height **531** is measured as a minimum normal distance between the apex of the friction protrusion **251** and its intersection with the superior side of the bridge **230.** Optionally, in this or any other embodiment, at least two of the friction protrusions **251** have the same friction protrusion height **531.** Optionally, in this or any other embodiment, at least two of the friction protrusions **251** have different friction protrusion heights **531.** Optionally, in this or any other embodiment, the friction protrusion height **531** is about 0.01 inches to about 0.06 inches. Optionally, in this or any other embodiment, the friction protrusion height **531** is at least about 0.01 inches. Optionally, in this or any other embodiment, the friction protrusion height **531** is at most about 0.06 inches.

Per **FIG. 5****,** the friction protrusion width **532** is measured as a maximal normal distance between a first intersection between the friction protrusion **251** and the superior side of the bridge **230,** and a second opposing intersection between the friction protrusion **251** and the superior side of the bridge **230.** Optionally, in this or any other embodiment, the friction protrusion width **532** is measured as a minimal normal distance between a first intersection between the friction protrusion **251** and the superior side of the bridge **230,** and a second opposing intersection between the friction protrusion **251** and the superior side of the bridge **230.** Optionally, in this or any other embodiment, at least two of the friction protrusions **251** have the same friction protrusion width **532.** Optionally, in this or any other embodiment, at least two of the friction protrusions **251** have different friction protrusion widths **532.** Optionally, in this or any other embodiment, the friction protrusion width **532** is about 0.01 inches to about 0.06 inches. Optionally, in this or any other embodiment, the friction protrusion width **532** is at least about 0.01 inches. Optionally, in this or any other embodiment, the friction protrusion width **532** is at most about 0.06 inches.

Optionally, in this or any other embodiment, the plurality of friction protrusion **231** is configured to interact with the female genitalia during intercourse. Optionally, in this or any other embodiment, the plurality of friction protrusion **231** is configured to stimulate the female genitalia during intercourse. Optionally, in this or any other embodiment, the plurality of friction protrusion **231** is configured to interact with the clitoris during intercourse. Optionally, in this or any other embodiment, at least one of the array of the plurality of friction protrusions **231,** the cross sectional shape of the plurality of friction protrusions **231,** and the friction protrusion height **531** are configured for increased stimulation of the female genitalia during intercourse. Optionally, in this or any other embodiment, at least one of the array of the plurality of friction protrusions **231,** the cross sectional shape of the plurality of friction protrusions **231,** and the friction protrusion height **531** are configured to prevent damage to the female genitalia during intercourse.

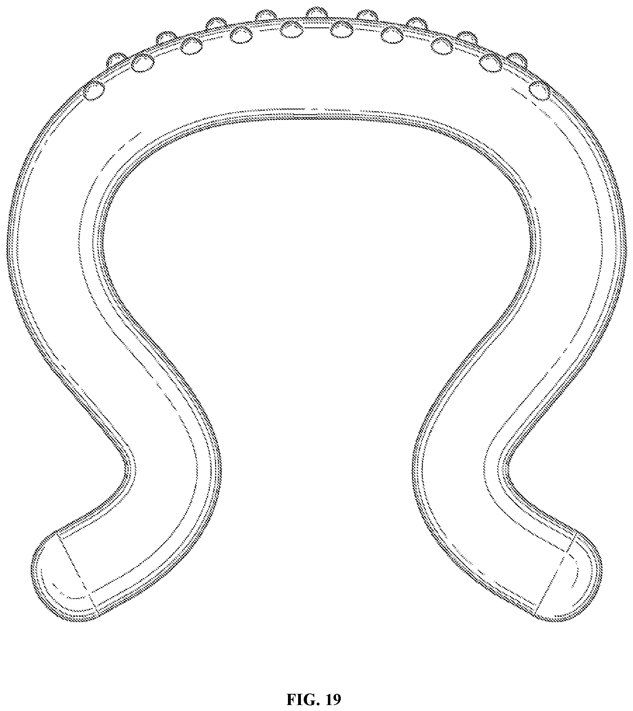

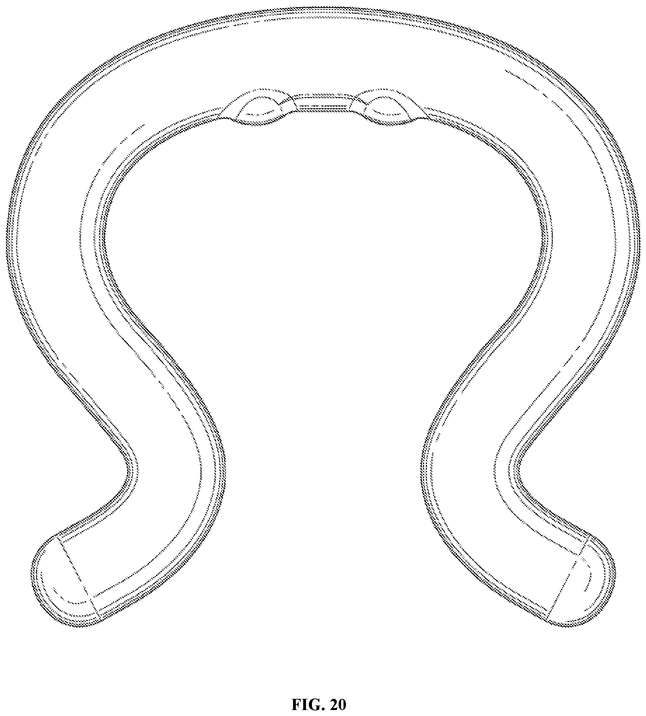

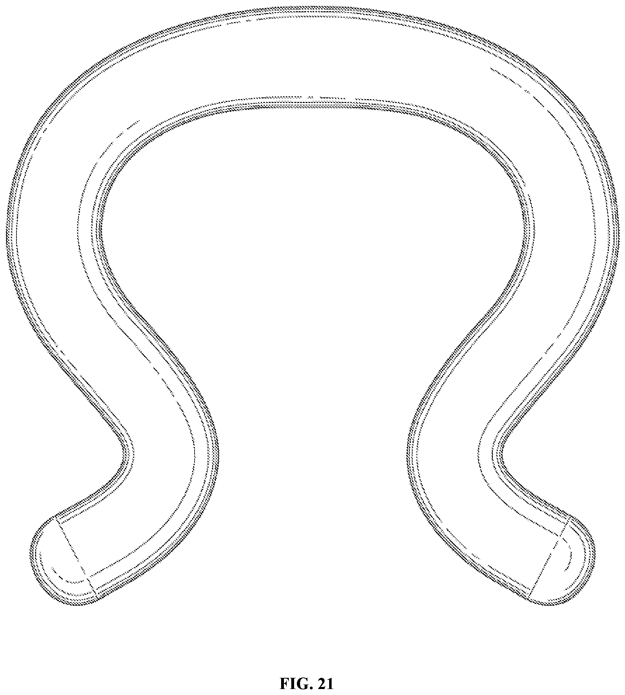

Optionally, in this or any other embodiment the penile constriction device does not comprise the first dorsal vein protrusion or the second dorsal vein protrusion. As shown in one embodiment, per **FIG. 19**, the penile constriction device comprises the friction protrusions and does not comprise the first dorsal vein protrusion or the second dorsal vein protrusion. Optionally, in this or any other embodiment, the penile constriction device does not comprise the friction protrusions. As shown in one embodiment, per **FIG. 20**, the penile constriction device comprises the first dorsal vein protrusion and the second dorsal vein protrusion and does not comprise the friction protrusions. Optionally, in this or any other embodiment, the penile constriction device does not comprise the friction protrusions, the first dorsal vein protrusion, or the second dorsal vein protrusion. As shown in one embodiment, per **FIG. 21**, the penile constriction device does not comprise the friction protrusion, the first dorsal vein protrusion, or the second dorsal vein protrusion.

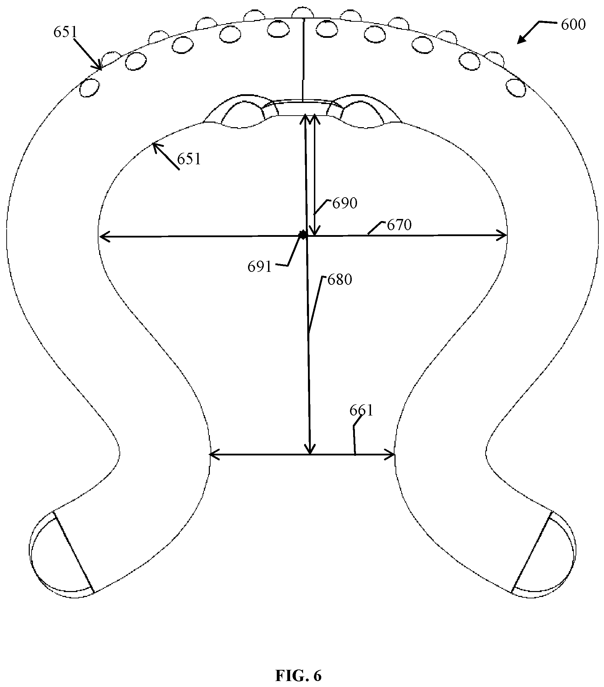

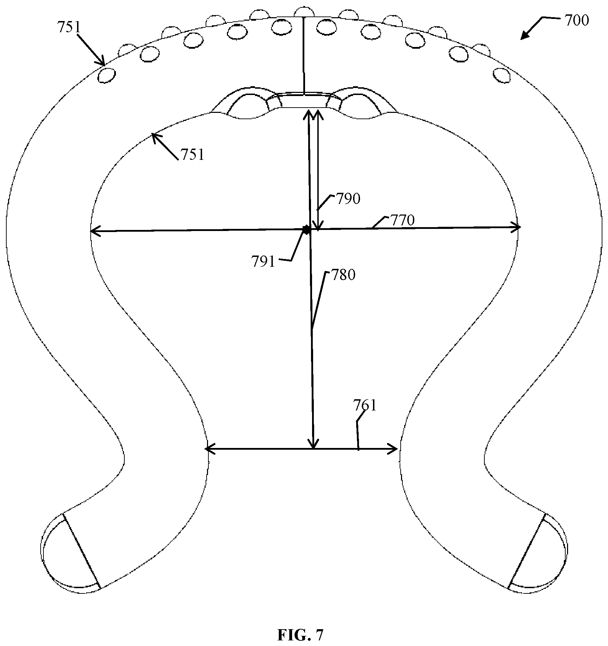

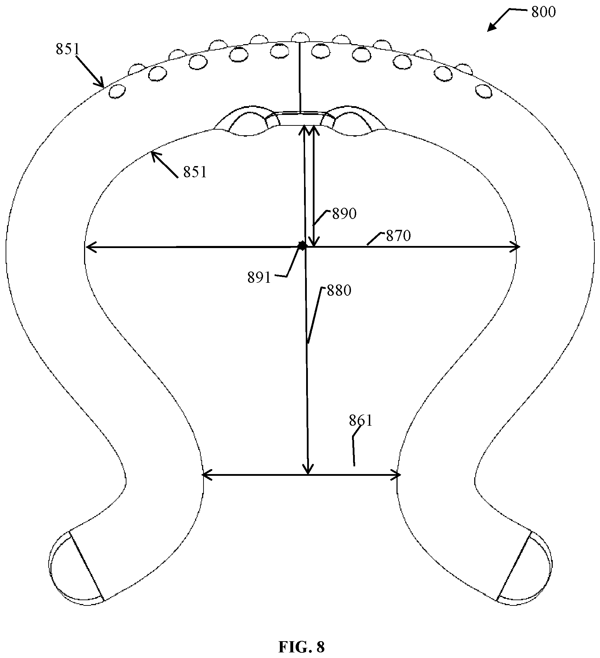

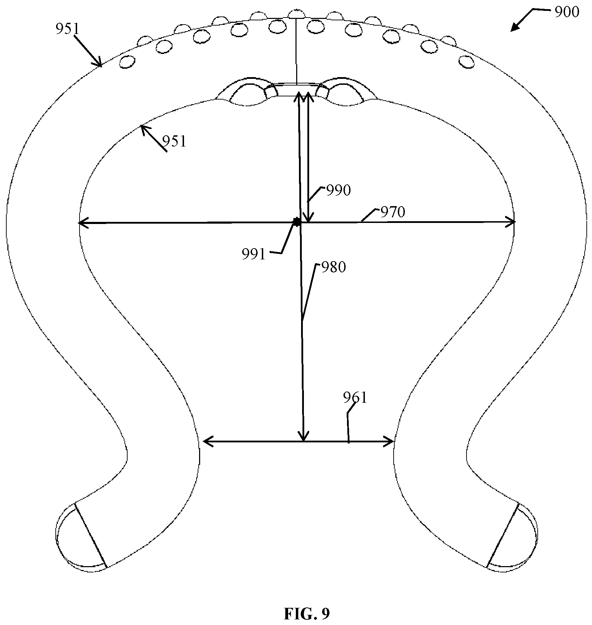

**FIG. 6** shows a front view of a non-limiting second example of a penile constriction device **600.** **FIG. 7** shows a front view of a non-limiting example of a third penile constriction device **700.** **FIG. 8** shows a front view of a non-limiting example of a fourth penile constriction device **800.** **FIG. 9** shows a front view of a non-limiting example of a fifth penile constriction device **900.**

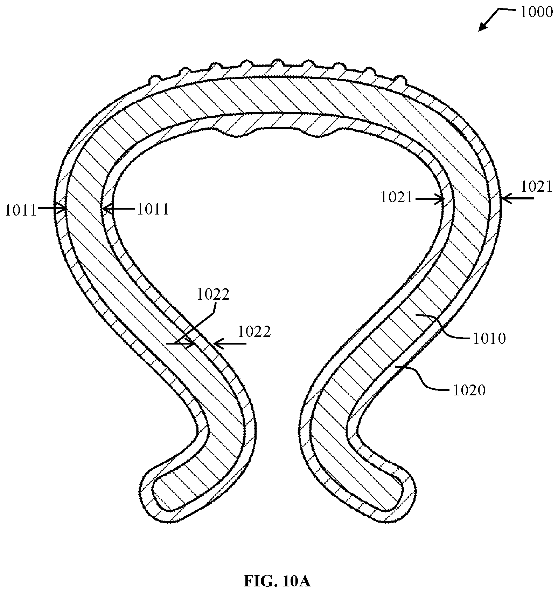

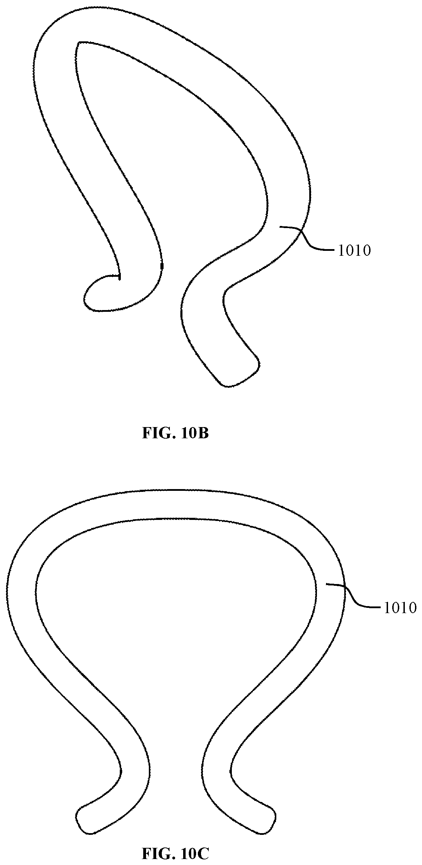

**FIG. 10** shows a front cross-sectioned view of a non-limiting example of a penile constriction device comprising a frame and a cover. Optionally, in this or any other embodiment, the penile constriction device **1000** comprises an arched rod comprising a cover **1020** substantially surrounding a frame **1010.**

Optionally, in this or any other embodiment, the frame **1010** comprises a frame portion of the first arm, a frame portion of the second arm, and a frame portion of the bridge. Optionally, in this or any other embodiment, the frame portion of the first arm is rigidly attached the frame portion of the bridge. Optionally, in this or any other embodiment, the frame portion of the second arm is rigidly attached to the frame portion of the bridge. Optionally, in this or any other embodiment, the frame portion of the first arm is flexibly attached to the frame portion of the bridge. Optionally, in this or any other embodiment, the frame portion of the second arm is flexibly attached to the frame portion of the bridge. Optionally, in this or any other embodiment, the frame portion of the first arm is removably attached the frame portion of the bridge. Optionally, in this or any other embodiment, the frame portion of the second arm is removably attached the frame portion of the bridge. Optionally, in this or any other embodiment, the frame portion of the first arm is not rigidly attached to the frame portion of the bridge. Optionally, in this or any other embodiment, the frame portion of the second arm is not attached the frame portion of the bridge.

Optionally, in this or any other embodiment, the cover **1020** comprises a cover portion of the first arm, a cover portion of the second arm, and a cover portion of the bridge. Optionally, in this or any other embodiment, the cover portion of the first arm is rigidly attached the cover portion of the bridge. Optionally, in this or any other embodiment, the cover portion of the second arm is rigidly attached to the cover portion of the bridge. Optionally, in this or any other embodiment, the cover portion of the first arm is flexibly attached to the cover portion of the bridge. Optionally, in this or any other embodiment, the cover portion of the second arm is flexibly attached to the cover portion of the bridge. Optionally, in this or any other embodiment, the cover portion of the first arm is removably attached the cover portion of the bridge. Optionally, in this or any other embodiment, the cover portion of the second arm is removably attached the cover portion of the bridge. Optionally, in this or any other embodiment, the cover portion of the first arm is not rigidly attached to the cover portion of the bridge. Optionally, in this or any other embodiment, the cover portion of the second arm is not attached the cover portion of the bridge.

Optionally, in this or any other embodiment, the frame **1010** comprises the compression region, wherein the cover **1020** surrounds the compression region. Optionally, in this or any other embodiment, the frame **1010** does not comprise the compression region, wherein the cover **1020** comprises the compression region. Optionally, in this or any other embodiment, both the frame **1010** and the cover **1020** comprise the compression region. Optionally, in this or any other embodiment, the frame **1010** comprises the plurality of friction protrusions, wherein the cover **1020** surrounds the plurality of friction protrusions. Optionally, in this or any other embodiment, the frame **1010** does not comprise the plurality of friction protrusions, wherein the cover **1020** comprises the plurality of friction protrusions. Optionally, in this or any other embodiment, both the frame **1010** and the cover **1020** comprise the plurality of friction protrusions. Optionally, in this or any other embodiment, the penile constriction device **1000** comprises an arched rod comprising a cover **1020** without a frame **1010.** Optionally, in this or any other embodiment, the penile constriction device **1000** comprises an arched rod comprising a frame **1010** without a cover **1020.**

As seen in **FIG. 10**, the frame **1010** is completely surrounded by the cover **1020.** Optionally, in this or any other embodiment, the cover **1020** completely surrounds a portion of the frame **1010.** Optionally, in this or any other embodiment, the cover **1020** does not surround at least a portion of the first terminus. Optionally, in this or any other embodiment, the cover **1020** does not surround at least a portion of the second terminus. Optionally, in this or any other embodiment, the cover **1020** is tubular in shape. Optionally, in this or any other embodiment, the cover **1020** does not comprise one or more of an appendage and a band. Optionally, in this or any other embodiment, the cover **1020** does not have a ribbed core. Optionally, in this or any other embodiment, the cover **1020** surrounds the frame **1010** of at least one of the first terminus and the second terminus. Optionally, in this or any other embodiment, the cover **1020** does not surround the frame **1010** of at least one of the first terminus and the second terminus.

Per **FIG. 10****,** the frame **1010** may comprise a frame thickness **1011** measured as a maximum outer distance of a cross-section of the frame **1010.** Optionally, in this or any other embodiment, the frame thickness **1011** is measured as a normal outer distance of a cross-section of the frame **1010.** Optionally, in this or any other embodiment, the frame thickness **1011** comprises a uniform inner thickness **1021.** Optionally, in this or any other embodiment, the frame thickness **1011** comprises a non-uniform inner thickness **1021.** Optionally, in this or any other embodiment, the frame thickness **1011** of the first arm **210** is greater than or equal to the frame thickness **1011** of the second arm **220.** Optionally, in this or any other embodiment, the frame thickness **1011** of the first arm **210** is less than to the frame thickness **1011** of the second arm **220.** Optionally, in this or any other embodiment, the frame thickness **1011** of the first arm **210** is greater than or equal to the frame thickness **1011** of the bridge **230.** Optionally, in this or any other embodiment, the frame thickness **1011** of the first arm **210** is less than to the frame thickness **1011** of the bridge **230.**

Per **FIG. 10****,** the cover **1020** may comprise a cover thickness **1021** measured as a maximum normal outer distance of the cross-section of the cover **1020.** Optionally, in this or any other embodiment, the cover thickness **1021** is measured as a maximum or a minimum outer distance of the cross-section of the cover **1020.** Optionally, in this or any other embodiment, the cover thickness **1021** comprises a uniform cover thickness **1021.** Optionally, in this or any other embodiment, the cover thickness **1021** comprises a non-uniform cover thickness **1021.**

Optionally, in this or any other embodiment, at least two of the cover portion of the first arm, the cover portion of the second arm, and the cover portion of the bridge have the same cover thickness **1021.** Optionally, in this or any other embodiment, at least two of the cover portion of the first arm, the cover portion of the second arm, and the cover portion of the bridge have the different cover thickness **1021.** Optionally, in this or any other embodiment, the cover thickness **1021** of at least a portion of the cover **1020** corresponds to the frame thickness **1011** of the frame **1010.** Optionally, in this or any other embodiment, the frame **1010** comprises the compression region, wherein the frame thickness **1011** of a portion of the frame **1010** within the compression region is greater than the frame thickness **1011** of a portion of the frame **1010** outside the compression region. Optionally, in this or any other embodiment, the frame **1010** does not comprise the compression region, wherein the cover **1020** comprises the compression region, and wherein the cover thickness **1021** of a portion of the cover **1020** within the compression region is greater than the cover thickness **1021** of a portion of the cover **1020** outside the compression region. Optionally, in this or any other embodiment, both the frame **1010** and the cover **1020** comprise the compression region, wherein the frame thickness **1011** of a portion of the frame **1010** within the compression region is greater than the frame thickness **1011** of a portion of the frame **1010** outside the compression region, and wherein the cover thickness **1021** of a portion of the cover **1020** within the compression region is greater than the cover thickness **1021** of a portion of the cover **1020** outside the compression region.

Per **FIG. 10****,** the cover **1020** may comprise a cover depth **1022** measured as a normal distance between the boundary of the frame **1010** and the cover **1020,** and the outer face of the cover **1020.** Optionally, in this or any other embodiment, the cover depth **1022** is measured as a minimum or maximum distance between the boundary of the frame **1010** and the cover **1020,** and a point on outer face of the cover **1020.** Optionally, in this or any other embodiment, the cover depth **1022** comprises a single cover depth **1022.** Optionally, in this or any other embodiment, the cover depth **1022** comprises a plurality of cover depths **1022.**

Optionally, in this or any other embodiment, a ratio between the frame thickness **1011** and the cover thickness **1021** is about 1: 1 to about 5:1. Optionally, in this or any other embodiment, a ratio between the frame thickness **1011** and the cover depth **1022** is about 1: 1 to about 10:1.

Optionally, in this or any other embodiment, the cover **1020** and the frame **1010** comprise the same material. Optionally, in this or any other embodiment, the cover **1020** and the frame **1010** comprise different materials. Optionally, in this or any other embodiment, at least one of the cover **1020** and the frame **1010** is pliable. Optionally, in this or any other embodiment, at least one of the cover **1020** and the frame **1010** is ductile. Optionally, in this or any other embodiment, at least one of the cover **1020** and the frame **1010** is pliable and ductile. Optionally, in this or any other embodiment, at least one of the cover **1020** and the frame **1010** is pliable and not ductile. Optionally, in this or any other embodiment, at least one of the cover **1020** and the frame **1010** is ductile and not pliable. Optionally, in this or any other embodiment, the cover **1020** is formed of a material that is safe for prolonged human contact, and which does not irritate or cut the skin the penile area.

Optionally, in this or any other embodiment, a frame modulus of elasticity of the frame **1010** of the first arm is higher than a frame modulus of elasticity of the frame **1010** of the second arm. Optionally, in this or any other embodiment, the frame modulus of elasticity of the frame **1010** of the first arm is less than or equal to the frame modulus of elasticity of the frame **1010** of the second arm. Optionally, in this or any other embodiment, a frame modulus of elasticity of the frame **1010** of the first arm is higher than a frame modulus of elasticity of the frame **1010** of the bridge. Optionally, in this or any other embodiment, the frame modulus of elasticity of the frame **1010** of the first arm is less than or equal to the frame modulus of elasticity of the frame **1010** of the bridge.

Optionally, in this or any other embodiment, at least one of the cover **1020** and the frame **1010** comprise plastic, silicone, glass, wood, metal, carbon fiber, fiberglass, or any combination thereof. Optionally, in this or any other embodiment, at least one of the cover **1020** and the frame **1010** comprise polyamide, polycarbonate, polyester, polyethylene, polyethylene terephthalate, polypropylene, polystyrene, polyurethanes, polyvinyl chloride, polyvinylidene chloride acrylonitrile butadiene styrene acrylonitrile butadiene styrene, polyethylene/acrylonitrile butadiene styrene, polyepoxide, polymethyl methacrylate, polytetrafluoroethylene, phenolics melamine formaldehyde urea-formaldehyde, polyetheretherketone, maleimide/bismaleimide, polyetherimide, polyimide, polylactic acid, furan silicone, polysulfone, or any combination thereof.

Optionally, in this or any other embodiment, the frame thickness is about 0.005 inches to about 0.55 inches. Optionally, in this or any other embodiment, the frame thickness is at least about 0.005 inches. Optionally, in this or any other embodiment, the frame thickness is at most about 0.55 inches. Optionally, in this or any other embodiment, the cover thickness is about 0.08 inches to about 0.6 inches. Optionally, in this or any other embodiment, the cover thickness is at least about 0.08 inches. Optionally, in this or any other embodiment, the cover thickness is at most about 0.6 inches. Optionally, in this or any other embodiment, the cover depth is about 0.005 inches to about 0.55 inches. Optionally, in this or any other embodiment, the cover depth is at least about 0.005 inches. Optionally, in this or any other embodiment, the cover depth is at most about 0.55 inches.

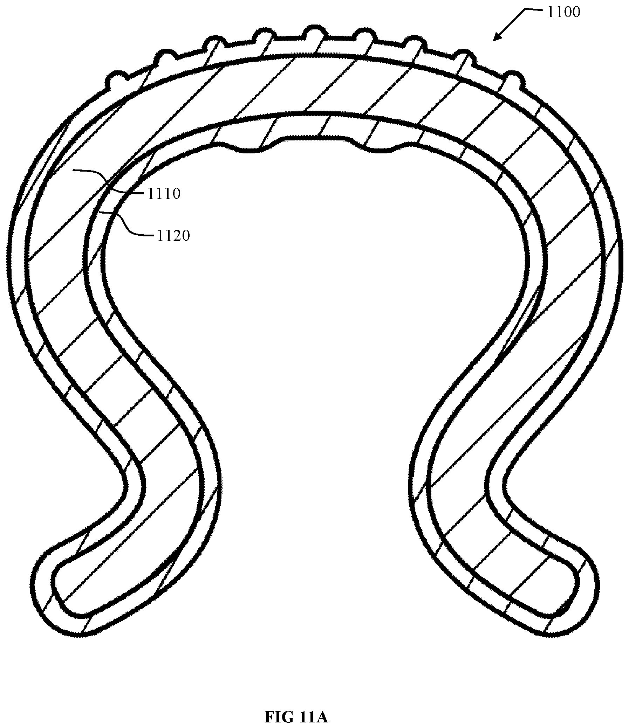

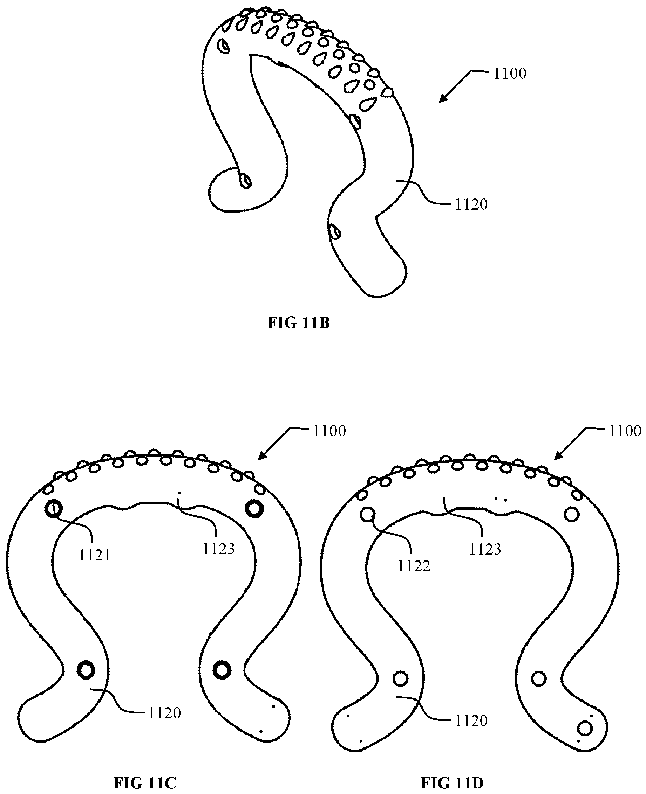

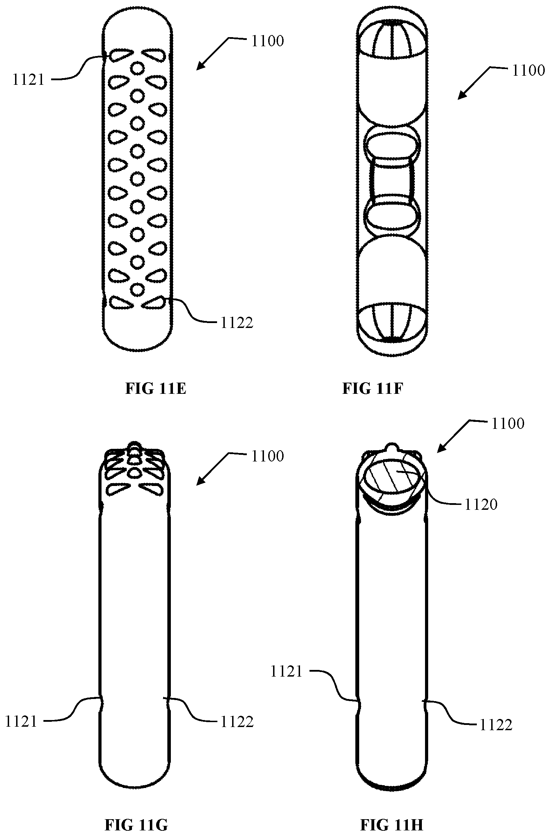

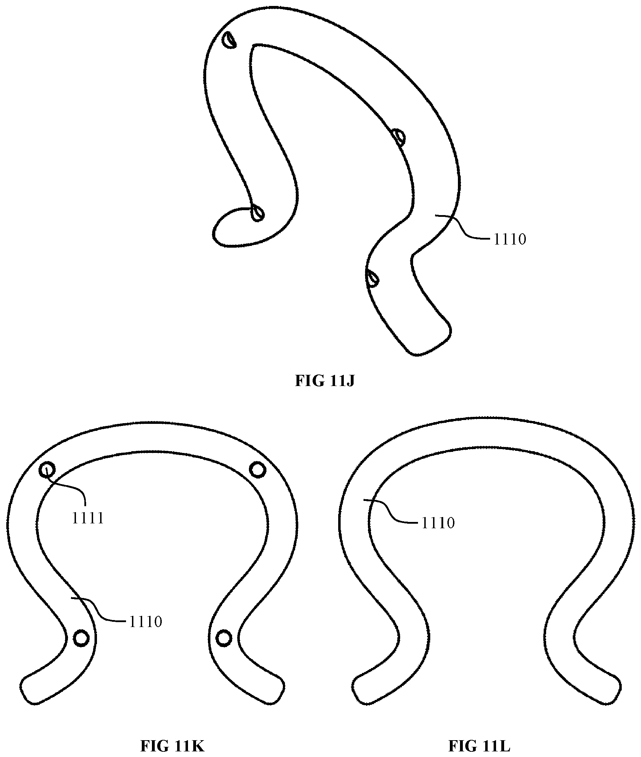

**FIG. 11A** shows a front cross-sectioned view of an alternative non-limiting example of a penile constriction device **1100** comprising a frame **1110** and a cover **1120.** **FIGS. 11B-H** show the manufacturing marks of penile constriction device **1100** of **FIG. 11A**. As seen in **FIGS. 11A****H,** at least a portion of the cover **1120** surrounds the frame **1110.** Per **FIGS. 11A-H****,** the cover **1120** of the penile constriction device **1100** comprises a first cover manufacturing mark **1121,** a second cover manufacturing mark **1123,** and a third cover manufacturing mark **1123.**

**FIGS. 11J-L** show a non-limiting example of the frame **1220** of **FIG. 11A**. Per **FIGS. 11J-L****,** the frame **1110** of the penile constriction device **1100** comprises a first frame manufacturing mark **1111.** Optionally, in this or any other embodiment, at least one of the first cover manufacturing mark **1121,** the second cover manufacturing mark **1123,** and the third cover manufacturing mark **1123** are configured to allow the cover **1210** to be over-molded around the frame **1110.** Optionally, in this or any other embodiment, at least one of the first cover manufacturing mark **1211,** the second cover manufacturing mark **1123,** and the third cover manufacturing mark **1123** comprise a witness mark.

As seen in **FIGS. 11J-L** the penile constriction device **1100** comprises the four first manufacturing marks **1121,** five second manufacturing marks **1123,** and ten third manufacturing marks **1123.**

Alternatively, the number of first cover manufacturing marks **1121** is optionally about 1 to about 10. Alternatively, the number of second cover manufacturing marks **1122** is optionally about 1 to about 10. Alternatively, the number of third cover manufacturing marks **1123** is optionally about 1 to about 20.

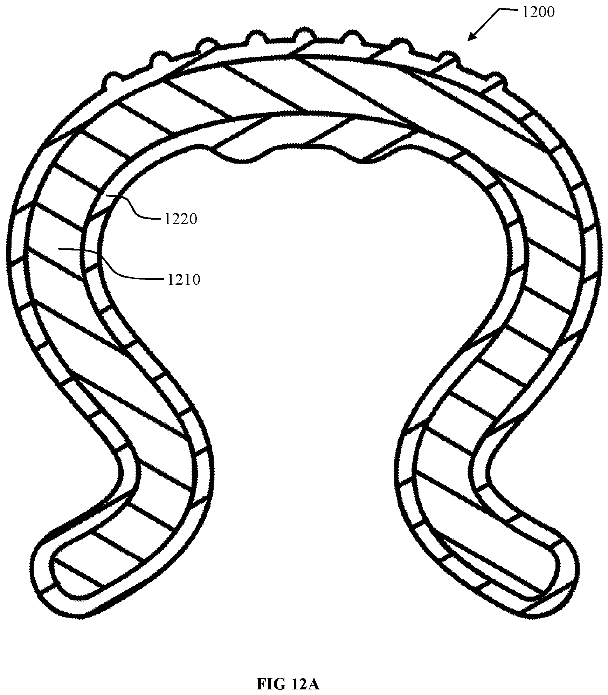

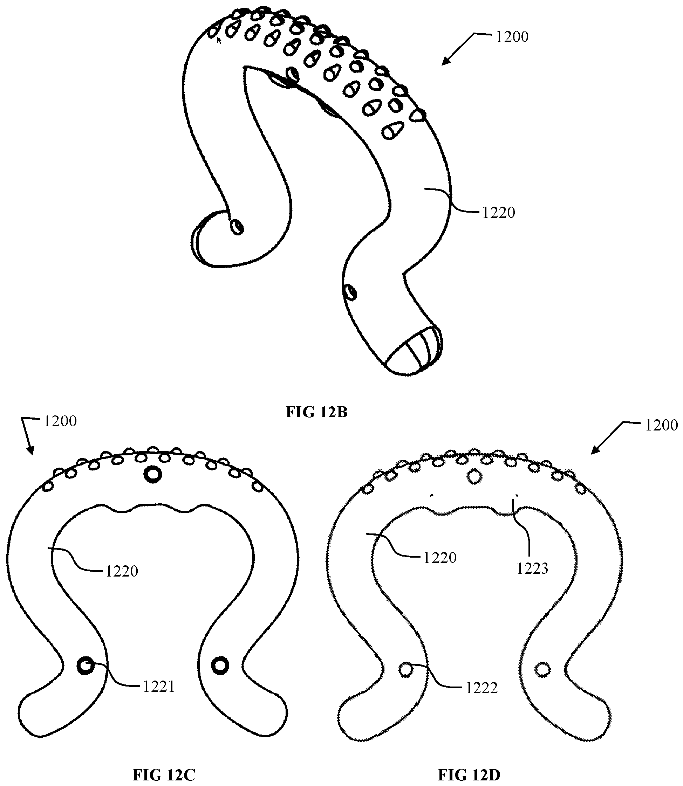

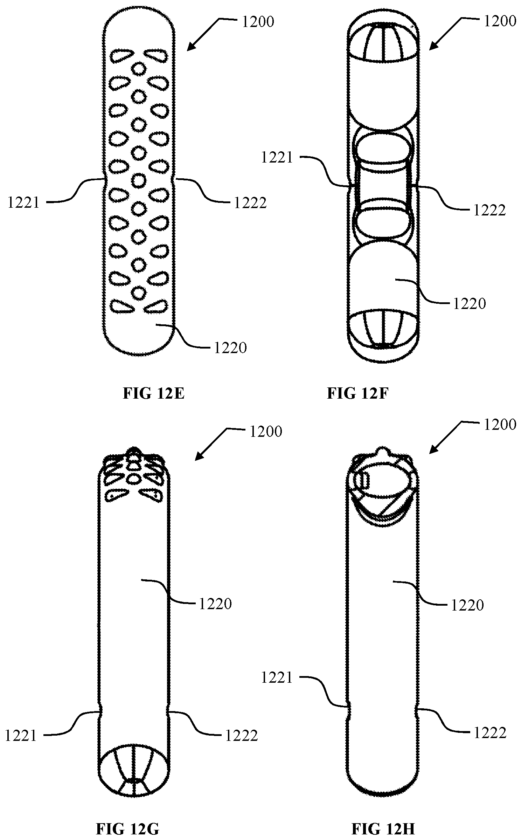

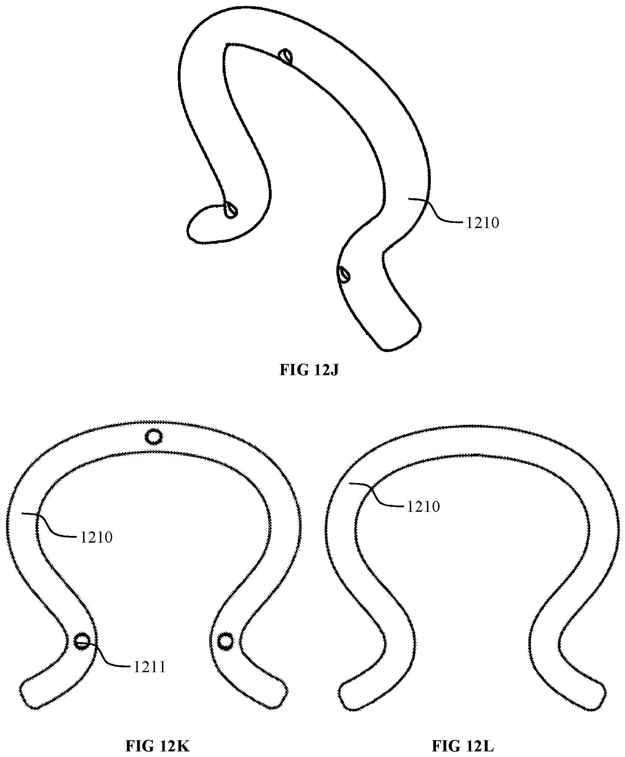

**FIG. 12A** shows a front cross-sectioned view of another alternative non-limiting example of a penile constriction device comprising a frame and a cover **FIGS. 12B-H** show the manufacturing marks of penile constriction device of **FIG. 12A****.** **FIGS. 12J-L** show a non-limiting example of the frame of **FIG. 12A**.

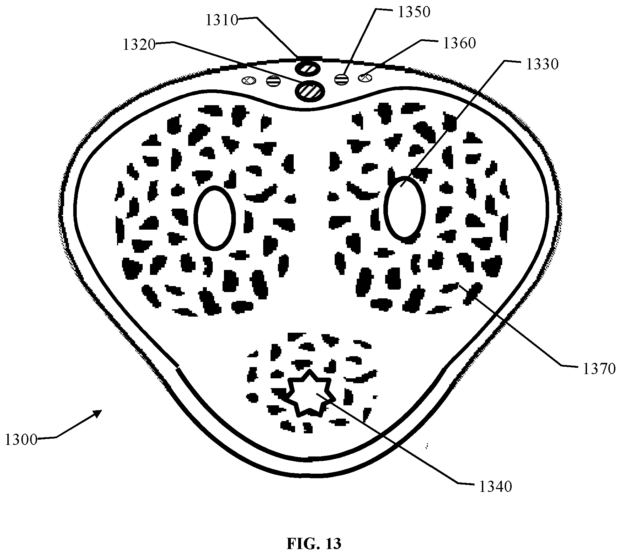

**FIG. 13** shows a front cross-sectioned view of the veins and arteries in a human penis. Per **FIG. 13** the human penis **1300** comprises a superficial dorsal vein **1310,** a deep dorsal vein **1320,** deep arteries **1330,** a urethra **1340,** dorsal arteries **1350,** and dorsal nerves **1360.** The average human penis **1300** has a general cross section shape comprising an ellipse toward the top portion and a smaller ellipse cross section shape toward the bottom portion. Erection is enabled in men through increased blood flow into the penis **1300** through the deep arteries **1330,** and decreased blood flow out from the penis **1300** through the superficial dorsal vein **1310** and the deep dorsal vein **1320.**

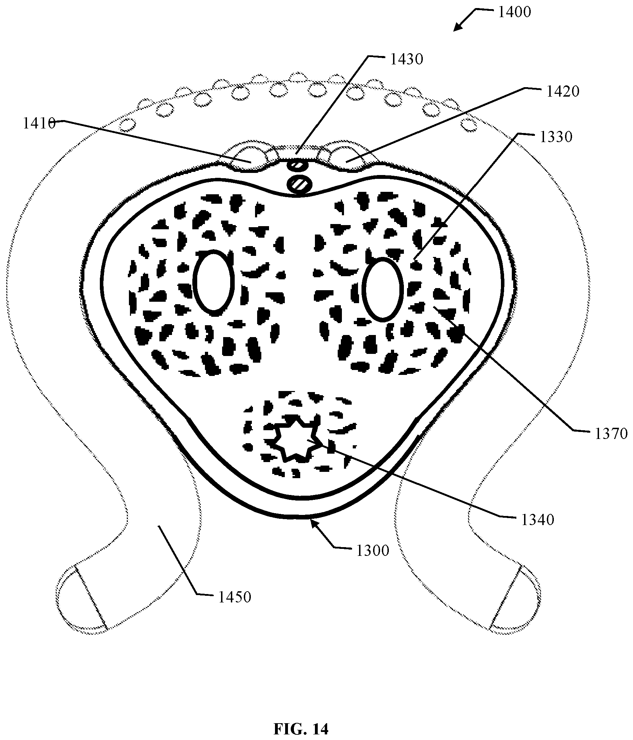

**FIG. 14** shows a front cross-sectioned view of a non-limiting example of a penile constriction device on a human penis. Optionally, in this or any other embodiment, at least one of the shape and elasticity of device **1400** is configured to prevent outwards blood flow from the penis **1300** to the body. Optionally, in this or any other embodiment, at least one of the shape and elasticity of device **1400** is configured to enable and/or not hinder blood flow from the body to the penis **1300.**

Optionally, in this or any other embodiment, at least one of the shape and elasticity of the device **1400** is configured to provide at least one of the non-uniform pressure and the selective non-uniform pressure is to at least one of a flaccid penis, a slightly flaccid penis, a slightly rigid penis, and an erect penis. Optionally, in this or any other embodiment, the at least one of the non-uniform pressure and the selective non-uniform pressure is to at least one of a flaccid penis, a slightly flaccid penis, a slightly rigid penis, and an erect penis **1300** enables the device to facilitate erection initiation. Optionally, in this or any other embodiment, at least one of the non-uniform pressure and the selective non-uniform pressure is to at least one of a flaccid penis, a slightly flaccid penis, a slightly rigid penis, and an erect penis **1300** enables to device **1400** to aid in erection stamina. Optionally, in this or any other embodiment, the at least one of the non-uniform pressure and the selective non-uniform pressure is to at least one of a flaccid penis, a slightly flaccid penis, a slightly rigid penis, and an erect penis **1300** enables to device **1400** to facilitate erection initiation and erection stamina.

Optionally, in this or any other embodiment, at least one of the shape and elasticity of the device **1400** is configured to provide at least one of the non-uniform pressure and the selective non-uniform pressure to compress at least one of the girth and the cross section area of a flaccid penis, a slightly flaccid penis, a slightly rigid penis, and an erect penis.

Optionally, in this or any other embodiment, at least one of the shape and elasticity of an arched rod **1450** is configured to a compress the penis **1300** against at least one of the first dorsal vein protrusion **1410,** the second dorsal vein protrusion **1420,** and the dorsal valley **1430.** Optionally, in this or any other embodiment, a compressive force applied to the penis **1300** by at least one of the first dorsal vein protrusion **1410,** the second dorsal vein protrusion **1420,** and the dorsal valley **1430,** constricts blood flowing out of the penis **1300** through at least the superficial dorsal vein **1310** and the deep dorsal vein **1320.** Optionally, in this or any other embodiment, a compressive force applied to the penis **1300** by at least one of the first dorsal vein protrusion **1410,** the second dorsal vein protrusion **1420,** and the dorsal valley **1430,** does not constrict at least one of the dorsal arteries **1350** and the urethra **1340.** Optionally, in this or any other embodiment, the device **1400** applies pressure to the veins of the penis to reduce blood flow out of the penis. This allows for the pressure to build up in the corpus cavernosa **1370** to achieve and/or maintain an erection. Optionally, in this or any other embodiment, the device **1400** has no negative effect on the dorsal nerve **1360.**

Optionally, in this or any other embodiment, the constriction gap width of the device **1400** is configured such that an erect or flaccid penis having an erect diameter and a flaccid diameter, respectively, can pass through the constriction gap. Optionally, in this or any other embodiment, the constriction gap width is configured such that an erect or flaccid penis, having an erect diameter and a flaccid diameter, respectively, can pass through the constriction gap when an opposing force is applied between the first converging end and the second converging end. Optionally, in this or any other embodiment, the device **1400** applies pressure to the veins of the penis to reduce blood outflow from the penis. Such outflow allows for the pressure to build up in the corpus cavernosa **1370** to achieve and or maintain an erection. Optionally, in this or any other embodiment, the device **1400** has no negative effect on the dorsal nerve.

Optionally, in this or any other embodiment, the constriction gap width is configured to impart minimal compressive force on the urethra, when worn on an erect penis. Optionally, in this or any other embodiment, the constriction height of the device **1400** is configured to impart minimal compressive force on the urethra **1340,** when worn on an erect penis. Optionally, in this or any other embodiment, the constriction height is configured to impart maximal compressive force on the deep arteries **1330,** on an erect or a flaccid penis having an erect diameter and a flaccid diameter, respectively. Optionally, in this or any other embodiment, the maximum inner width of the device **1400,** is configured to impart minimal or negligible compressive force on the deep arteries **1330,** the urethra **1340,** and the dorsal nerves **1360** of an erect or a flaccid penis having an erect diameter and a flaccid diameter, respectively. Optionally, in this or any other embodiment, the inferior height is configured to impart a significant compressive force on at least one of the superficial dorsal vein **1310** and the deep dorsal vein **1320.** Optionally, in this or any other embodiment, the thickness of the device **1400** is configured to provide at least one of a sufficient elasticity, rigidity, and pliability to enable the device **1400** to provide a specific pressure against an erect or a flaccid penis having an erect diameter and a flaccid diameter, respectively.

Optionally, in this or any other embodiment, at least one of the first dorsal vein protrusion **1410,** the second dorsal vein protrusion **1420,** and the dorsal valley **1430** are configured to apply no force, or a minimal force to at least one of the dorsal arteries **1350** and the urethra **1340.** Optionally, in this or any other embodiment, at least one of the shape and elasticity of the device **100** is configured to apply a non-uniform pressure to the penis **1300.** Optionally, in this or any other embodiment, at least one of the shape and elasticity of the device **100** is configured to apply a selective non-uniform pressure to the penis **1300.** Optionally, in this or any other embodiment, at least one of the non-uniform pressure and the selective non-uniform pressure is configured to allow at least one of blood and seminal fluids to flow into the penis **1300.** Optionally, in this or any other embodiment, at least one of the non-uniform pressure and the selective non-uniform pressure is configured to allow a user to perform Kegel exercises to increase blood flow into the penis **1300.** Optionally, in this or any other embodiment, at least one of the non-uniform pressure and the selective non-uniform pressure is configured to maintain the health of at least one of cells, arteries, and veins within the penis **1300.** Optionally, in this or any other embodiment, at least one of the shape and elasticity of the device **100** is configured to apply a greater or equal pressure to the penis **1300** at the compression region than by at least one of the first arm and the second arm. Optionally, in this or any other embodiment, at least one of the shape and elasticity of the device **100** is configured to apply less pressure to the penis **1300** at the compression region than by at least one of the first arm and the second arm.

Optionally, in this or any other embodiment, at least one of the dorsal vein valley distance, the compression region width, the first dorsal height, the second dorsal height, the maximum inner width, the constriction distance, and the constriction gap are configured to constrict blood flowing out of the penis **1300** through at least the superficial dorsal vein **1310** and the deep dorsal vein **1320.** Optionally, in this or any other embodiment, at least one of the dorsal vein valley distance, the compression region width, the first dorsal height, the second dorsal height, and the constriction gap are configured to apply no force, or a minimal force to at least one of the dorsal arteries **1350** and the urethra **1340.** Optionally, in this or any other embodiment, at least one of the dorsal vein valley distance, the compression region width, the first dorsal height, the second dorsal height, the maximum inner width, the constriction distance, and the constriction gap are configured to constricts blood flowing out of the penis **1300** through at least the superficial dorsal vein **1310** and the deep dorsal vein **1320** and to apply no force, or a minimal force to at least one of the dorsal arteries **1350** and the urethra **1340.**

Optionally, in this or any other embodiment, the elasticity of at least one of the first arm, the second arm, and the bridge enables the device **1400** to provide continuous pressure on both a flaccid and erect penis **1300.** Optionally, in this or any other embodiment, the pliability of at least one of the first arm, the second arm, and the bridge enables the device **1400** to provide continuous pressure on flaccid penis **1300** as it becomes erect.

Optionally, in this or any other embodiment, at least one of the shape and elasticity of the device **100** is configured to provide at least one of the non-uniform pressure and the selective non-uniform pressure that compresses at least one of the girth of the penis **1300** and the cross section area of the penis **1300** by at least about 10%. Optionally, in this or any other embodiment, at least one of the shape and elasticity of the device **100** is configured to provide at least one of the non-uniform pressure and the selective non-uniform pressure that compresses at least one of the girth of the penis **1300** and the cross section area of the penis **1300** by at most about 60%. Optionally, in this or any other embodiment, at least one of the shape and elasticity of the device **100** is configured to provide at least one of the non-uniform pressure and the selective non-uniform pressure that compresses at least one of the girth of the penis **1300** and the cross section area of the penis **1300** by about 10% to about 60%. Optionally, in this or any other embodiment, at least one of the shape and elasticity of the device **100** is configured to provide at least one of the non-uniform pressure and the selective non-uniform pressure that compresses at least one of the girth of the penis **1300** and the cross section area of the penis **1300** by about 15% to about 45%. Optionally, in this or any other embodiment, at least one of the shape and elasticity of the device **100** is configured to provide at least one of the non-uniform pressure and the selective non-uniform pressure that compresses at least one of the girth of the penis **1300** and the cross section area of the penis **1300** by about 37%.

Optionally, in this or any other embodiment, the negligible or minimal force applied by the device **100** on the dorsal arteries **1350** allows blood and oxygen to flow into the penis **1300 1300,** with or without the aid of Kegel exercises, which prevents damage to the cells of the penis **1300 1300** and to ensure their ability to achieve an erection. Optionally, in this or any other embodiment, the negligible or minimal force applied by the device **100** on the urethra **1340** enables the release of pre-seminal fluid that act as a lubricant during sexual intercourse, masturbation, or both. Optionally, in this or any other embodiment, the negligible or minimal force applied by the device **100** on the urethra **1340** prevents injaculation or retrograde ejaculation, wherein a blockage within the seminal vesicle causes semen to flow backwards into the bladder. Such injaculation causes infertility, by damaging the perineum, nerves, and blood vessels within the male reproductive system. Optionally, in this or any other embodiment, at least one of the first elbow **214** and the second elbow **224** are configured to prevent abrasion or lacerations of the penis **1300.**

Optionally, in this or any other embodiment, at least one of the shape and elasticity of the device **100** is configured to avoid inhibiting male pleasure during intercourse. Optionally, in this or any other embodiment, at least one of the first dorsal vein protrusion, the second dorsal vein protrusion, and the dorsal valley are configured to prevent rotation of the device **100** about the penis **1300.**

In some embodiments, the device **100** comprises one or more tactile size indications to indicate size. In some embodiments, the tactile size indication is concave relative to at least one surface of the device. In some embodiments, the tactile size indication is convex relative to at least one surface of the device. In some embodiments, the device **100** does not comprise a valve. In some embodiments, the device **100** is not inflatable. In some embodiments, the device **100** does not comprise a valve. In some embodiments, the device **100** is not inflated. In some embodiments, the device **100** is not pressurized. In some embodiments, the device **100** is configured for use during intercourse.

### Penile Constriction Systems

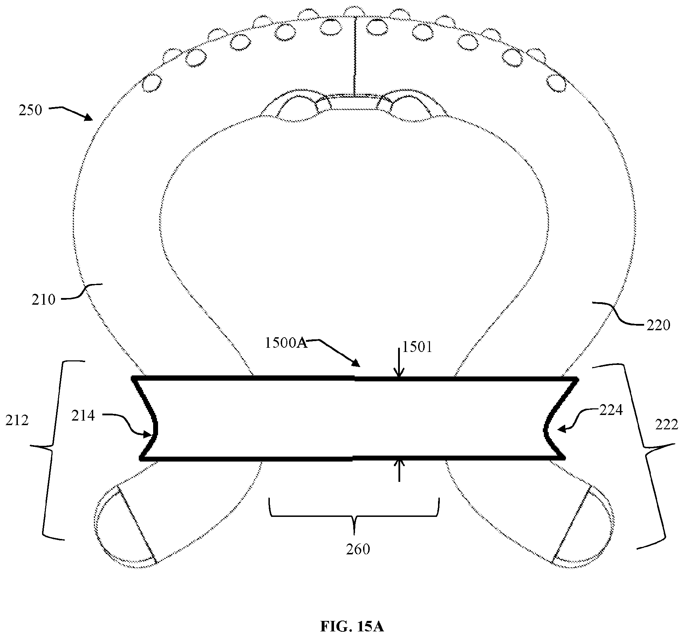

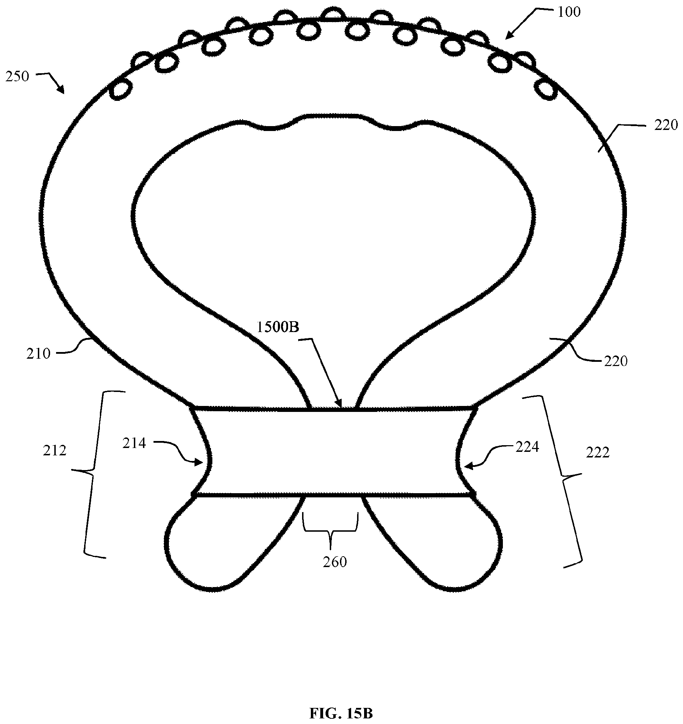

**FIG. 15A** shows a front view of a non-limiting example of a penile constriction system comprising an exemplary penile constriction device and a band. **FIG. 15B** shows a front view of a non-limiting example of a penile constriction system comprising an exemplary penile constriction device and a high tension band. Provided herein, per **FIGS. 15A-B****,** is a system comprises a penile constriction device **100** and a band **1500.** Optionally, in this or any other example, the band **1500** is configured to attach to the first converging end **212** and the second converging end **212** of the device **100.** Optionally, in this or any other example, the band **1500** is configured to attach to the first elbow **214** and at least one of the second elbow **224,** the second converging end **222,** and the second terminus **223** of the device **100.** Optionally, in this or any other example, the band **1500** is configured to attach to the first converging end **212** and at least one of the second elbow **224,** the second converging end **222,** and the second terminus **223** of the device **100.** Optionally, in this or any other example, the band **1500** is configured to attach to the first terminus **213** and at least one of the second elbow **224,** the second converging end **222,** and the second terminus **223** of the device **100.** In some examples, the system provided herein does not comprise a pump.

### Penile Constriction Bands

Optionally, in this or any other example, at least one of the first arm **210,** the second arm **220,** the first converging end **212,** the second converging end **222,** first elbow **214,** and the second elbow **224** of the arched rod device **100** is configured to couple to a band **1500.** Optionally, in this or any other example, the arched rod **250** does not comprise the band **1500.** Optionally, in this or any other example, the penile constriction device **100** does not comprise the band **1500.**

Optionally, in this or any other example, the band **1500** is configured to removably attach, temporarily attach, or both to the device **100.** Optionally, in this or any other example, the band **1500** is rigidly attached to the device **100.** Optionally, in this or any other example, the band is separate and distinct from the device. Optionally, in this or any other example, the band is not rigidly attached to the device. Optionally, in this or any other example, the band is removably attached to the first converging end, the second converging end, or the bridge of the device **100.**

Optionally, in this or any other example, the band **1500** is configured to impart a compressive force between the first converging **212** and the second converging **212** of the device **100.** Optionally, in this or any other example, the band **1500** is configured to impart a compressive force between the first elbow **214** and the second elbow **224** of the device **100.** Optionally, in this or any other example, the band **1500** is configured to impart a compressive force between the first converging **212** and the second elbow **224** of the device **100.** Optionally, in this or any other example, the band **1500** is configured to impart a compressive force between the second converging **212** and the first elbow **214** of the device **100.**

Optionally, in this or any other example, per **FIG. 15A****,** the band **1500** comprises a low tension band **1500A.** Alternatively, in this or any other example, per **FIG. 15B****,** the band **1500** comprises a high tension band **1500B,** wherein the high tension band **1600** is configured to impart a greater force between the first arm **210** and the second arm **220** than the low tension band **1500.**

Optionally, in this or any other example, the band **1500** is configured to provide increased penile compressive force. Optionally, in this or any other example, the penile compressive force compresses the cross section area of the penis by at least about 10%. Optionally, in this or any other example, the penile compressive force compresses the cross section area of the penis by at most about 60%. Optionally, in this or any other example, the penile compressive force compresses the cross section area of the penis by about 10% to about 60%. Optionally, in this or any other example, the penile compressive force compresses the cross section area of the penis by about 37%. Optionally, in this or any other example, the increased penile compressive force prevents the device **100** from rotating or translating from its originally worn position during intercourse.

Optionally, in this or any other example, per **FIG. 15A****,** the shape and elasticity of at least one of the band **1500** and the device **100** are configured such that, the shape of the device is relatively undeformed when the band **1500** is affixed to the device **100.** Optionally, in this or any other example, the shape and elasticity of at least one of the band **1500** and the device **100** are configured such that the band **1500** only slightly increases the penile compressive force (e.g. by less than about 10%), whereby the band **1500** is configured to increase device **100** stability about the penis by acting as a friction surface.

Optionally, in this or any other example, per **FIG. 15B****,** the shape and elasticity of at least one of the band **1500** and the device **100** are configured such that, the shape of the device is significantly deformed when the band **1500** is affixed to the device **100.** Optionally, in this or any other example, the shape and elasticity of at least one of the band **1500** and the device **100** are configured such that the band **1500** substantially increases the penile compressive force (e.g. by more than about 10%), whereby the band **1500** is configured to decrease at least one of the constriction gap, the constriction height, the maximum inner width, and the inferior height of the device **100.** Optionally, in this or any other example, the shape and elasticity of at least one of the band **1500** and the device **100** are configured such that, the shape of the device is significantly deformed when the band **1500** is affixed to the device **100,** to allow the device **100** to be employed on a penis with a smaller girth or circumference.

Optionally, in this or any other example, per **FIG. 15A****,** the band **1500** comprises a band width **1501.** Optionally, in this or any other example, the band width **1501** is about 0.1 inches to about 1.5 inches. Optionally, in this or any other example, the band width **1501** is at least about 0.1 inches. Optionally, in this or any other example, the band width **1501** is at most about 1.5 inches. Optionally, in this or any other example, the band **1500** has a spring constant of about 0.1 pounds/inches to about 1 pound/inch. Optionally, in this or any other example, the band **1500** has a spring constant of at least about 0.1 pounds/inches. Optionally, in this or any other example, the band **1500** has a spring constant of at most about 1 pound/inch.

Optionally, in this or any other example, the band **1500** is elastic. Optionally, in this or any other example, the band **1500** is inelastic. Optionally, in this or any other example, the band **1500** is rigid. Optionally, in this or any other example, the band **1500** comprises a spring, a loop, a cam, a hook or any combination thereof. Optionally, in this or any other example, the bands herein are soft to the touch and/or non-abrasive. Optionally, in this or any other example, the bands herein are non-allergenic. Optionally, in this or any other example, the bands herein are formed from a material that is washable. Optionally, in this or any other example, the bands herein have a sufficient tensile strength to prevent breaking or tearing when installing the band between the first elbow and the second elbow of the device **100.** Optionally, in this or any other example, the bands herein have a sufficient modulus of elasticity such that the band can be stretched by hand over first converging end and the second converging end and onto the first elbow and the second elbow of the device **100.** Optionally, in this or any other example, the bands herein have a sufficient durometer to prevent breaking or tearing during over-extension and frequent reuse. Optionally, in this or any other example, a surface of the bands herein have a texture that prevents the bands from sliding off the device **100.**

Optionally, in this or any other example, the bands herein are formed from a thermoplastic elastomer. Optionally, in this or any other example, the band comprises rubber, plastic, metal, wood, silicone, Kevlar, string, fabric, or any combination thereof. Optionally, in this or any other example, the band comprises plastic, glass, wood, metal, carbon fiber, fiberglass, or any combination thereof. Optionally, in this or any other example, the band comprises polyamide, polycarbonate, polyester, polyethylene, polyethylene terephthalate, polypropylene, polystyrene, polyurethanes, polyvinyl chloride, polyvinylidene chloride acrylonitrile butadiene styrene acrylonitrile butadiene styrene, polyethylene/acrylonitrile butadiene styrene, polyepoxide, polymethyl methacrylate, polytetrafluoroethylene, phenolics melamine formaldehyde urea-formaldehyde, polyetheretherketone, maleimide/bismaleimide, polyetherimide, polyimide, polylactic acid, furan silicone, polysulfone, or any combination thereof. Optionally, in this or any other example, the bands herein are not formed from PVC, latex, phthalates, or any combination thereof.

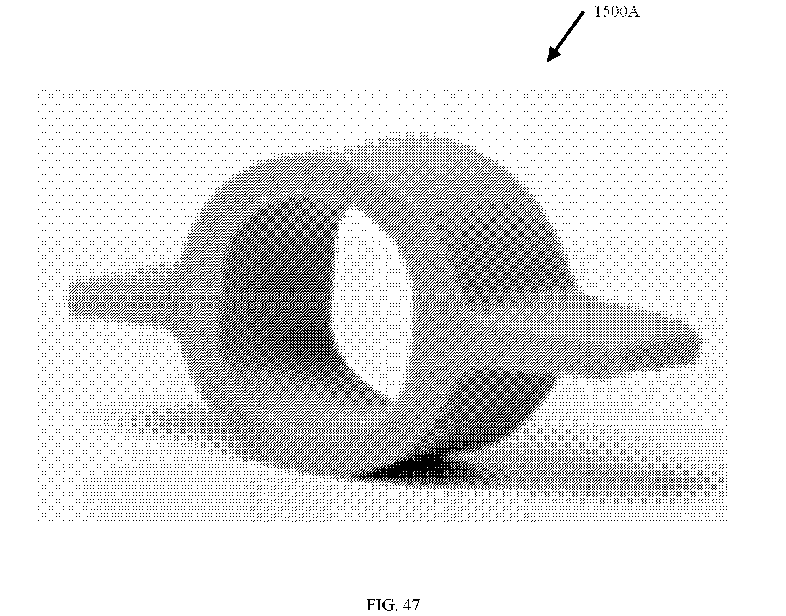

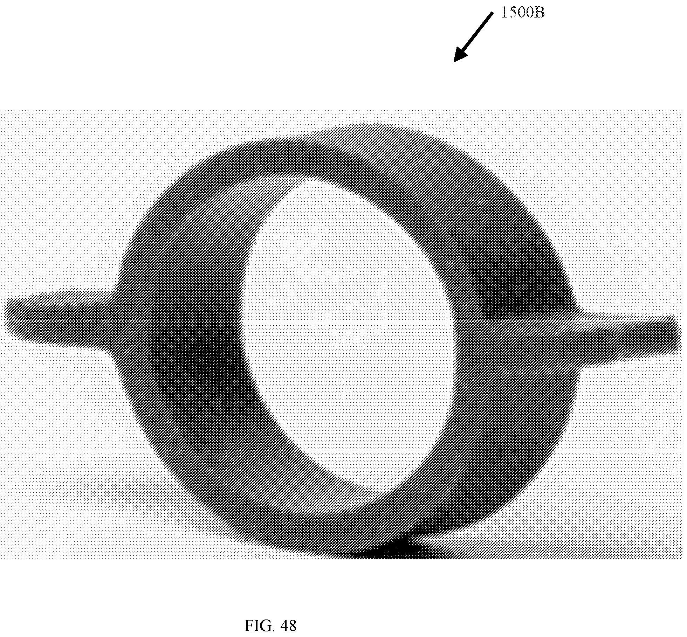

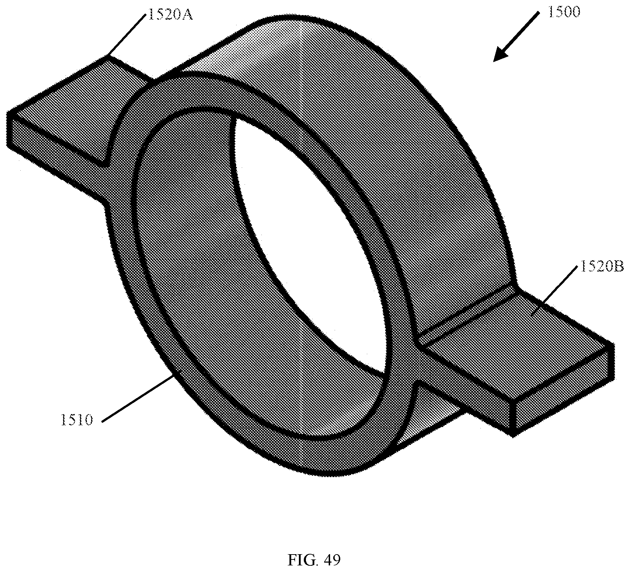

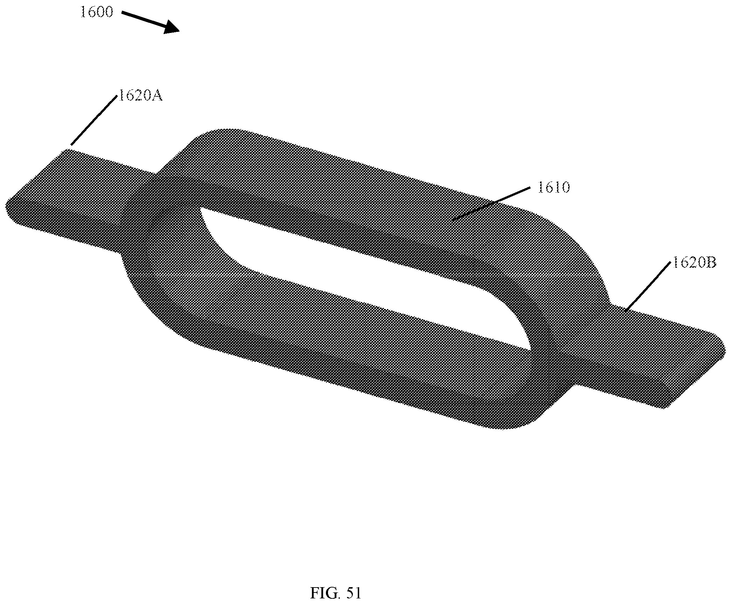

**FIGS. 47-52** show non-limiting exemplary images of a primary band **1500** penile constriction device herein. **FIG. 47** shows a non-limiting exemplary image of a low tension primary band **1500A.** **FIG. 48** shows a non-limiting exemplary image of a high tension primary band **1500B. Per** **FIG. 49** the primary band **1500** comprises a hoop **1510,** a first tab **1520A,** and a second tab **1520B.** Per **FIG. 51****,** the secondary band **1600** an elongated hoop **1510,** the first tab **1520A,** and the second tab **1520B.** As shown, the first tab **1520A,** and the second tab **1520B** have equal tab lengths from their distal edges to their intersection with the hoop **1610.** Alternatively, in this or any other example, the tab length of the first tab **1520A** is greater than the tab length of the second tab **1520B.** Alternatively, in this or any other example, the tab length of the first tab **1520A** is less than the tab length of the second tab **1520B.**

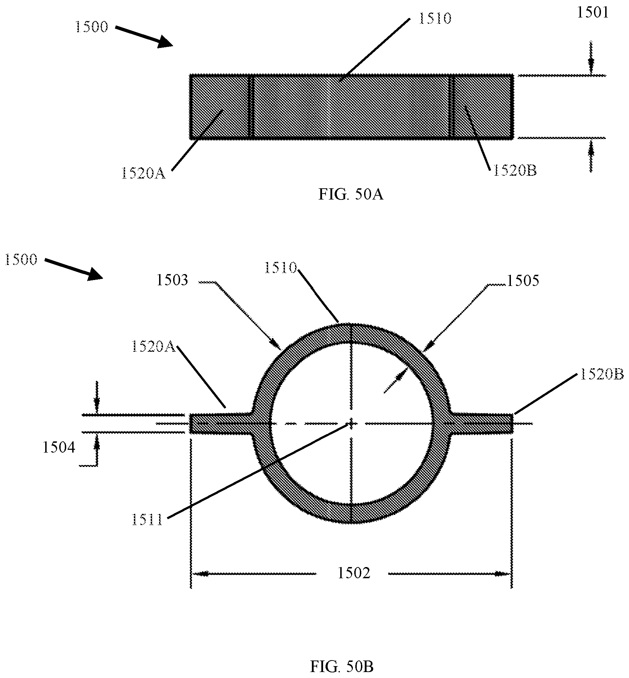

As shown per **FIG 50B****,** the tabs **1520A 1520B** extend perpendicularly from a center axis **1511** of the hoop **1510** from opposite sides of the hoop **1510.** Alternatively, in this or any other example, the primary band **1500** comprises 3, 4, 5, 6 or more tabs. Alternatively, in this or any other example, the tabs **1520A 1520B** extend parallel to a center axis **1511** of the hoop **1510** from opposite sides of the hoop **1510.** The tabs **1520A 1520B** provide a grip for a user to place the primary band **1500** on the device. Optionally, in this or any other example, an upper surface, a lower surface, or both of one or more of the first tab **1520A** and the second tab **1520B** has a raised or embossed texture for increased grip.

As shown, the primary band **1500** has a depth **1501** measured from a front surface to a back surface of the primary band **1500.** Optionally, in this or any other example, the depth **1501** is measured as a minimum depth, a maximum depth, or an average depth. As shown, the hoop **1510** and the tabs **1520A 1520B** have equal depths **1501.** Optionally, in this or any other example, a depth **1501** of the hoop **1510** is greater than a depth **1501** of the tabs **1520A 1520B.** Optionally, in this or any other example, a depth **1501** of the hoop **1510** is less than a depth **1501** of the tabs **1520A 1520B.**

Further per **FIG. 50B****,** the primary band **1500** has a width **1502** measured from a distal surface of the first tab **1520A** to a distal surface of the second tab **1520B.** Alternatively, in this or any other example, the width **1502** is measured as a maximum, an average, or a minimum distance from a distal surface of the first tab **1520A** to the distal surface of the second tab **1520B.** Alternatively, in this or any other example, the width **1502** is measured as a maximum, an average, or a minimum width of the primary band **1500.** Optionally, in this or any other example, the width **1502** is measured when the primary band **1500** is at rest and not applied to the device.

As shown, the hoop **1510** of the primary band **1500** has an outer diameter **1503.** Optionally, in this or any other example, the outer diameter **1503** is measured as a maximum, an average, or a minimum outer diameter of the hoop 1510. Optionally, in this or any other example, the outer diameter **1503** is measured as a maximum, an average, or a minimum outer diameter of a portion of the hoop **1510** not connected to the tabs **1520A 1520B.** Optionally, in this or any other example, the outer diameter **1503** is measured when the primary band **1500** is at rest and not applied to the device.

Also as shown, the tabs **1520A 1520B** have a tab thickness **1504** measured as a distance between opposing faces of each of the tabs **1520A 1520B.** Optionally, in this or any other example, the tab thickness **1504** is measured as a maximum, an average, or a minimum thickness of the tabs **1520A 1520B.** Optionally, in this or any other example, the first tab **1520A** and the second tab **1520B** have the same the tab thickness 1504. Optionally, in this or any other example, the first tab **1520A** has a greater tab thickness **1504** than the second tab **1520B.** Optionally, in this or any other example, the first tab **1520A** has a smaller tab thickness **1504** than the second tab **1520B.** Optionally, in this or any other example, the tab thickness **1504** is uniform from a distal edge to a proximal edge of the tabs **1520A 1520B.** Optionally, in this or any other example, the tab thickness **1504** is measured when the primary band **1500** is at rest and not applied to the device.

Further, per **FIG. 50B** the hoop **1510** has a hoop thickness **1505** measured as a normal distance between an outer surface and an inner surface of the hoop **1510.** Optionally, in this or any other example, the hoop thickness **1505** is measured as a maximum, an average, or a minimum distance. Optionally, in this or any other example, the hoop thickness **1505** is equal across the circumference of the hoop **1510.** Optionally, in this or any other example, the hoop thickness **1505** varies across the circumference of the hoop **1510.** Optionally, in this or any other example, the hoop thickness **1505** is measured when the primary band **1500** is at rest and not applied to the device.

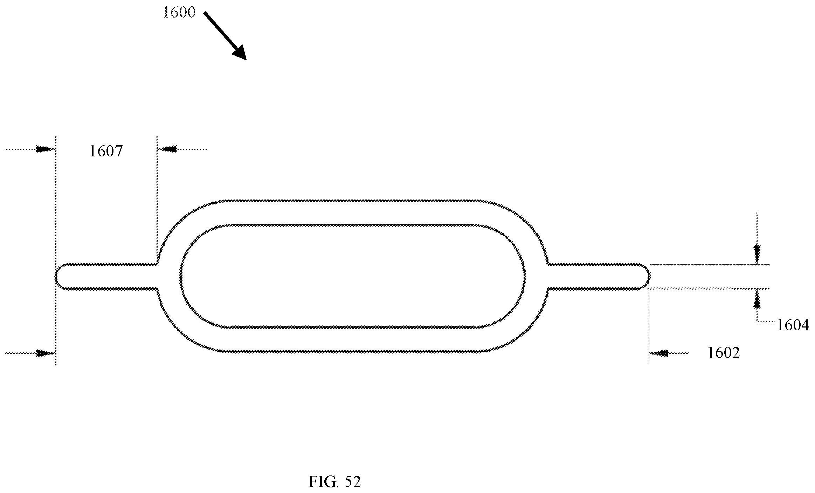

**FIGS. 51** and **52** show exemplary illustrations of a secondary band **1600.** As shown, the secondary band **1600** comprises an elongated hoop **1610,** a first secondary tab **1620A,** a second secondary tab **1620B.** Further as shown, the first secondary tab **1620A** and the second secondary tab **1620B** extend outwards from opposite sides of the elongated hoop **1610.** Per **FIG. 52****,** the secondary band **1600** has an elongated width **1602.** Optionally, in this or any other example, the elongated width **1602** is measured from a distal surface of the first secondary tab **1620A** to a distal surface of the second secondary tab **1620B.** Alternatively, in this or any other example, the width **1602** is measured as a maximum, an average, or a minimum distance from a distal surface of the first secondary tab **1620A** to the distal surface of the second secondary tab **1620B.** Alternatively, in this or any other example, the elongated width **1602** is measured as a maximum, an average, or a minimum width of the secondary band **1600.** Optionally, in this or any other example, the width **1602** is measured when the secondary band **1600** is at rest and not applied to the device. Further as shown, the secondary tabs **1620A 1620B** have a tab thickness **1604** measured as a distance between opposing faces of each of the secondary tabs **1620A 1620B.** Optionally, in this or any other example, the tab thickness **1604** is measured as a maximum, an average, or a minimum thickness of the secondary tabs **1620A 1620B.** Optionally, in this or any other example, the first secondary tab **1620A** and the second secondary tab **1620B** have the same the tab thickness **1604.** Optionally, in this or any other example, the first secondary tab **1620A** has a greater tab thickness **1604** than the second secondary tab **1620B.** Optionally, in this or any other example, the first secondary tab **1620A** has a smaller tab thickness **1604** than the second secondary tab **1620B.** Optionally, in this or any other example, the tab thickness **1604** is uniform from a distal edge to a proximal edge of the secondary tabs **1620A 1620B.** Optionally, in this or any other example, the tab thickness **1604** is measured when the secondary band **1600** is at rest and not applied to the device. Also as shown, the first secondary tab **1620A,** and the second secondary tab **1620B** have equal tab lengths **1607** from their distal edges to their intersection with the hoop **1610.** Alternatively, in this or any other example, the tab length **1607** of the first secondary tab **1620A** is greater than the tab length **1607** of the second secondary tab **1620B.** Alternatively, in this or any other example, the tab length **1607** of the first secondary tab **1620A** is less than the tab length **1607** of the second secondary tab **1620B.** Optionally, in this or any other example, the elongated width **1602** is about 1 inch to about 2 inches. Optionally, in this or any other example, the tab thickness **1604** is about 0.04 inches to about 0.08 inches. Optionally, in this or any other example, the tab length **1607** is about 0.2 inches to about 0.3 inches.

Optionally, in this or any other example, the primary band **1500** comprises a high tension primary band **1500A**. Optionally, in this or any other example, the primary band **1500** comprises a low tension primary band **1500B.**

Optionally, in this or any other example, the high tension primary band **1500A** has one or more of a depth **1501** of about 0.1 inches to about 0.4 inches, a width **1502** of about 0.7 inches to about 1.0 inches, an outer diameter **1503** of about 0.2 inches to about 0.6 inches, a tab thickness **1504** of about 0.05 inches to about 0.1 inches, and a hoop thickness **1505** of about 0.05 inches to about 0.1 inches. Optionally, in this or any other example, the high tension primary band **1500A** has one or more of a depth **1501** of at least about 0.1 inches, a width **1502** of at least about 0.7 inches, an outer diameter **1503** of at least about 0.2 inches, a tab thickness **1504** of at least about 0.05 inches, and a hoop thickness **1505** of at least about 0.05 inches. Optionally, in this or any other example, the high tension primary band **1500A** has a depth **1501** of at most about 0.4 inches, a width **1502** of at most about 1.0 inches, an outer diameter **1503** of at most about 0.6 inches, a tab thickness **1504** of at most about 0.1 inches, and a hoop thickness **1505** of at about 0.1 inches.

Optionally, in this or any other example, the low tension primary band **1500B** has a depth **1501** of about 0.1 inches to about 0.4 inches, a width **1502** of about 0.8 inches to about 1.4 inches, an outer diameter **1503** of about 0.4 inches to about 1.5 inches, a tab thickness **1504** of about 0.05 inches to about 0.1 inches, and a hoop thickness **1505** of about 0.05 inches to about 0.1 inches. Optionally, in this or any other example, the low tension primary band **1500B** has a depth **1501** of at least about 0.1 inches, a width **1502** of at least about 0.8 inches, an outer diameter **1503** of at least about 0.4 inches, a tab thickness **1504** of at least about 0.05 inches, and a hoop thickness **1505** of at least about 0.05 inches. Optionally, in this or any other example, the low tension primary band **1500B** has a depth **1501** of at most about 0.4 inches, a width **1502** of at most about 1.4 inches, an outer diameter **1503** of at most about 1.5 inches, a tab thickness **1504** of at most about 0.05 inches to about 0.1 inches, and a hoop thickness **1505** of at most about 0.1 inches.

### Methods For Using the Penile Constriction Devices Herein

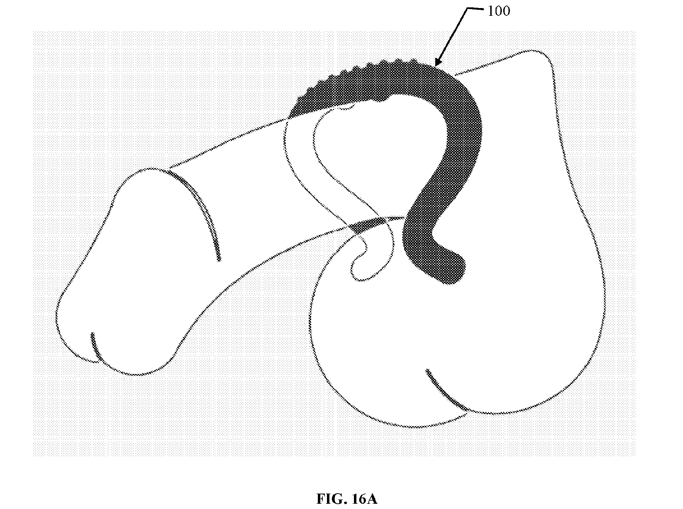

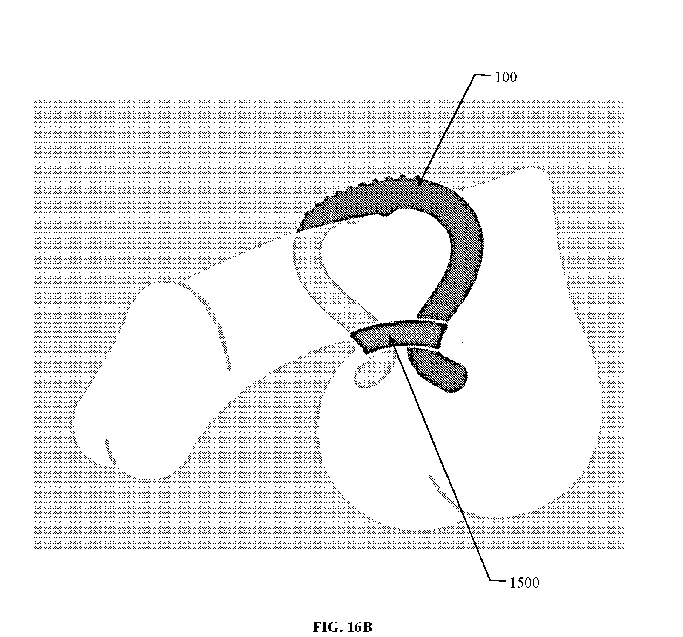

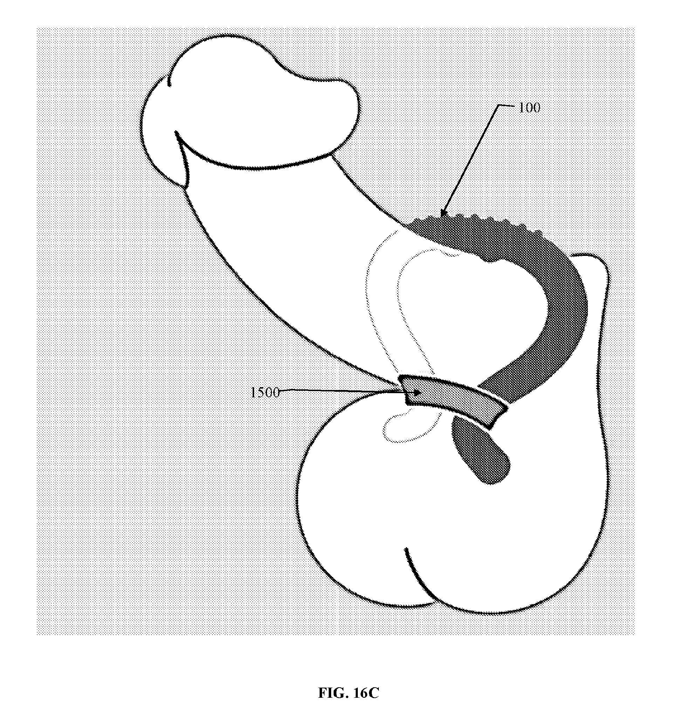

Further provided herein, per **FIGS. 16A-C****,** are methods for using a penile constriction device for alleviating erectile dysfunction (ED). **FIG. 16A** shows a perspective view a non-limiting example of a penile constriction device on a flaccid human penis. **FIG. 16B** shows a perspective view a non-limiting example of a penile constriction device and a band on a flaccid human penis. **FIG. 16C** shows a perspective view a non-limiting example of a penile constriction device and a band on an erect human penis. Optionally, in this or any other example, the penile constriction device comprises an arched rod. Optionally, in this or any other example, the arched rod comprises a first arm, a second arm, and a bridge. Optionally, in this or any other example, the first arm comprises at least one of a first coupling end and a first converging end having a first terminus. Optionally, in this or any other example, the second arm comprises at least one of a second coupling end and a second converging end having a second terminus. Optionally, in this or any other example, the second terminus is disconnected from the first terminus. Optionally, in this or any other example, the first converging end and the second converging end converge to form a constriction region. Optionally, in this or any other example, the bridge connects the first coupling end to the second coupling end. Optionally, in this or any other example, the bridge comprises a plurality of friction protrusions on a superior side of the bridge. Optionally, in this or any other example, the bridge comprises a compression region rising inferiorly from an inferior side of the bridge.

Optionally, in this or any other example, the method comprises applying a force between the first terminus and the second terminus of the device 100 to increase a constriction gap between the first converging end and the second converging end, and passing the penis through the constriction gap. Optionally, in this or any other example, the method comprises applying a force between the first coupling end and the second coupling end to increase the constriction gap.

Optionally, in this or any other example, per **FIG. 16A****,** the method further comprises passing the penis through the constriction gap of the device **100.** Optionally, in this or any other example, the method further comprises moving the device **100** to the proximal base of the penis. Optionally, in this or any other example, the method further comprises aligning the device such that at least one of the friction protrusions and the compression region are oriented on the penis opposite the testis. Optionally, in this or any other example, the penis is flaccid when passed through the constriction gap, when passed in between the legs/ends of the device, when positioned underneath the dorsal apex of the device **100.** Optionally, in this or any other example, the penis is flaccid when the device **100** is moved to the proximal base of the penis. Optionally, in this or any other example, the penis is erect when passed through the constriction gap of the device **100.** Optionally, in this or any other example, the penis is erect when the device **100** is moved to the proximal base of the penis. Optionally, in this or any other example, the method does not comprise inflating the device, pressurizing the device, or both.

Another aspect provided herein is a method of treating an ailment in a patient, the method comprising: wearing the penile constriction device to promote intercourse; maintaining an erection; and engaging in sexual intercourse, masturbation, or both with the worn penile constriction device, wherein engaging in sexual intercourse, masturbation, or both with the worn penile constriction device sexual intercourse, masturbation, or both treats the ailment by facilitating ejaculation and/or orgasm, releasing endorphins, oxytocin, serotonin, and dopamine, and reducing cortisol levels, facilitating sexual activity, or any combination thereof. In some examples, the ailment comprises anxiety, depression, post-traumatic stress disorder, low self-esteem, seasonal affective disorder (SAD), low testosterone, stress, ED, prostate cancer, hypertension, coronary artery disease, diabetes, heart disease, high blood pressure, low blood pressure, coronary artery disease (CAD), COPD, hypertension, headache, migraines, obesity, or any combination thereof. In some examples, the methods, devices, and systems herein enable improved family planning and/or sperm donation.

Yet another aspect provided herein is a method of reducing the required intake of a medication, the method comprising: wearing the penile constriction device to promote intercourse; maintaining an erection; and engaging in sexual intercourse, masturbation, or both with the worn penile constriction device, wherein engaging in sexual intercourse, masturbation, or both with the worn penile constriction device reduces the required intake of a medication by facilitating ejaculation and/or orgasm, releasing endorphins, oxytocin, serotonin, and dopamine, and reducing cortisol levels, facilitating sexual activity, or any combination thereof. In some examples, the medication comprises an anti-anxiety medication, an anti-depression medication, a pain medication, an opioid, a heart medication, a blood-pressure medication, a cholesterol medication, or any combination thereof.

Optionally, in this or any other example, per **FIGS. 16B-C****,** the method further comprises installing a band **1500** on the device **100.** Optionally, in this or any other example, installing a band **1500** on the device comprises installing the band **1500** between the first elbow and the second elbow of the device **100.** Optionally, in this or any other example, installing a band **1500** on the device comprises installing the band **1500** over the first terminus and over the second terminus of the device **100.** Optionally, in this or any other example, installing a band **1500** on the device comprises installing the band **1500** within the constriction region of the device **100.**

In some examples, wearing the device **100** comprises placing the device **100** around a vacuum pump, using the vacuum pump to achieve an erection, and sliding the device **100** off the pump and onto the penis, wherein trapped blood maintains the erection. In some examples, the method further comprises employing an effective amount of an ED medication. In some examples, the method further comprises dispensing a lubricant onto the penis, the device **100,** or both. In some examples, method further comprises shaving/trimming of one's pubic hair, to minimize the risk of pulling, yanking, or uncomfortable friction between the device **100** and pubic hair. In some examples, the method further comprises placing the device at the base of the shaft of the flaccid or erect penis, and applying a condom once an erection is achieved.

### Methods For Selecting a Penile Constriction Device

Additionally, provided herein, is a method for selecting a penile constriction device.

**Table 1**

| **General Penis Size I** | | **Girth** | | **Circular Diameter** | | **Cross-Sectional Area** | |
|---|---|---|---|---|---|---|---|
| | | Flaccid I | Erect I | Flaccid | Erect | Flaccid | Erect |
| **A** | Min | 2.990 | 3.750 | 0.952 | 1.194 | 0.712 | 1.119 |
| | Max | 3.373 | 4.230 | 1.074 | 1.346 | 0.905 | 1.424 |
| **B** | Min | 3.373 | 4.230 | 1.074 | 1.346 | 0.905 | 1.424 |
| | Max | 3.692 | 4.630 | 1.175 | 1.474 | 1.085 | 1.706 |
| **C** | Min | 3.692 | 4.630 | 1.175 | 1.474 | 1.085 | 1.706 |
| | Max | 4.003 | 5.020 | 1.274 | 1.598 | 1.275 | 2.005 |
| **D** | Min | 4.003 | 5.020 | 1.274 | 1.598 | 1.275 | 2.005 |
| | Max | 4.633 | 5.810 | 1.475 | 1.849 | 1.708 | 2.686 |

To ensure proper fit for a wide range of human anatomies, devices herein can be configured with an array of dimensions and shapes. Table 1, above, shows that penile anatomical dimensions can be categorized into four groups to allow for easy selection of the proper sized device by a user. As the devices provided herein are flexible and configured to impart a pressure force against a portion of the erect or flaccid penis, the recommended size and shape for a particular user correlates to the size of their particular anatomy, as well as the severity of their erectile dysfunction conditions.

Optionally, in this or any other embodiment, the method comprises at least one of a user and a medical practitioner measuring at least one of a circular diameter and a girth of a user's erect or flaccid penis, and selecting a penile constriction device associated with at least one of the circular diameter and the girth of a user's penis according to the information provided in Table 1. Optionally, in this or any other embodiment, the method comprises the user estimating or measuring the size of their erect or flaccid penis as belonging to two or more general sizes, and selecting a penile constriction device associated with the general size. Optionally, in this or any other embodiment, the method further comprises the user trying the selected penile construction device to confirm a proper fit and comfortability. Optionally, in this or any other embodiment, the method further comprises the user trying the selected penile construction device and the band to confirm a proper fit and comfortability. Optionally, in this or any other embodiment, trying the selected penile construction device to confirm a proper fit and comfortability comprises confirming that the penile construction device does not freely rotate about at least one of the user's flaccid penis, and the user's erect penis. Optionally, in this or any other embodiment, trying the selected penile constriction device to confirm a proper fit and comfortability comprises performing vaginal intercourse, anal intercourse, oral sex, or masturbation with the device installed.

### Methods for Selecting a Penile Constriction Band

Additionally, provided herein, is a method for selecting a band for a penile constriction device. Optionally, in this or any other example, the method comprises selecting a low tension band or a high tension band. Optionally, in this or any other example, the method comprises trying on the device with the low tension band and replacing the low tension band with the high tension band if the device significantly shifts during intercourse with the low tension band is employed. Optionally, in this or any other example, the method comprises trying on the device with the high tension band and replacing the high tension band with the low tension band if the device is uncomfortable with the high tension band employed. Optionally, in this or any other example, the method further comprises selecting a size of the low tension band or the high tension band based on the device size.

Another aspect provided herein according to the invention is a method of selecting a penile constriction device for an individual, the method comprising: providing a measurement device having color coded demarcations, each demarcation indicating a size of the erectile device; measuring the individual's penis using the measurement device; and selecting the penile constrictive device based on the demarcation. Also provided herein according to the invention is a measurement device for sizing a penile constriction device comprising color-coded demarcations, each demarcation indicating a size of the penile constriction device.

### Methods For Using the Penile Constriction Band Herein

Further provided herein, are methods for using the bands herein. In one example, the method comprises a user pinching the first tab between two of their fingers of a first hand, pinching the second tab between two of their fingers of a second hand, and inserting the first converging end and the second converging end of the device herein into the hoop of the band, such that the band rests between the first elbow and the second elbow of the device. In one example, the method comprises a user pinching the first tab between two of their fingers of a first hand, pinching the second tab between two of their fingers of a second hand, inserting the first converging end of the device herein into the hoop of the band, and inserting the second converging end of the device herein into the hoop of the band, such that the band rests between the first elbow and the second elbow of the device. Optionally, in this or any other example, the two fingers comprise a thumb and a pointer finger. Optionally, in this or any other example, the band is installed onto the device while the user wears the device. Optionally, in this or any other example, the band is installed onto the device while the user is not wearing the device.

### Kits Comprising a Penile Constriction Device

Finally, provided herein is a kit comprising a penile constriction device and instructions for using the device to alleviate erectile dysfunction (ED). Optionally, in this or any other example, the penile constriction device comprises an arched rod comprising: a first arm comprising a first coupling end and a first converging end having a first terminus; a second arm comprising a second coupling end, and a second converging end having a second terminus disconnected from the first terminus, wherein the first converging end and the second converging end converge to form a constriction region; and a bridge connecting the first coupling end to the second coupling end, the bridge comprising a plurality of friction protrusions on a superior side of the bridge, and a compression region rising inferiorly from an inferior side of the bridge.

Optionally, in this or any other example, the kit further comprises at least one of: a box, a low tension band, a high tension band, printed Instructions for Use, and "Date Night" content series. Optionally, in this or any other example, the band is configured to attach to the first coupling end and the second coupling end to provide increased penile compressive force. Optionally, in this or any other example, the box comprises a label. Optionally, in this or any other example, the container comprises a plurality of segments. Optionally, in this or any other example, the measuring tool comprises at least one of a ruler, a tape, a jig, and a caliper. Optionally, in this or any other example, the cleaning element comprises an alcohol pad, a wet pad, alcohol, or any combination thereof. In some examples, the kit provided herein does not comprise a pump. In some examples, the penile constriction device is not connected to a pump.

In one example a 1-Pack kit is assembled by:
**1)** Placing 1 device into three separate inner boxes
**2)** Placing 3 high tension bands and 3 low tension bands in a plastic polyfold bag, and place the bag into an inner box, and repeat two more times, so that there are 3 boxes with 6 bands each
**3)** Placing 2 inner boxes along with 1 Filler Box into the Main Box, creating a snug square of inner boxes inside the bottom layer of the Main Box
**4)** Placing 2 inner boxes with the devices into the Main Box, creating another snug square of inner boxes on top of the bottom tow inner boxes and 1 filler Box
**5)** Placing 2 inner boxes with bands into the Main Box, creating another snug square of inner boxes on top of the bottom tow inner boxes and 1 filler Box
**6)** Placing a printed instruction booklet on top of inner boxes
**7)** Closing a flap and slide Outer Sleeve over main box
**8)** Placing a UPC sticker on the text that states "PLACE UPC HERE"
**9)** Placing a UDI sticker on the text that states "PLACE UDI HERE"
**10)** Putting a shrink-wrap around entire closed box container and sleeve

In one example a 2-Pack kit is assembled by:
**1)** Placing 2 devices into three separate inner boxes
**2)** Placing 3 high tension bands and 3 low tension bands in a plastic polyfold bag, and place the bag into an inner box, and repeat two more times, so that there are 3 boxes with 6 bands each
**3)** Placing 2 inner boxes along with 1 Filler Box into the Main Box, creating a snug square of inner boxes inside the bottom layer of the Main Box
**4)** Placing 2 inner boxes with the devices into the Main Box, creating another snug square of inner boxes on top of the bottom tow inner boxes and 1 filler Box
**5)** Placing 2 inner boxes with bands into the Main Box, creating another snug square of inner boxes on top of the bottom tow inner boxes and 1 filler Box
**6)** Placing a printed instruction booklet on top of inner boxes
**7)** Closing a flap and slide Outer Sleeve over main box
**8)** Placing a UPC sticker on the text that states "PLACE UPC HERE"
**9)** Placing a UDI sticker on the text that states "PLACE UDI HERE"
**10)** Putting a shrink-wrap around entire closed box container and sleeve

In one example a 3-Pack kit is assembled by:
**1)** Placing 3 devices into three separate inner boxes
**2)** Placing 3 high tension bands and 3 low tension bands in a plastic polyfold bag, and place the bag into an inner box, and repeat two more times, so that there are 3 boxes with 6 bands each
**3)** Placing 2 inner boxes along with 1 Filler Box into the Main Box, creating a snug square of inner boxes inside the bottom layer of the Main Box
**4)** Placing 2 inner boxes with the devices into the Main Box, creating another snug square of inner boxes on top of the bottom tow inner boxes and 1 filler Box
**5)** Placing 2 inner boxes with bands into the Main Box, creating another snug square of inner boxes on top of the bottom tow inner boxes and 1 filler Box
**6)** Placing a printed instruction booklet on top of inner boxes
**7)** Closing a flap and slide Outer Sleeve over main box
**8)** Placing a UPC sticker on the text that states "PLACE UPC HERE"
**9)** Placing a UDI sticker on the text that states "PLACE UDI HERE"
**10)** Putting a shrink-wrap around entire closed box container and sleeve

In one example a 4-Pack kit is assembled by:
**1)** Placing 4 devices into four separate inner boxes
**2)** Placing 3 high tension bands and 3 low tension bands in a plastic polyfold bag, and place the bag into an inner box, repeat three more times, so that there are 4 boxes with 6 bands each
**3)** Placing 4 inner boxes with the devices into the Main Box, creating a snug square of boxes along the bottom layer of the Main Box
**4)** Placing 4 inner boxes with bands on top of the 4 devices, creating another snug square of inner boxes on top of the bottom four inner boxes
**5)** Placing a printed instruction booklet on top of inner boxes
**6)** Closing flap and slide Outer Sleeve over main box
**7)** Placing an UPC sticker on the text that states "PLACE UPC HERE"
**8)** Placing an UDI sticker on the text that states "PLACE UDI HERE"
**9)** Putting a shrink-wrap around entire closed box container and sleeve

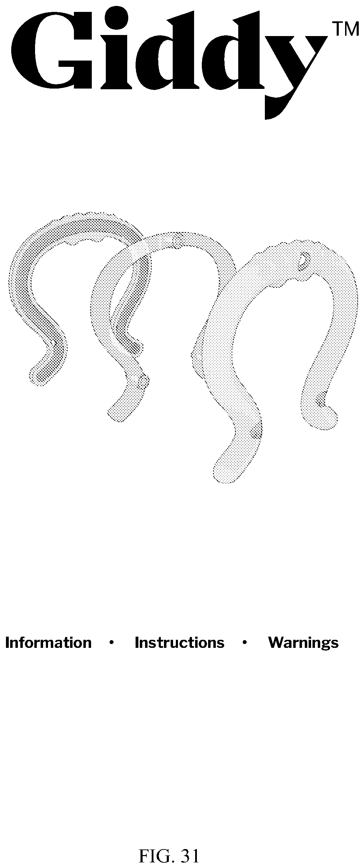

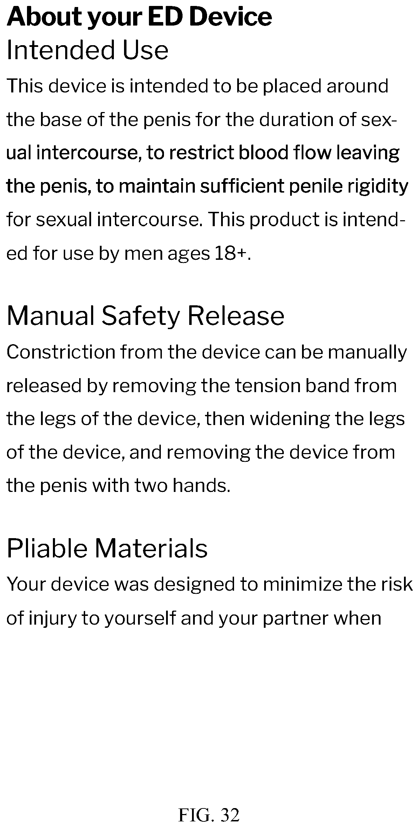

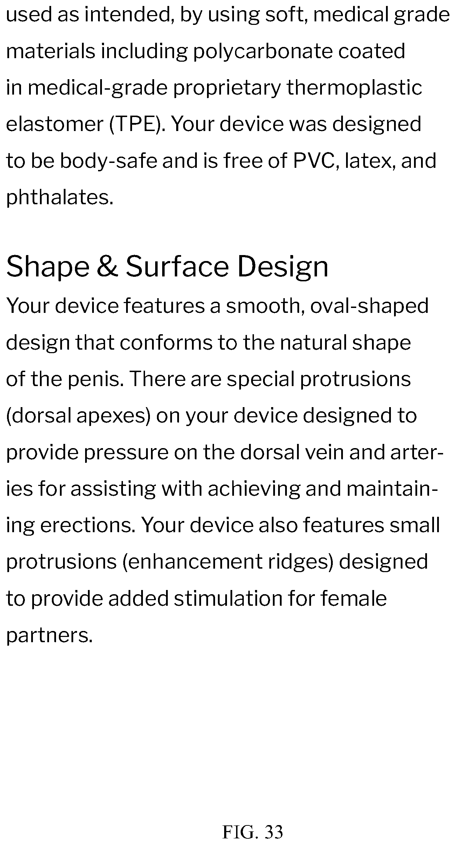

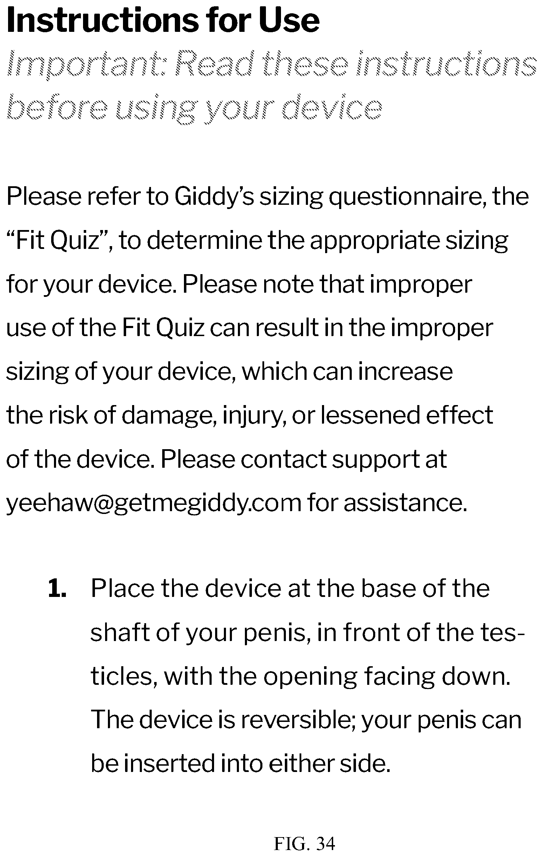

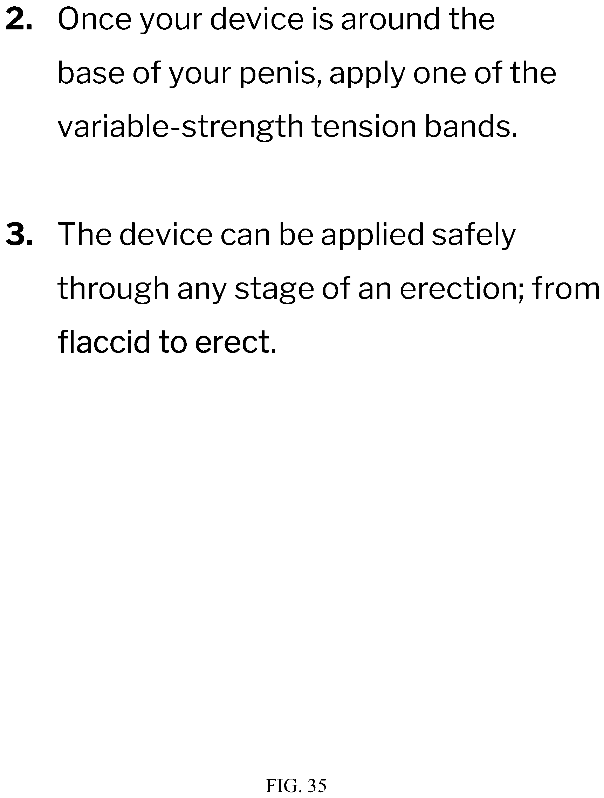

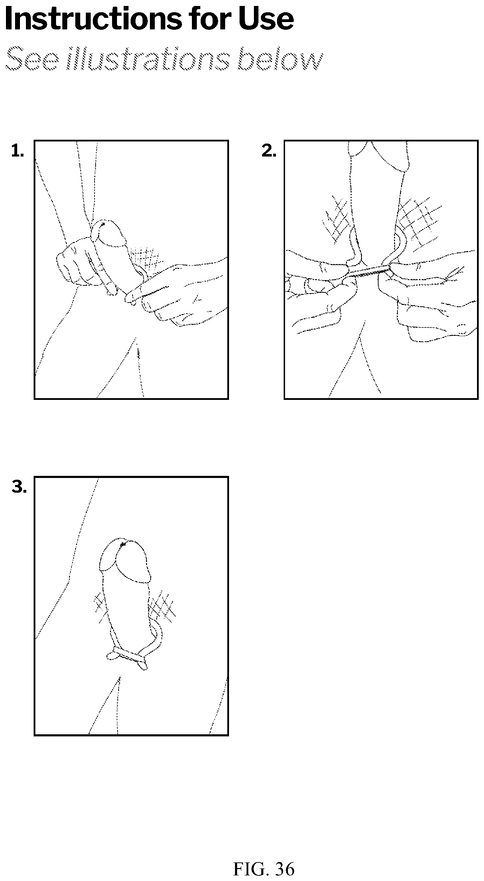

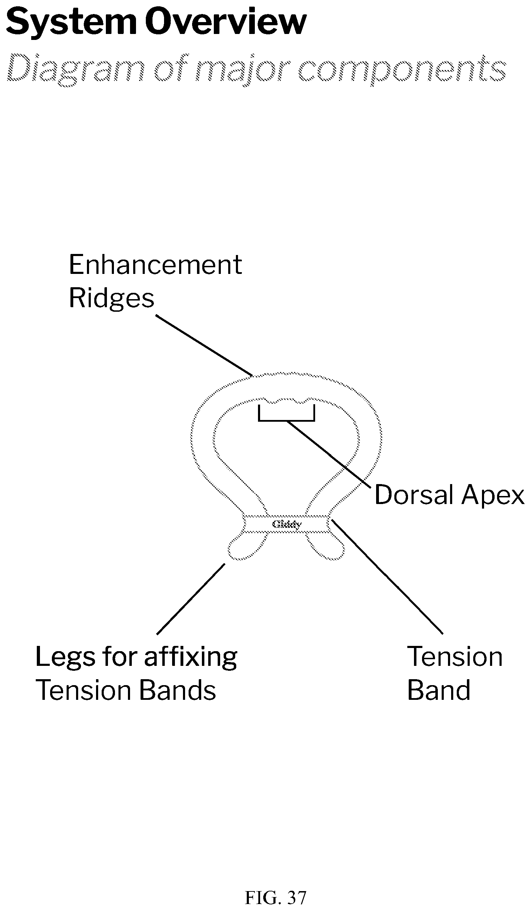

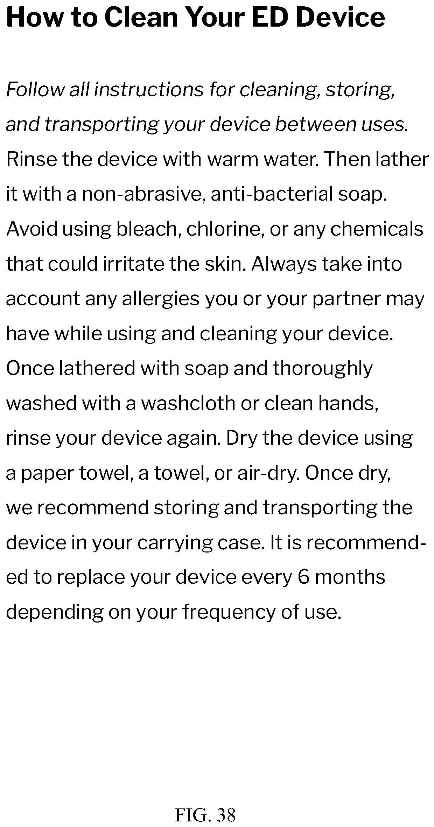

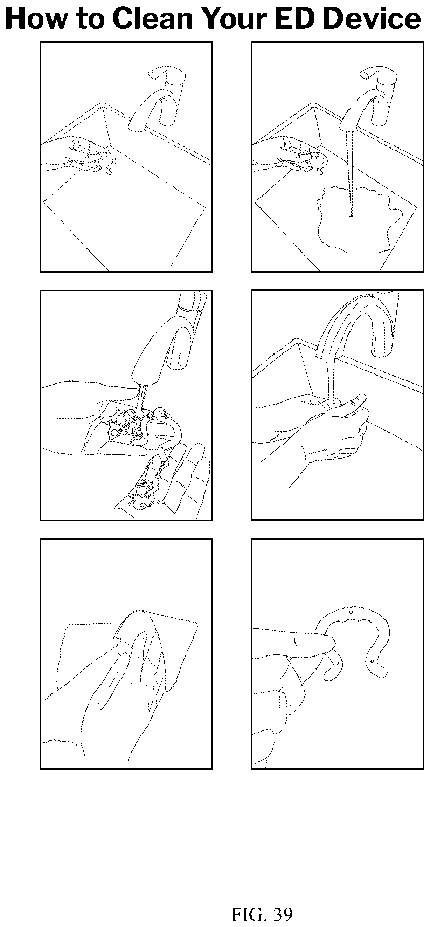

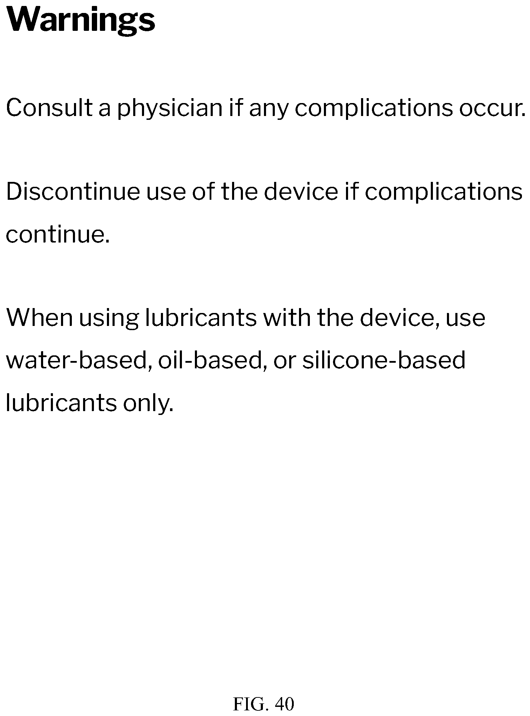

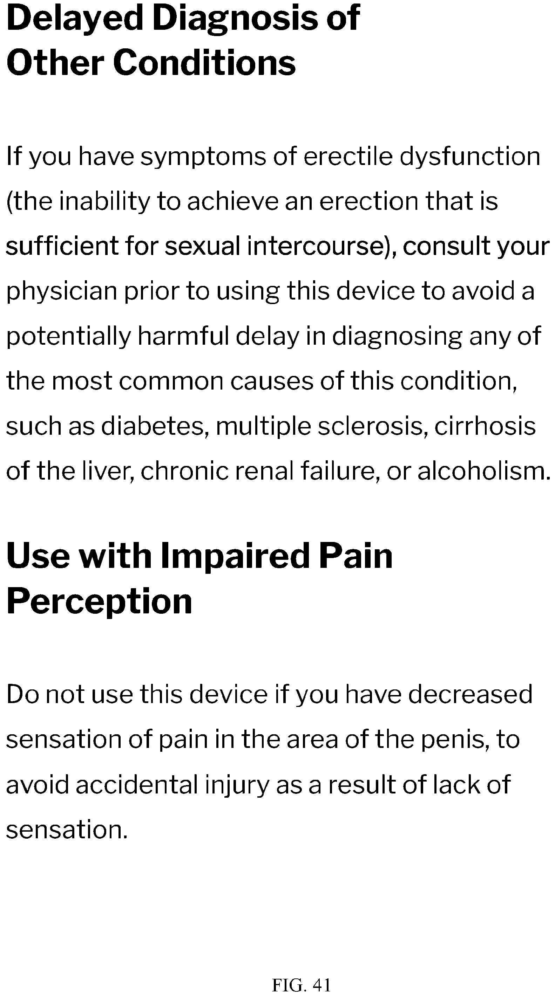

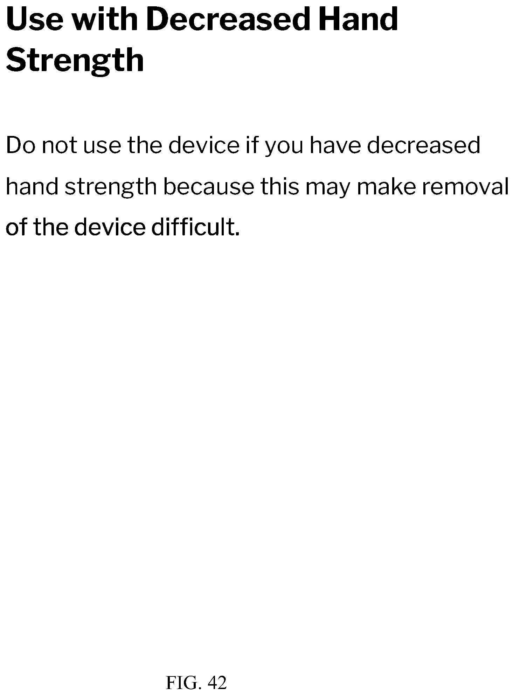

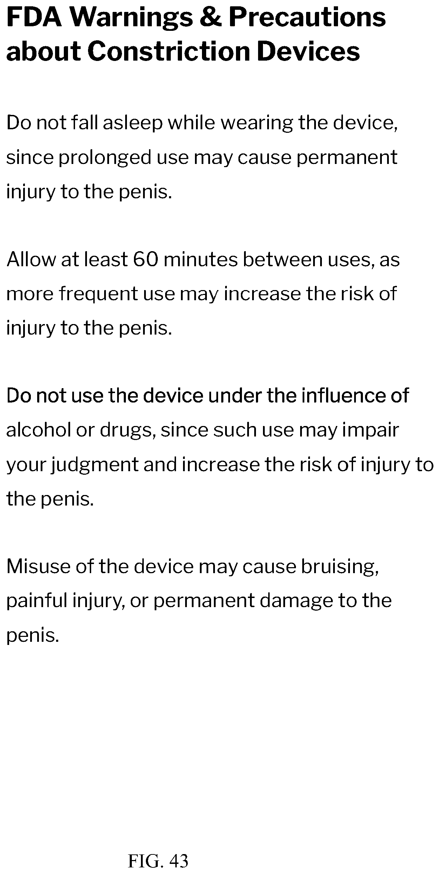

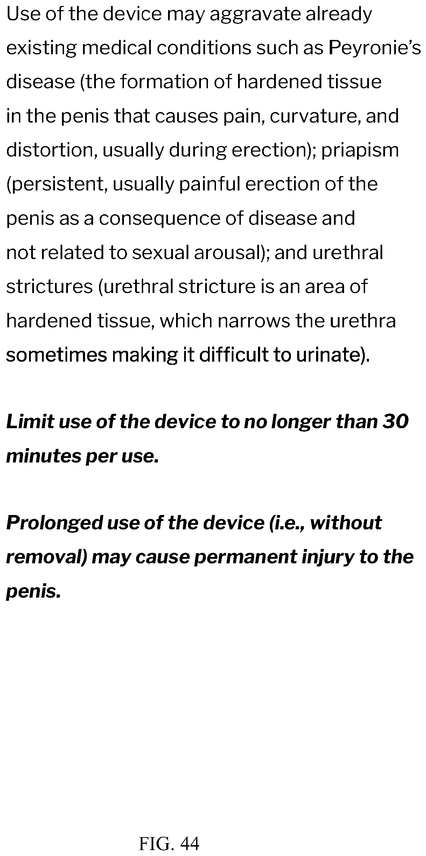

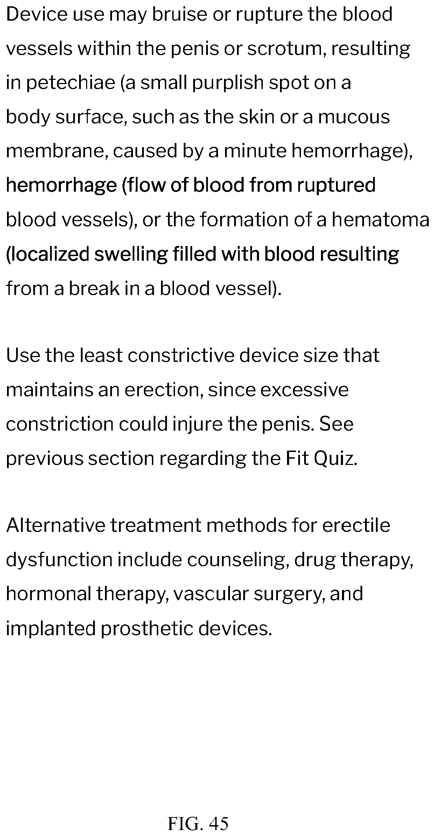

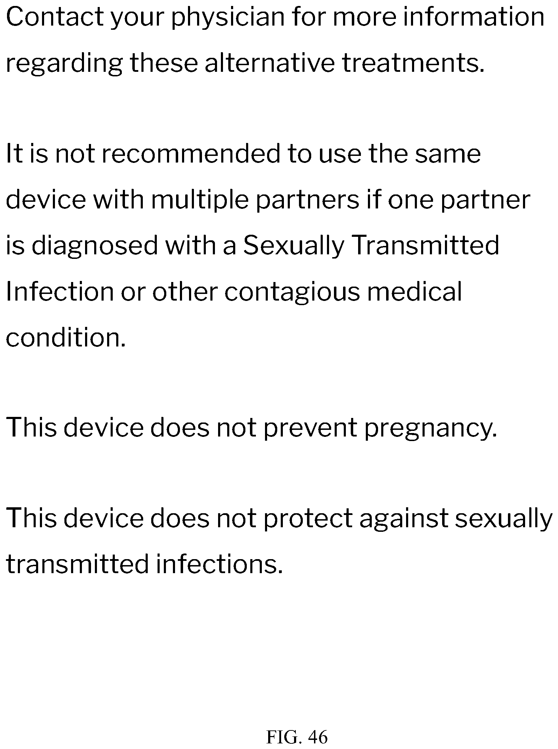

In some examples, the instruction booklet, per **FIGS. 31-46** contains the following instructions:

### About Giddy's ED Device

### Intended Use

This device is intended to maintain penile rigidity in men with erectile dysfunction (ED). The device is intended to be placed around the base of the penis to assist with maintaining an erection for the duration of sexual intercourse, masturbation, or both or masturbation, to restrict venous blood flow leaving the penis, to maintain sufficient penile rigidity for sexual to reach the point of ejaculation during intercourse or masturbation. This product is designed to be a natural and non-prescription alternative to ED pills, vacuum pumps, circular constriction rings, surgical implants, injections, and other ED treatments. This product is intended for use by men ages 18+.

### Manual Safety Release

Constriction from the device can be manually released by removing the tension band from the legs/ends of the device, then widening the legs/ends of the device, and removing the device from the penis with two hands. It is recommended not to pull apart the ends of the device too far, to prevent damage or breakage.

### Pliable Materials

The device was designed to minimize the risk of injury to the user and their partner when used as intended, by using soft, medical grade materials including polycarbonate coated in medical-grade proprietary thermoplastic elastomer (TPE). The device was designed to be body-safe and is free of Polyvinyl Chloride (PVC), latex, and phthalates.

### Shape & Surface Design

Your device features a smooth, oval-shaped design that conforms to the natural shape of the penis. Your device is designed to apply optimal pressure to the veins, while leaving the arteries and urethra unencumbered, to assist with maintaining erections and natural-feeling ejaculation. Traditional circular-shaped constriction rings squeeze the urethra, causing blocked, uncomfortable, painful ejaculation. By blocking the urethra, circular rings can even cause retrograde or "backwards" ejaculation, where semen enters the bladder instead of exiting the penis. The curvatures and open-bottom design of Your device leaves the urethra unrestricted, so during orgasm, ejaculation is unrestricted, feeling more natural, and "free-flowing."

### Instructions for Use

Important: Read these instructions before using your device
Please refer to Giddy's sizing process (available at EddiebyGiddy.com/sizing), to determine the appropriate sizing for your device. Please note that improper use of the Size process can result in the improper sizing of your device, which can increase the risk of damage, injury, or lessened effect of the device. Thoroughly clean your device after opening it from the original sealed packaging. For optimal cleanliness, clean your device before and after each use Please contact support at customercare@eddiebygiddy.com for assistance.
1. Wash your device thoroughly. Place the device at the base of the shaft of your penis, in front of the testicles, with the opening facing down. The device is reversible; your penis can be inserted into either side.
2. Once your device is around the base of your penis, apply one of the variable-strength tension bands.
3. The device can be applied safely whether your penis is flaccid (soft) or erect (hard). When an erection can be achieved but not maintained, the device can be used alone, or when creating an erection is not possible, it can be used in conjunction with vacuum pumps

### Tension Bands

There are two types of tension bands. Use the blue "comfort" band to achieve a moderate amount of constriction. Use the red "sport" band to achieve maximum constriction. Use whichever band feels most comfortable while achieving the desired result. The tension bands provide an added level of customization from the device depending on what the user needs/wants in the moment. If ED symptoms are more severe than usual, a user can change to a smaller band. Likewise, if ED symptoms are less severe than usual, a user can change to the larger band. The different tension bands are also a way to determine a user's ideal size "in between sizes." For example: Bob may see a better result from using a Size B with a red/sport tension band than he does from a Size C with a blue/comfort tension band.

### How to Clean the Device

Follow all instructions for cleaning, storing, and transporting the device and Tension Bands between uses. Rinse the device and any used Tension Bands with warm water. Then lather them with a non-abrasive, anti-bacterial soap. Avoid using bleach, chlorine, or any chemicals that could irritate the skin. Always take into account any allergies you or your partner may have while using and cleaning your device and Tension Bands. Once lathered with soap and thoroughly washed with a washcloth or clean hands, rinse your device and Tension Bands again. Dry them using a paper towel, a towel, or air-dry. It is recommended to replace your device and tension bands every 4 months depending on your frequency of use.

### Warnings

Consult a physician if any complications occur for you or your partner.

Discontinue use of the device if complications continue for you or your partner.

When using lubricants with the device, use water-based, oil-based, or silicone-based lubricants only.

Do not fall asleep while wearing the device, since prolonged use may cause permanent injury to the penis.

Allow at least 60 minutes between uses, as more frequent use may increase the risk of injury to the penis.

It is not recommended to use the same device with multiple partners if one partner is diagnosed with a sexually transmitted infection or other contagious medical conditions.

This device does not prevent pregnancy.

This device does not protect against sexually transmitted infections.

Do not use the device under the influence of alcohol or drugs, since such use may impair your judgment and increase the risk of injury to the penis.

Misuse of the device may cause bruising, painful injury, or permanent damage to the penis.

### Delayed Diagnosis of Other Conditions

If you have symptoms of erectile dysfunction (the inability to achieve an erection that is sufficient for sexual intercourse, masturbation, or both), consult your physician prior to using this device to avoid a potentially harmful delay in diagnosing any of the most common causes of this condition, such as diabetes, multiple sclerosis, cirrhosis of the liver, chronic renal failure, or alcoholism.

### Use with Impaired Pain Perception

Do not use this device if you have decreased sensation of pain in the area of the penis, to avoid accidental injury as a result of lack of sensation.

### Use with Decreased Hand Strength

Do not use the device if you have decreased hand strength because this may make removal of the device difficult. An alternative method for applying the device for users with reduced hand strength is to wrap the Tension Band around the legs/ends of the device before placing it on your penis. Using two hands, pull apart the legs/ends of the device, holding them apart slightly to create a wider opening, while sliding the device onto your penis. To avoid damaging the device, use caution not to pull apart the legs/ends too far.

For a second alternative method, cover the device in lubricant, and slide it onto your penis until it reaches the base. Feel free to use any water-based, oil-based, or silicone-based lubricants. If you experience any pain or bruising, stop and consult your physician before continuing use.

### Precautions

Use of the device may aggravate already existing medical conditions such as Peyronie's disease (the formation of hardened tissue in the penis that causes pain, curvature, and distortion, usually during erection); priapism (persistent, usually painful erection of the penis as a consequence of disease and not related to sexual arousal); and urethral strictures (urethral stricture is an area of hardened tissue, which narrows the urethra sometimes making it difficult to urinate).

Limit use of the device to no longer than 30 minutes per use.

Prolonged use of the device (i.e., without removal) may cause permanent injury to the penis.

Device use may bruise or rupture the blood vessels within the penis or scrotum, resulting in petechiae (a small purplish spot on a body surface, such as the skin or a mucous membrane, caused by a minute hemorrhage), hemorrhage (flow of blood from ruptured blood vessels), or the formation of a hematoma (localized swelling filled with blood resulting from a break in a blood vessel).

Use the least constrictive device size that maintains an erection, since excessive constriction could injure the penis. See previous information regarding the Size instructions.

And in some examples, the following exemplary troubleshooting guide accompanying an erectile dysfunction device can be provided:

| | | | | | |
|---|---|---|---|---|---|
| **Problem** | Discomfort/Pain while putting on or wearing your device | Discomfort/Pain during intercourse | Discomfort/Pain during ejaculation | Discomfort/Pain around testicles or scrotum | No erection |
| **Likely Cause** | Lack of familiarity with your device and/or tension band; device incorrectly sized | Inadequate lubrication; pressure on a sensitive area, excessively deep penetration; device being worn incorrectly; lack of familiarity with device and/or tension bands; device incorrectly sized | Long period of abstinence; infection or inflammation of the prostate gland; device and/or tension bands too tight or being worn incorrectly | Lack of familiarity with your device and/or tension band; device incorrectly sized | Existing medical condition |
| **Remedy** | More practice using your device and tension bands; using a smaller or larger device size; continue to use device | More lubrication; change sexual position; less penetration; more practice using your device and tension bands; using a smaller or larger sized device; continue to use your device | Try a larger device or weaker tension band | More practice using your device and tension bands; using a smaller or larger device size; continue to use device | Consult your physician |
| Comments | Problem usually resolves as you continue to use your device. Reference the printed "Instructions for Use" booklet included with your device for more detailed information about wearing and using your device | Problem usually resolves as you continue to use your device. Reference the printed "Instructions for Use" booklet included with your device for more detailed information about wearing and using your device | Problem usually resolves itself if you change the size of your device and/or tension bands. | Problem usually resolves as you continue to use your device. Reference the printed "Instructions for Use" booklet included with your device for more detailed information about wearing and using your device | This device is intended to help men maintain an erection. This device is not intended to help achieve an erection or provide any of the physical and/or mental stimulus required to create the blood flow needed for an erection. |

In some examples, the devices and systems herein are designed to improve anxiety by allowing sexual activity that results in orgasms, which release endorphins, oxytocin, serotonin, and dopamine, and reduce cortisol levels.

In some examples, the devices and systems herein are designed to improve depression by allowing sexual activity that results in orgasms, which release endorphins, oxytocin, serotonin, and dopamine, and reduce cortisol levels. In some examples, the devices and systems herein allow for sexual activity including increased exercise, heart rate, and blood flow, with fights depression and impacts sexual function. With a leaner, toned body and a better sense of well-being and self-esteem made possible by some examples of the devices and systems herein, the user is more likely to feel sexually confident.

In some examples, the devices and systems herein are designed to improve post-traumatic stress disorder (PTSD) by allowing sexual activity that results in orgasms, which release endorphins, oxytocin, serotonin, and dopamine, and reduce cortisol levels. In some examples, the devices and systems herein assist with reconnecting with partner(s) in a relationship, restoring intimacy levels, communication, reinforcing support structure to help navigate PTSD. In some examples, the devices and systems herein are designed to give the user an increased sense of control. In some examples, the methods, devices, and systems herein enable improved family planning and/or sperm donation.

In some examples, the devices and systems herein are designed to relieve stress by allowing sexual activity that results in orgasms, which reduces the stress hormone cortisol, and releases endorphins, oxytocin, serotonin, and dopamine into the bloodstream.

In some examples, the devices and systems herein are designed to reduce users' intake of anti-anxiety medication by improving anxiety symptoms, by allowing sexual activity that results in orgasms, which reduces the stress hormone cortisol, and releases endorphins, oxytocin, serotonin, and dopamine into the bloodstream.

In some examples, the devices and systems herein are designed to reduce users' intake of anti-depression medication by improving anxiety symptoms, by allowing sexual activity that results in orgasms, which reduces the stress hormone cortisol, and releases endorphins, oxytocin, serotonin, and dopamine into the bloodstream.

In some examples, the devices and systems herein are designed to improve confidence of the user by providing successful sexual function, which can improve or restore positive self-perception as a healthy, functioning, purpose-fulfilling individual that can satisfy others; by facilitating the ability to provide physical and emotional satisfaction to partner(s). Sexual activity made possible by some examples of the devices and systems herein can result in orgasms, which release endorphins, oxytocin, serotonin, and dopamine, and reduce cortisol levels. With a leaner, toned body and a better sense of well-being and self-esteem made possible by some examples of the devices and systems herein, the user is more likely to feel sexually confident, resulting in an increased sense of overall confidence.

In some examples, the devices and systems herein are designed to improve self-esteem of the user by providing successful sexual function, which can improve or restore positive self-perception as a healthy, functioning, purpose-fulfilling individual that can satisfy others; by facilitating the ability to provide physical and emotional satisfaction to partner(s). Sexual activity made possible by the devices and systems herein can result in orgasms, which release endorphins, oxytocin, serotonin, and dopamine, and reduce cortisol levels. With a leaner, toned body and a better sense of well-being made possible by some examples of the devices and systems herein, the user is more likely to feel sexually confident, resulting in an increased sense of self-esteem.

In some examples, the devices and systems herein are designed to improve relationships by allowing successful sexual activity and mutual orgasms, which release endorphins, oxytocin, serotonin, and dopamine, and reduce levels of the stress hormone cortisol. Having sex helps build trust and connection between partners because they come to associate one another with these positive feelings. The physical and emotional satisfaction, including sexual satisfaction and orgasms provided by the devices and systems herein, can improve couples' communication, patience, understanding, and sense of collaboration during problem solving. In some examples, the devices and systems herein can be purchased over the counter not just by men with ED, but by their partner(s) as well, which provides them the purchasing power to make a positive change for all members of the relationship.

In some examples, the devices and systems herein are designed to improve couples' communication by allowing successful sexual activity and mutual orgasms, which solves the ongoing and monopolizing topic of the erectile dysfunction and sexual dissatisfaction in the relationship. Orgasms made possible by the devices and systems herein also release endorphins, oxytocin, serotonin, and dopamine, and reduce stress/cortisol levels. Having sex helps build trust and connection between partners because they come to associate one another with these positive feelings. In some examples, the devices and systems herein can be purchased over the counter by men with ED or their partner(s), giving the partner(s) the purchasing power to make a positive change for all members of the relationship; this act is its own form of communication, and is designed to stimulate further conversation about sexual satisfaction, expectations for the relationship, or increase the frequency of more generally pleasing, positive, uplifting subject matter.

In some examples, the devices and systems herein are designed to improve user's overall quality of life by improving their satisfaction with the sexual aspects of their life. In some examples, the devices and systems herein allows sexual activity that results in orgasms, which release endorphins, oxytocin, serotonin, and dopamine, and reduce cortisol levels. In some examples, the devices and systems herein can improve the user's sense of confidence by providing successful sexual function, which can improve or restore their positive self-perception as a healthy, functioning, purpose-fulfilling individual that can satisfy others physically and emotionally. Increased sexual activity using some examples of the devices and systems herein are designed to improve the user's self-image, and increase their satisfaction with their appearance and lifestyle, leading to an increased quality of life.

In some examples, the devices and systems herein are designed to allow sexual activity and orgasms that inspire action, improvement, or problem solving in other areas of the user's life including diet, exercise, stress, communication. By engaging in sex, in some examples, the devices and systems herein can facilitate improved self-image and confidence. Orgasms release endorphins, oxytocin, serotonin, and dopamine, and reduce stress/cortisol levels in the body. By confirming improvement and satisfaction is possible in the area of ED and sexual dissatisfaction, the user applies this realization knowingly or unknowingly as they face other challenges related to their health and wellness and quality of life. Where improvement was once thought impossible, there is now a sense of hope and determination. (Example: quitting smoking cigarettes to explore even further improvement of their sexual satisfaction.)

In some examples, the devices and systems herein are designed to improve focus and concentration by allowing sexual activity that results in orgasms, which release endorphins, oxytocin, serotonin, and dopamine, and reduces cortisol levels. By relaxing the state of the user's brain physiologically with an orgasm, the user will be able to concentrate better and focus on the task at hand.

In some examples, the devices and systems herein are designed to reduce the frequency and probability of couples' counseling by improving relationships, by allowing successful sexual activity and mutual orgasms, which release endorphins, oxytocin, serotonin, and dopamine, and reduce stress/cortisol levels. Having sex helps build trust and connection between partners because they come to associate one another with these positive feelings. The physical and emotional sexual satisfaction and orgasms provided by the devices and systems herein can improve couples' communication, patience, understanding, and sense of collaboration during problem solving. In some examples, the devices and systems herein can be purchased over the counter not just by men with ED, but by their partner(s) as well, which provides them the purchasing power to make a positive change for all members of the relationship.

In some examples, the devices and systems herein are designed to reduce the frequency of individual therapy or psychiatric care by improving the user's overall quality of life by improving their satisfaction with the sexual aspects of their life. In some examples, the devices and systems herein allows sexual activity that results in orgasms, which release endorphins, oxytocin, serotonin, and dopamine, and reduce cortisol levels. In some examples, the devices and systems herein can improve the user's sense of confidence by providing successful sexual function, which can improve or restore their positive self-perception as a healthy, functioning, purpose-fulfilling individual that can satisfy others physically and emotionally. Increased sexual activity using the devices and systems herein will improve the user's self-image, and increase their satisfaction with their appearance and lifestyle.

In some examples, the devices and systems herein are designed to reduce the probability of adultery or infidelity by improving the sexual satisfaction of all parties, and improving the relationship by allowing successful sexual activity and mutual orgasms, which release endorphins, oxytocin, serotonin, and dopamine, and reduce stress/cortisol levels. Having sex helps build trust and connection between partners because they come to associate one another with these positive feelings. The physical and emotional sexual satisfaction and orgasms provided by In some examples, the devices and systems herein can improve couples' communication, patience, understanding, and sense of collaboration during problem solving. In some examples, the devices and systems herein can be purchased over the counter not just by men with ED, but by their partner(s) as well, which provides them the purchasing power to make a positive change for all members of the relationship, all of which improves the overall satisfaction in the relationship and reduces the probability of adultery or infidelity.

In some examples, the devices and systems herein are to improve Diabetes by facilitating sexual activity that increases heart rate, blood flow, and activity rate, which increases the body's sensitivity and reaction to insulin, allowing the medicine to work more effectively. In some examples, the devices and systems herein allows for sexual activity and orgasms, which releases endorphins, oxytocin, serotonin, and dopamine, reduces stress/cortisol levels, and improves the user's positivity outlook on the treatment of their diabetes.

In some examples, the devices and systems herein are designed to improve High Blood Pressure by facilitating sexual intercourse, masturbation, or both, which lowers systolic blood pressure, the first number on the blood pressure test. High blood pressure is also improved with healthy sleeping habits, which In some examples, the devices and systems herein enables orgasms, that release prolactin, which is responsible for feelings of relaxation and sleepiness, in addition to facilitating ejaculation and/or orgasm, releasing endorphins, oxytocin, serotonin, and dopamine.

In some examples, the devices and systems herein are designed to help the user lose weight by facilitating sexual intercourse, masturbation, or both which increases heart rate, blood flow, and burns approximately five calories per minute. In some examples, the devices and systems herein can provide an increase in confidence by providing successful sexual experiences, and by allowing the user to provide physical and emotional satisfaction to themselves or their partner(s). Sexual activity made possible by In some examples, the devices and systems herein allows for orgasms, which release endorphins, oxytocin, serotonin, and dopamine, and reduce stress/cortisol levels. With perceived improvement in heart rate, exercise, and toward a leaner, toned body, the user is more likely to feel sexually confident and proceed with exercise and positive lifestyle changes.

In some examples, the devices and systems herein is designed to improve heart disease by making possible sexual activity, which lowers systolic blood pressure. Men who have sex at least twice a week, and women who report having satisfying sex lives, are less likely to have a heart attack. In some examples, the devices and systems herein is designed to strengthen couples' bond, lower feelings of loneliness, depression and anxiety, which have been linked to higher heart disease risk.

In some examples, the devices and systems herein are designed to decrease the risk of prostate cancer by allowing sexual activity that leads to higher ejaculation frequency, shown to flush out harmful chemicals that might build up in semen, decreasing the risk of prostate cancer.

In some examples, the devices and systems herein are designed to reduce pain by facilitating sexual activity and orgasms, which releases pain-relieving endorphins, dopamine and oxytocin. For female partners, vaginal stimulation can block chronic back and leg pain.

In some examples, the devices and systems herein are designed to increase exercise by facilitating sexual intercourse, masturbation, or both which increases heart rate, blood flow, and burns about five calories per minute. In some examples, the devices and systems herein can provide the confidence and inspiration to seek improvement and continuation in other facets of the user's life, like exercise.

In some examples, the devices and systems herein are designed to improve the user's heart health by enabling sexual activity that increases heart rate, blood flow, exercise levels, and reduces stress and blood pressure, cutting the chances of heart diseases by approximately 50%. In some examples, the devices and systems herein can provide orgasms that release endorphins, oxytocin, serotonin, and dopamine, and reduce stress/cortisol levels. Sex helps keep estrogen and testosterone levels in balance which helps avoid osteoporosis and heart disease.

In some examples, the devices and systems herein are designed to increase testosterone in men with Low T by enabling sexual activity and orgasms, which increases testosterone production. In some examples, the devices and systems herein also increases testosterone by improving sleep, reducing stress, increasing exercise, staying active, and maintaining a healthy weight.

In some examples, the devices and systems herein are designed to help individuals start a family by enabling sexual intercourse, masturbation, or both, and ejaculation of sperm.

In some examples, the devices and systems herein facilitates sexual activity and orgasms which lead to partial or complete relief from headaches and migraines, due to the release of oxytocin, serotonin, dopamine, and endorphins during sex, which act as natural painkillers.

In some examples, the devices and systems herein are designed to decrease the risk of prostate cancer by allowing sexual activity that leads to higher ejaculation frequency, shown to flush out harmful chemicals that might build up in semen, decreasing the risk of prostate cancer.

In some examples, the devices and systems herein are designed to allow for sexual activity that improves skin health by increasing the hormone deydroepiandrosterone (DHEA), secreted by the adrenal gland, shown to give a more youthful and moisturized appearance to skin. Sexual intercourse, masturbation, or both releases the human growth hormone, which makes skin look more elastic.

In some examples, the devices and systems herein are designed to provide healthy sleep by increasing physical exertion and activity, and providing orgasms that release endorphins, oxytocin, serotonin, dopamine, and the hormone prolactin, which is responsible for the feelings of relaxation and sleepiness.

In some examples, the devices and systems herein are designed to improve the user's immune system by providing orgasms that increase the body's defense against germs and viruses. The device facilitates sexual intercourse, masturbation, or both which increases physical activity, heart rate, mobility, and exercise, which also improves the efficiency of the user's immune system.

In some examples, the devices and systems herein are designed to treat erectile dysfunction caused by excessive alcohol consumption. In some examples, the devices and systems herein constricts the veins in the penis but avoids the arteries, to assist in maintaining erections, a scientific process purposely designed to be effective against the body's physiological reaction to alcohol.

In some examples, the devices and systems herein are designed to treat erectile dysfunction caused by stress. In some examples, the devices and systems herein constricts the veins in the penis but avoids the arteries, to assist in maintaining erections, a scientific process purposely designed to be effective against the body's physiological reaction to stress. In some examples, the devices and systems herein is designed to relieve stress by allowing sexual activity that results in orgasms, which reduces the stress hormone cortisol, and releases endorphins, oxytocin, serotonin, and dopamine into the bloodstream.

In some examples, the devices and systems herein are designed to treat erectile dysfunction caused by anxiety. In some examples, the devices and systems herein constricts the veins in the penis but avoids the arteries, to assist in maintaining erections, a scientific process purposely designed to be effective against the body's physiological reaction to anxiety. In some examples, the devices and systems herein is designed to relieve stress by allowing sexual activity that results in orgasms, which reduces the stress hormone cortisol, and releases endorphins, oxytocin, serotonin, and dopamine into the bloodstream.

In some examples, the devices and systems herein are designed to treat erectile dysfunction caused by depression. In some examples, the devices and systems herein constricts the veins in the penis but avoids the arteries, to assist in maintaining erections, a scientific process purposely designed to be effective against the body's physiological reaction to depression. In some examples, the devices and systems herein is designed to relieve depression by allowing sexual activity that results in orgasms, which reduces the stress hormone cortisol, and releases endorphins, oxytocin, serotonin, and dopamine into the bloodstream.

In some examples, the devices and systems herein are designed to treat erectile dysfunction caused by obesity. In some examples, the devices and systems herein constricts the veins in the penis but avoids the arteries, to assist in maintaining erections, a scientific process purposely designed to be effective against the body's physiological reaction to obesity. In some examples, the devices and systems herein is designed to facilitate sexual intercourse, masturbation, or both which increases exercise, heart rate, activity rate, and can burn 5 calories per minute, helping to reduce the user's weight, and, in some examples, the devices and systems herein is designed to relieve stress by allowing sexual activity that results in orgasms, which reduces the stress hormone cortisol, and releases endorphins, oxytocin, serotonin, and dopamine into the bloodstream.

In some examples, the devices and systems herein are designed to treat erectile dysfunction caused by diabetes. In some examples, the devices and systems herein constricts the veins in the penis but avoids the arteries, to assist in maintaining erections, a scientific process purposely designed to be effective against the body's physiological reaction to diabetes. In some examples, the devices and systems herein is designed to relieve stress by allowing sexual activity that results in orgasms, which reduces the stress hormone cortisol, and releases endorphins, oxytocin, serotonin, and dopamine into the bloodstream. In some examples, the devices and systems herein are designed to increase heart rate, blood flow, and activity rate, which increases the body's sensitivity and reaction to insulin, allowing the medicine to work more effectively. In some examples, the devices and systems herein allow for sexual activity and orgasms, which releases endorphins, oxytocin, serotonin, and dopamine, reduces stress/cortisol levels, and improves the user's positivity outlook on the treatment of their diabetes.

In some examples, the devices and systems herein are designed to be used with a vacuum pump by (1) placing the device around the vacuum pump, (2) using the pump to achieve an erection, and (3) sliding the device off the pump and onto the penis, wherein trapped blood maintains the erection. In some examples, the devices and systems herein are designed to be used with ED medications. In some examples, the devices and systems herein are designed to be used with all body-safe lubricants, especially when installing the device with the tension band. In some examples, the devices and systems herein are designed to be particularly effective and satisfying after shaving/trimming of one's pubic hair, to minimize the risk of pulling, yanking, or uncomfortable friction between the device and pubic hair. In some examples, the devices and systems herein are designed to be used with all male condoms and female contraceptive devices.

### Alternative Treatment Methods

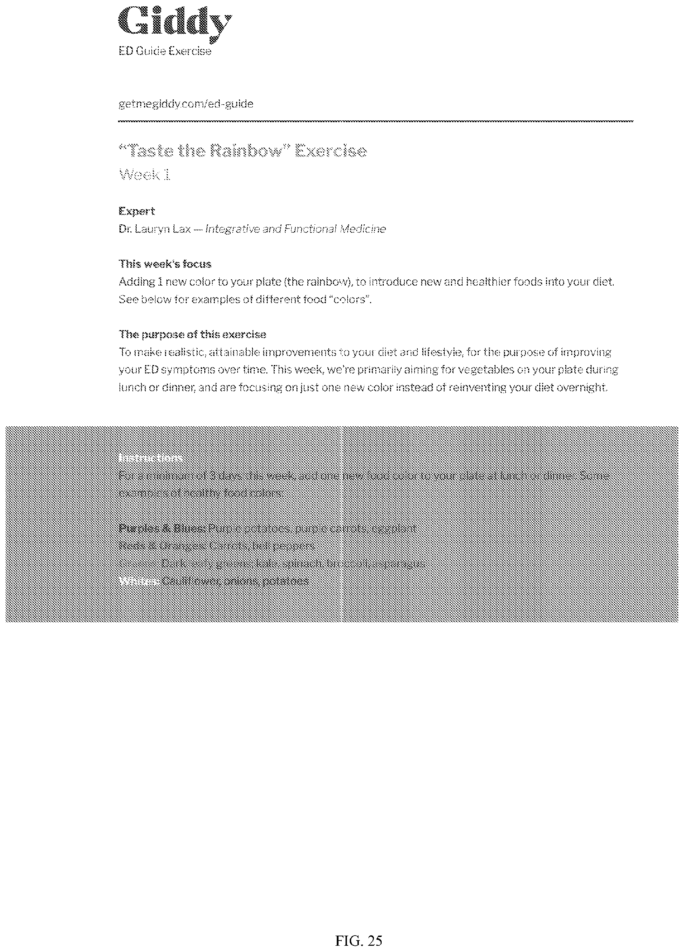

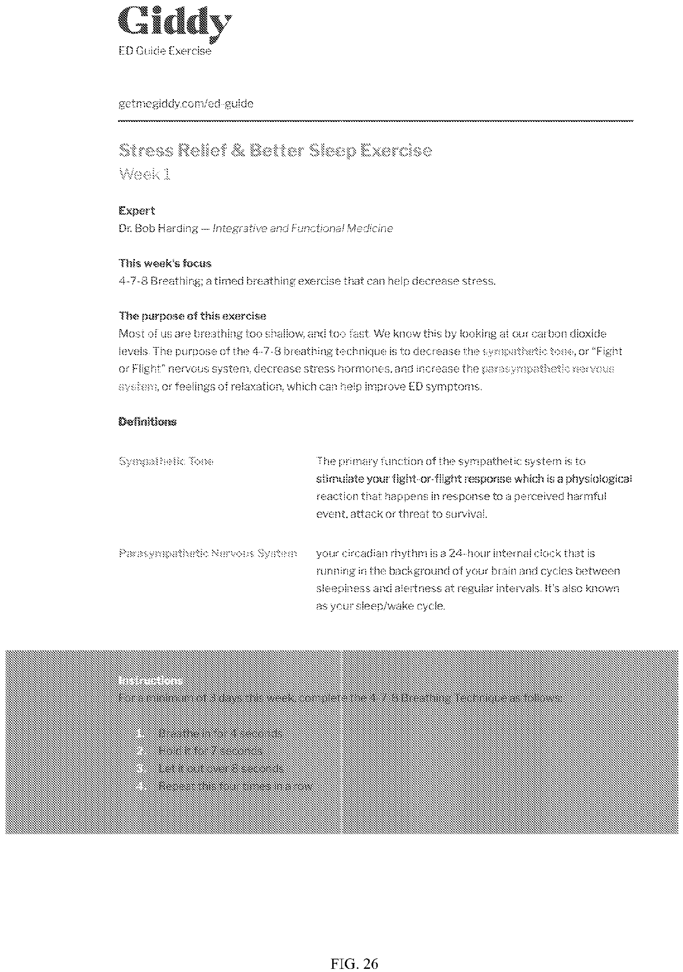

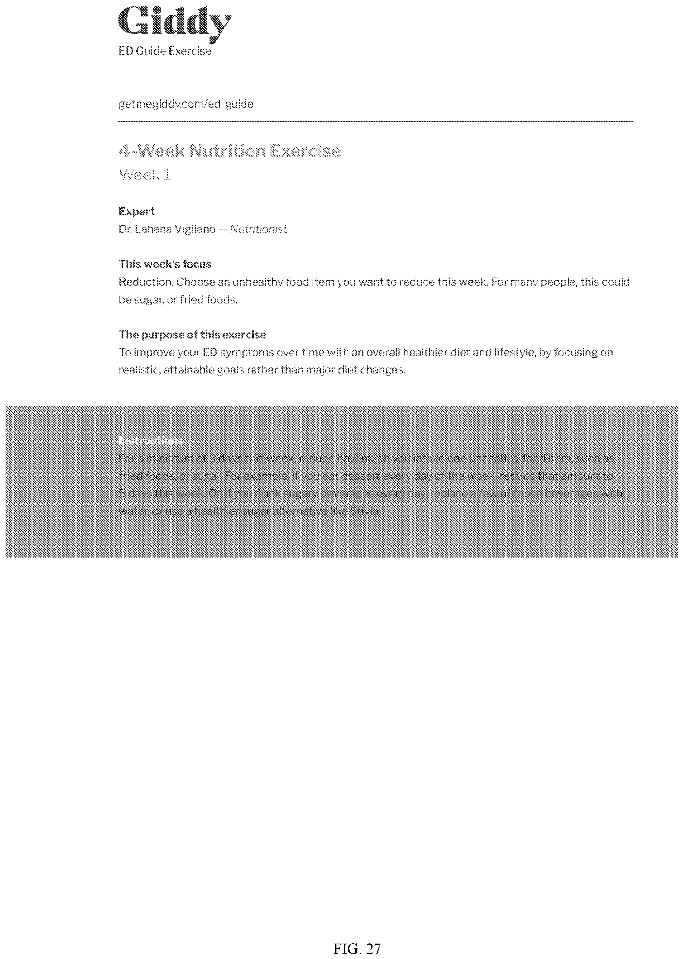

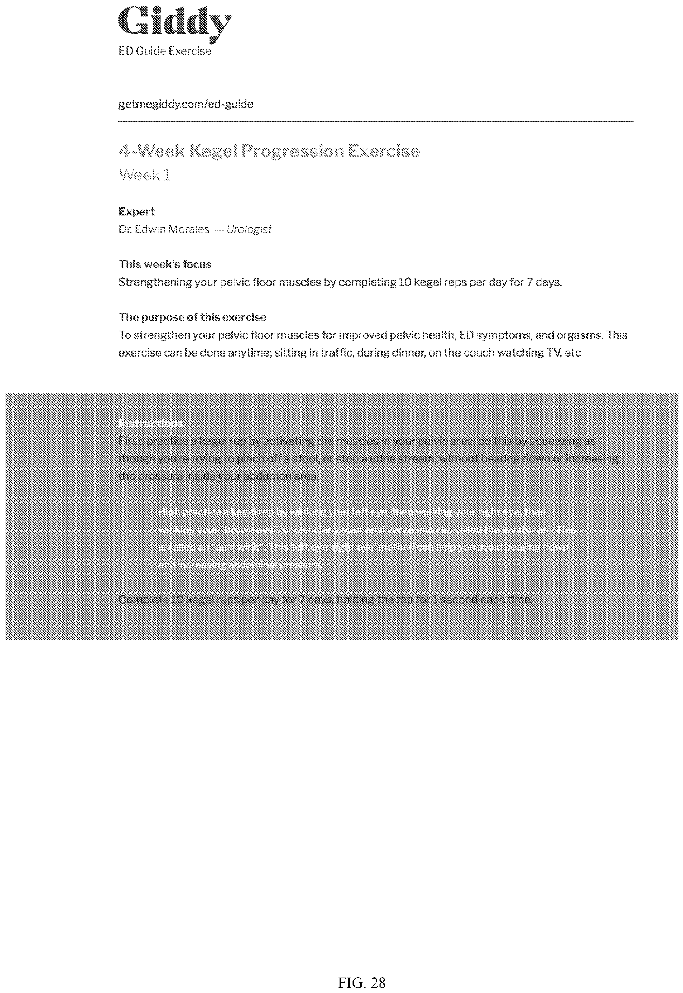

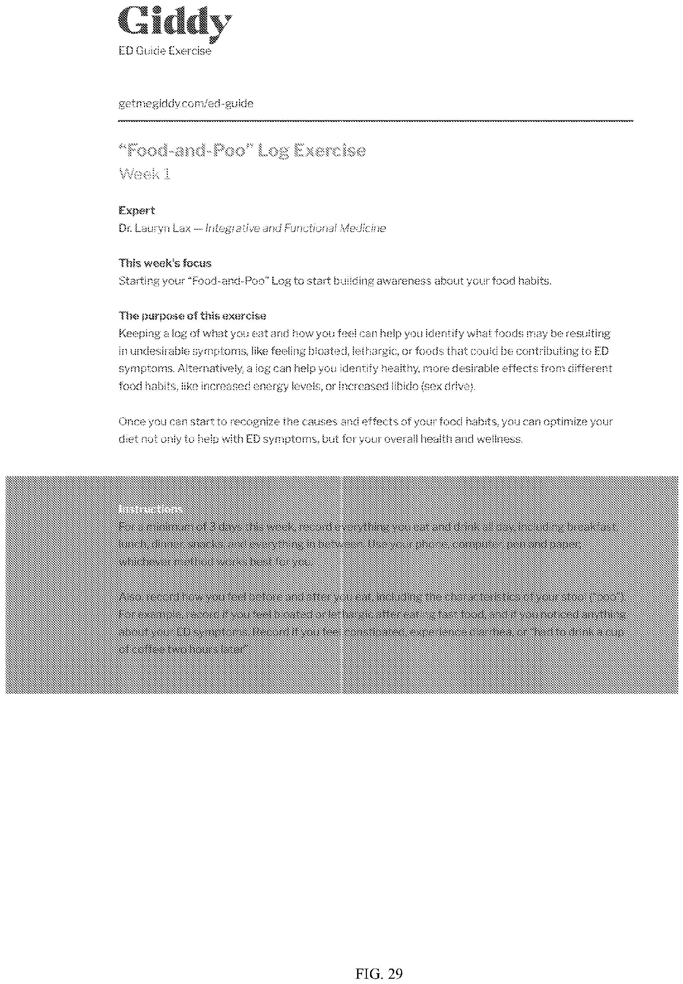

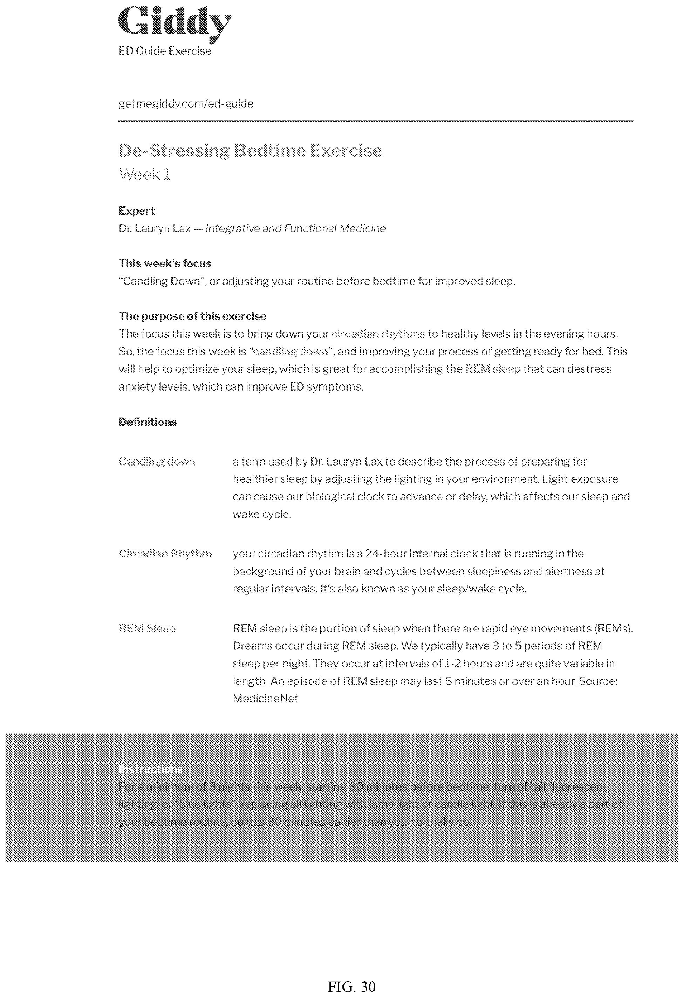

Alternative treatment methods for erectile dysfunction include counseling, drug therapy, hormonal therapy, vascular surgery, and implanted prosthetic devices. Contact your physician for more information regarding these alternative treatments. In some examples, the kit may further include further instructions to treat erectile dysfunction through, per **FIGS. 25-30****,** diet, stress relief, exercise, nutrition, Kegel exercises, and tracking.

Optionally, in this or any other example, the number of penile constriction devices is 1 to 500. Optionally, in this or any other example, the penile constriction devices comprises two or more different sizes, models or and/or shapes of penile constriction devices. Optionally, in this or any other example, the two or more different sizes, models or and/or shapes of penile constriction devices are color coded. Optionally, in this or any other example, the two or more different sizes, models or and/or shapes of penile constriction devices are labeled. Optionally, in this or any other example, the two or more different sizes, models or and/or shapes of penile constriction devices enable a user to select a device that functions best, is most comfortable, and most optimally corresponds with the size, shape, and biology of their penis.

Optionally, in this or any other example, the number of bands is 1 to 500. Optionally, in this or any other example, the bands comprises two or more different sizes, models or and/or shapes of bands. Optionally, in this or any other example, the two or more different sizes, models or and/or shapes of bands are color coded. Optionally, in this or any other example, two or more different sizes, models or and/or shapes of bands are labeled. Optionally, in this or any other example, two or more different sizes, models or and/or shapes of bands enable a user to customize the compressive force imparted by the device onto the penis that is most optimal for with the size, shape, and biology of their penis.

### Current Penile Construction Devices

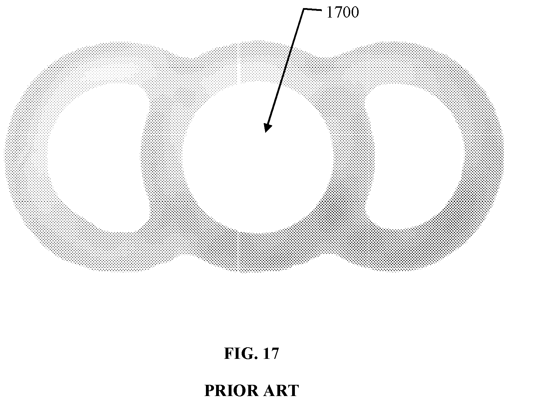

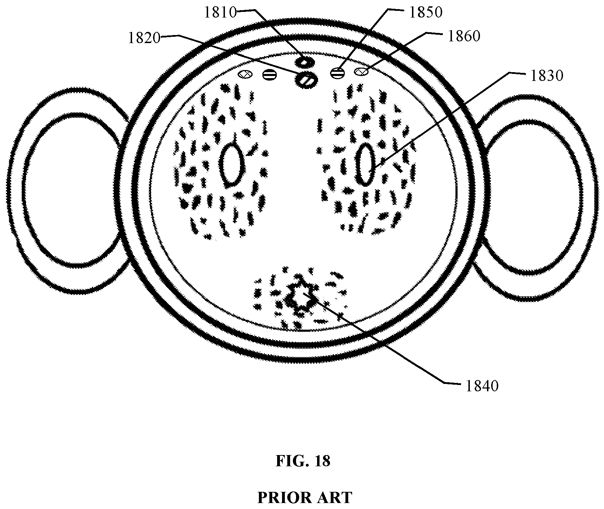

**FIG. 17** shows a front view of a penile constriction device in the prior art. **FIG. 18** shows a front cross-sectioned view of the prior art penile constriction device in **FIG. 17** on a human penis. To highlight, by contrast, the prior art Encore Tension Device, per **FIGS. 17** and **18****,** is configured to apply both a uniform pressure and a non-selective pressure on the entire circumference of the penis, because its penile cavity **1700** is circular, continuous, elastic, and non-rigid. Further, the prior art Encore Tension Band is not configured to apply a non-uniform pressure or a selective non-uniform pressure on the circumference of the penis, because its penile cavity **2000** is circular, continuous, elastic, and non-rigid. As such, in contrast to the devices and systems of the current disclosure, the Encore Tension Band per **FIG. 18** is configured to apply a significant compressive force to the deep arteries **1830,** the dorsal arteries **1850,** the urethra **1870,** and the dorsal nerves **1860,** which prevents blood from flowing into the penis, prevents the use of Kegel exercises to maintain an erection, and causes injaculation and damages the dorsal nerves **1860.**

Further, because the prior art Encore Tension Band has a circular penile cavity **2000,** and as the cross sectional shape of the human penis is elliptical, the majority of the uniform pressure applied by the Encore Tension Band is directed towards the deep arteries **1830,** which bring blood into the penis to maintain an erection.

Additionally, because the prior art Encore Tension Band is fully elastic and configured to provide a uniform penile pressure, it is only effective when worn on an erect penis, and is ineffective when worn on a flaccid penis, a slightly flaccid penis, or a slightly rigid penis. As such, the prior art Encore Tension Band is incapable of facilitating erection initiation.

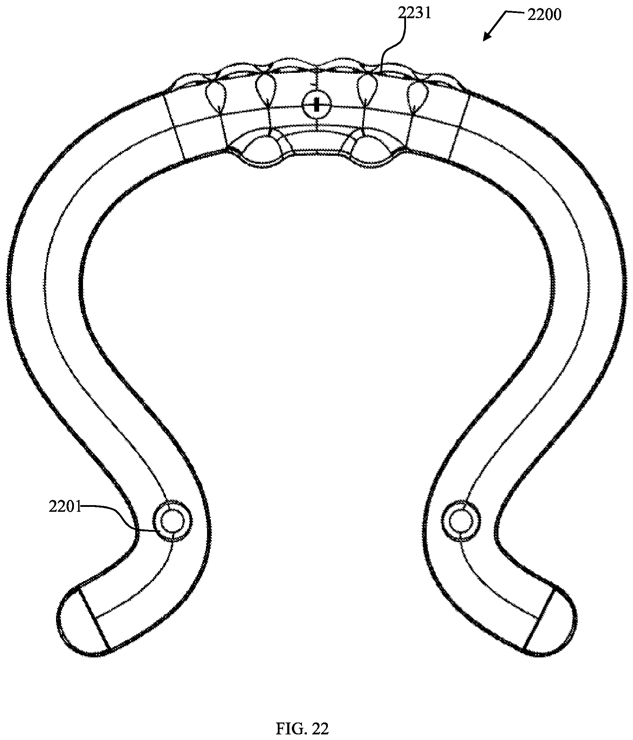

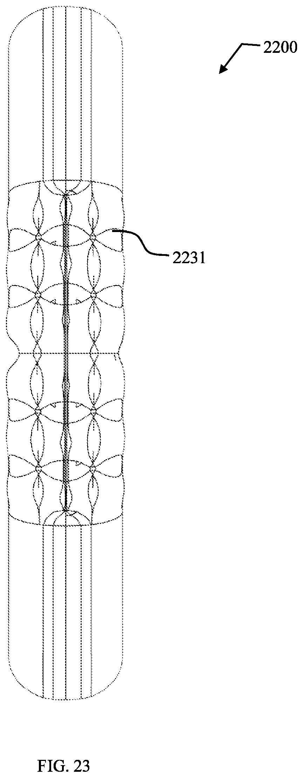

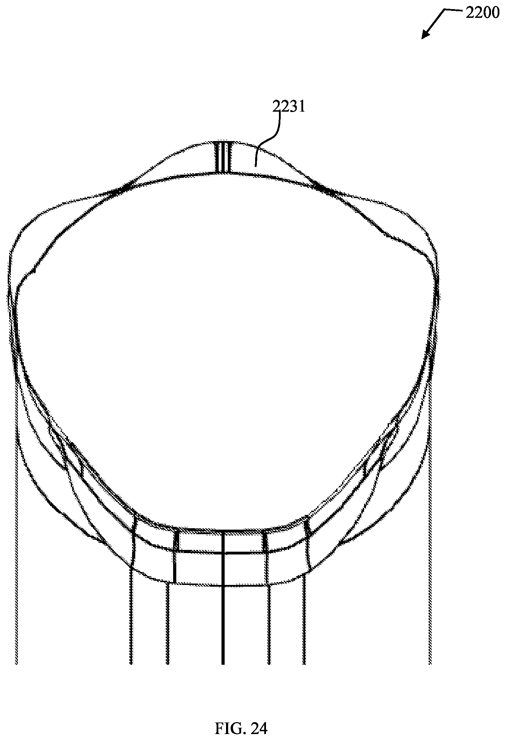

**FIGS. 22-24** shows a non-limiting example of a penile constriction device **2200** having a manufacturing mark **2201.** In some examples, a size, orientation, location, depth, or any combination thereof of the manufacturing mark **2201** enables the production of the penile constriction device **2200** that provides sufficient force between its arms.

### Terms and Definitions

Unless otherwise defined, all technical terms used herein have the same meaning as commonly understood by one of ordinary skill in the art to which this disclosure belongs.

As used herein, the singular forms "a," "an," and "the" include plural references unless the context clearly dictates otherwise. Any reference to "or" herein is intended to encompass "and/or" unless otherwise stated.

As used herein, the term "about" refers to an amount that is at the stated amount or near the stated amount by 25%, 20%, 15%, 10%, 5%, 2%, or 1%, including increments therein. When the amount is a percentage, the term "about" means a range around the given amount, e.g. about 10% refers to 10% +/-1% (i.e. 9%-11%), or 10% +/- 2% (i.e. 8%-12%), etc. depending on the example and amount.

As used herein, the term "mesial" refers to a longitudinal direction or relation towards the center.

As used herein, the term "transverse" refers to a longitudinal direction or relation away from the center.

As used herein, the term "apex" refers to a point of inflection in one or more directions.

As used herein, the term "normal" refers to perpendicularity in one or more directions.

As used herein, the term "curvilinear" refers to a shape comprising one or more curves.

As used herein, the term "girth" refers to a circumference of a portion of a male human penis.

As used herein, the term "circular diameter" refers to an outer width, *e.g.* a transverse width, of a portion of a male human penis.

### EXAMPLES

The following illustrative examples are representative of embodiments of the applications, systems, and methods described herein and are not meant to be limiting in any way. Optionally, in other embodiments, each of the following examples can additionally include any of the aforementioned embodiments and elements.

### Example 1: First Penile Constriction Device

Provided herein, per **FIG. 6****,** is a first penile constriction device **600** comprising an arched rod having a constriction gap width **661,** a constriction height **680,** a maximum inner width **670,** an inferior height **690,** and a thickness **651,** as shown in Table 2 below. For the first penile constriction device **600,** the constriction gap width **661** is measured as a minimum distance between the first converging end and the second converging end within the constriction region. For the first penile constriction device **600,** the constriction height **680** is measured as a normal distance between a constriction point **662** and the inferior side of the bridge, wherein the constriction point **662** is defined as the midpoint of the constriction gap width **661.** For the first penile constriction device **600,** the maximum inner width **670** is measured as a maximum normal distance between a mesial vertex of the surface of the first arm and a mesial vertex of the surface of the second arm. For the first penile constriction device **600,** the inferior height **690** is measured as a normal distance between the center point **662** and the inferior side of the bridge. For the first penile constriction device **600,** the thickness **651** is measured as a maximum normal cross-sectional width of at least a portion of at least one of the first arm, the second arm, and the bridge.

**Table 2**

| Dimension | Min | Max |
|---|---|---|
| Constriction Gap Width | 0.526 | 0.594 |
| Constriction Height | 0.975 | 1.099 |
| Maximum Inner Width | 1.170 | 1.319 |
| Inferior Height | 0.390 | 0.440 |
| Thickness | 0.2 | 0.7 |

A cross-sectional area of a penis may be defined as the cross sectional area within the circumference of a flaccid or erect penis. Alternatively, the cross-sectional area is defined as the area of a circle having a diameter equal to the girth of a flaccid or erect penis. Alternatively, the cross-sectional area is defined as the area of a circle having a diameter equal to the circular diameter of a flaccid or erect penis. Exemplary cross sectional areas are shown in Tables 10 and 11. A cross-sectional constriction percentage is defined as a percentage by which the cross-sectional area of the flaccid or erect penis at the intersection of the device **600** and the flaccid or erect penis is reduced when the device **600** is worn on the flaccid or erect penis. Alternatively, the cross-sectional constriction percentage is defined as a percentage by which the cross-sectional area of the flaccid or erect penis at a point on the flaccid or erect penis is reduced when the device **600** is worn on the flaccid or erect penis. In some embodiments, the flaccid and erect cross-sectional constriction percentages represent a uniform cross-sectional constriction of the flaccid penis and the erect penis, respectively. In some embodiments, the flaccid and erect cross-sectional constriction percentages represent a non-uniform cross-sectional constriction of the flaccid penis and the erect penis, respectively. The cross-sectional constriction percentage imparted by the first penile constriction device **600** on a penis of general size A, per Table 1, is shown in Table 3 below.

**Table 3**

| | Flaccid | Erect |
|---|---|---|
| Min | 20.00 % | 10.36 % |
| Max | 37.13 % | 29.55 % |

In some cases, the minimum and maximum cross sectional constriction percentages per Table 3, represent the minimum and maximum cross sectional constrictions recommended for use of the first penile constriction device **600** on a flaccid penis or an erect penis, respectively. In some cases, the minimum and maximum cross sectional constriction percentages per Table 3, and the dimensions of the first penile constriction device **600** (Size S), per Table 3, indicate the recommended penis size dimensions, per Table 10 or 11 below. In some embodiments, the first penile constriction device **600** does not comprise the friction protrusions. In some embodiments, the first penile constriction device **600** does not comprise the first dorsal vein protrusion or the second dorsal vein protrusion. In some embodiments, the first penile constriction device **600** does not comprise the friction protrusions, the first dorsal vein protrusion, or second dorsal vein protrusion.

### Example 2: Second Penile Constriction Device

Provided herein, per **FIG. 7****,** is a second penile constriction device **700** comprising an arched rod having a constriction gap width **761,** a constriction height **780,** a maximum inner width 770, an inferior height **790,** and a thickness **751,** as shown in Table 4 below. For the second penile constriction device **700,** the constriction gap width **761** is measured as a minimum distance between the first converging end and the second converging end within the constriction region. For the second penile constriction device **700,** the constriction height **780** is measured as a normal distance between a constriction point **762** and the inferior side of the bridge, wherein the constriction point **762** is defined as the midpoint of the constriction gap width **761.** For the second penile constriction device **700,** the maximum inner width **770** is measured as a maximum normal distance between a mesial vertex of the surface of the first arm and a mesial vertex of the surface of the second arm. For the second penile constriction device **700,** the inferior height **790** is measured as a normal distance between the center point **762** and the inferior side of the bridge. For the second penile constriction device **700,** the thickness **751** is measured as a maximum normal cross-sectional width of at least a portion of at least one of the first arm, the second arm, and the bridge.

**Table 4**

| Dimension | Min | Max |
|---|---|---|
| Constriction Gap Width | 0.594 | 0.650 |
| Constriction Height | 1.099 | 1.203 |
| Maximum Inner Width | 1.319 | 1.444 |
| Inferior Height | 0.440 | 0.481 |
| Thickness | 0.2 | 0.7 |

A cross-sectional area of a penis may be defined as the cross sectional area within the circumference of a flaccid or erect penis. Alternatively, the cross-sectional area is defined as the area of a circle having a diameter equal to the girth of a flaccid or erect penis. Alternatively, the cross-sectional area is defined as the area of a circle having a diameter equal to the circular diameter of a flaccid or erect penis. Exemplary cross sectional areas are shown in Tables 10 and 11. A cross-sectional constriction percentage is defined as a percentage by which the cross-sectional area of the flaccid or erect penis at the intersection of the device **600** and the flaccid or erect penis is reduced when the device **600** is worn on the flaccid or erect penis. Alternatively, the cross-sectional constriction percentage is defined as a percentage by which the cross-sectional area of the flaccid or erect penis at a point on the flaccid or erect penis is reduced when the device **600** is worn on the flaccid or erect penis. In some embodiments, the flaccid and erect cross-sectional constriction percentages represent a uniform cross-sectional constriction of the flaccid penis and the erect penis, respectively. In some embodiments, the flaccid and erect cross-sectional constriction percentages represent a non-uniform cross-sectional constriction of the flaccid penis and the erect penis, respectively. The cross-sectional constriction percentage imparted by the second penile constriction device **700** on a penis of general size B, per Table 1, is shown in Table 5 below.

**Table 5**

| | Flaccid | Erect |
|---|---|---|
| Min | 20.00 % | 10.36 % |
| Max | 33.22 % | 25.18 % |

In some cases, the minimum and maximum cross sectional constriction percentages per Table 5, represent the minimum and maximum cross sectional constrictions recommended for use of the second penile constriction device **700** on a flaccid penis or an erect penis, respectively. In some cases, the minimum and maximum cross sectional constriction percentages per Table 5, and the dimensions of the second penile constriction device **700** (Size M), per Table 5, indicate the recommended penis size dimensions, per Table 10 or 11 below. In some embodiments, the first penile constriction device **600** does not comprise the friction protrusions. In some embodiments, the first penile constriction device **600** does not comprise the first dorsal vein protrusion or the second dorsal vein protrusion. In some embodiments, the first penile constriction device **600** does not comprise the friction protrusions, the first dorsal vein protrusion, or second dorsal vein protrusion.

### Example 3: Third Penile Constriction Device

Provided herein, per **FIG. 8****,** is a third penile constriction device **800** comprising an arched rod having a constriction gap width **861,** a constriction height **880,** a maximum inner width **870,** an inferior height **890,** and a thickness **851,** as shown in Table 6 below. For the third penile constriction device **800,** the constriction gap width **861** is measured as a minimum distance between the first converging end and the second converging end within the constriction region. For the third penile constriction device **800,** the constriction height **880** is measured as a normal distance between a constriction point **862** and the inferior side of the bridge, wherein the constriction point **862** is defined as the midpoint of the constriction gap width **861.** For the third penile constriction device **800,** the maximum inner width **870** is measured as a maximum normal distance between a mesial vertex of the surface of the first arm and a mesial vertex of the surface of the second arm. For the third penile constriction device **800,** the inferior height **890** is measured as a normal distance between the center point **862** and the inferior side of the bridge. For the third penile constriction device **800,** the thickness **851** is measured as a maximum normal cross-sectional width of at least a portion of at least one of the first arm, the second arm, and the bridge.

**Table 6**

| Dimension | Min | Max |
|---|---|---|
| Constriction Gap Width | 0.650 | 0.705 |
| Constriction Height | 1.203 | 1.305 |
| Maximum Inner Width | 1.444 | 1.566 |
| Inferior Height | 0.481 | 0.522 |
| Thickness | 0.2 | 0.7 |

A cross-sectional area of a penis may be defined as the cross sectional area within the circumference of a flaccid or erect penis. Alternatively, the cross-sectional area is defined as the area of a circle having a diameter equal to the girth of a flaccid or erect penis. Alternatively, the cross-sectional area is defined as the area of a circle having a diameter equal to the circular diameter of a flaccid or erect penis. Exemplary cross sectional areas are shown in Tables 10 and 11. A cross-sectional constriction percentage is defined as a percentage by which the cross-sectional area of the flaccid or erect penis at the intersection of the device **600** and the flaccid or erect penis is reduced when the device **600** is worn on the flaccid or erect penis. Alternatively, the cross-sectional constriction percentage is defined as a percentage by which the cross-sectional area of the flaccid or erect penis at a point on the flaccid or erect penis is reduced when the device **600** is worn on the flaccid or erect penis. In some embodiments, the flaccid and erect cross-sectional constriction percentages represent a uniform cross-sectional constriction of the flaccid penis and the erect penis, respectively. In some embodiments, the flaccid and erect cross-sectional constriction percentages represent a non-uniform cross-sectional constriction of the flaccid penis and the erect penis, respectively. The cross-sectional constriction percentage imparted by the third penile constriction device **800** on a penis of general size C, per Table 1, is shown in Table 7 below.

**Table 7**

| | Flaccid | Erect |
|---|---|---|
| Min | 20.00 % | 10.36 % |
| Max | 31.95 % | 23.75 % |

In some cases, the minimum and maximum cross sectional constriction percentages per Table 7, represent the minimum and maximum cross sectional constrictions recommended for use of the third penile constriction device **800** on a flaccid penis or an erect penis, respectively. In some cases, the minimum and maximum cross sectional constriction percentages per Table 7, and the dimensions of the third penile constriction device **800** (Size L), per Table 7, indicate the recommended penis size dimensions, per Table 10 or 12 below. In some embodiments, the third penile constriction device **800** does not comprise the friction protrusions. In some embodiments, the third penile constriction device **800** does not comprise the first dorsal vein protrusion or the second dorsal vein protrusion. In some embodiments, the third penile constriction device **800** does not comprise the friction protrusions, the first dorsal vein protrusion, or second dorsal vein protrusion.

### Example 4: Fourth Penile Constriction Device

Provided herein, per **FIG. 9****,** is a fourth penile constriction device **900** comprising an arched rod having a constriction gap width **961,** a constriction height **980,** a maximum inner width **970,** an inferior height **990,** and a thickness **951,** as shown in Table 8 below. For the fourth penile constriction device **900,** the constriction gap width **961** is measured as a minimum distance between the first converging end and the second converging end within the constriction region. For the fourth penile constriction device **900,** the constriction height **980** is measured as a normal distance between a constriction point **962** and the inferior side of the bridge, wherein the constriction point **962** is defined as the midpoint of the constriction gap width **961.** For the fourth penile constriction device **900,** the maximum inner width **970** is measured as a maximum normal distance between a mesial vertex of the surface of the first arm and a mesial vertex of the surface of the second arm. For the fourth penile constriction device **900,** the inferior height **990** is measured as a normal distance between the center point **962** and the inferior side of the bridge. For the fourth penile constriction device **900,** the thickness **951** is measured as a maximum normal cross-sectional width of at least a portion of at least one of the first arm, the second arm, and the bridge.

**Table 8**

| Dimension | Min |
|---|---|
| Constriction Gap Width | 0.705 |
| Constriction Height | 1.305 |
| Maximum Inner Width | 1.566 |
| Inferior Height | 0.522 |
| Thickness | 0.2 |

A cross-sectional area of a penis may be defined as the cross sectional area within the circumference of a flaccid or erect penis. Alternatively, the cross-sectional area is defined as the area of a circle having a diameter equal to the girth of a flaccid or erect penis. Alternatively, the cross-sectional area is defined as the area of a circle having a diameter equal to the circular diameter of a flaccid or erect penis. Exemplary cross sectional areas are shown in Tables 10 and 11. A cross-sectional constriction percentage is defined as a percentage by which the cross-sectional area of the flaccid or erect penis at the intersection of the device **600** and the flaccid or erect penis is reduced when the device **600** is worn on the flaccid or erect penis. Alternatively, the cross-sectional constriction percentage is defined as a percentage by which the cross-sectional area of the flaccid or erect penis at a point on the flaccid or erect penis is reduced when the device **600** is worn on the flaccid or erect penis. In some embodiments, the flaccid and erect cross-sectional constriction percentages represent a uniform cross-sectional constriction of the flaccid penis and the erect penis, respectively. In some embodiments, the flaccid and erect cross-sectional constriction percentages represent a non-uniform cross-sectional constriction of the flaccid penis and the erect penis, respectively. The cross-sectional constriction percentage imparted by the fourth penile constriction device **900** on a penis of general size D, per Table 1, is shown in Table 9 below.

**Table 9**

| | Flaccid | Erect |
|---|---|---|
| Min | 20.00 % | 10.36 % |
| Max | 40.27 % | 30.08 % |

In some cases, the minimum and maximum cross sectional constriction percentages per Table 9, represent the minimum and maximum cross sectional constrictions recommended for use of the fourth penile constriction device **900** on a flaccid penis or an erect penis, respectively. In some cases, the minimum and maximum cross sectional constriction percentages per Table 9, and the dimensions of the fourth penile constriction device **900** (Size XL), per Table 8, indicate the recommended penis size dimensions, per Table 10 or 11 below. In some embodiments, the fourth penile constriction device **900** does not comprise the friction protrusions. In some embodiments, the fourth penile constriction device **900** does not comprise the first dorsal vein protrusion or the second dorsal vein protrusion. In some embodiments, the fourth penile constriction device **900** does not comprise the friction protrusions, the first dorsal vein protrusion, or second dorsal vein protrusion.

Although the exemplary, first, second, third, and fourth penile constriction devices 600 700 800 900 may be formed to different sizes, each exemplary device can include any of the elements in one or more of the embodiments herein.

### Example 5: Chart for Constriction Device Sizing

As described in the examples above, the exemplary second, third, fourth, and fifth penile constriction devices **600, 700, 800, 900,** respectively, alternatively denoted das sizes S, M, L, and XL, respectively may be generally prescribed to alleviate erectile dysfunction in males with a general penis size of A, B, C, and D, respectively. However, if a greater cross-sectional constriction percentage than those listed in Tables 3, 5, 7, and 9 is required to alleviate a severe ED condition, the S, M, L, and XL, devices may be prescribed per Table 10 below.

**Table 10**

| | | **Girth (cm)** | | **Circular Diameter (cm)** | | **ED Device** | |
|---|---|---|---|---|---|---|---|
| **General Penis Size** | | Flaccid | Erect | Flaccid | Erect | ED Condition Mild Severe | |
| **A** | Min | 2.990 | 3.750 | 0.952 | 1.194 | S | S |
| | Max | 3.373 | 4.230 | 1.074 | 1.346 | | |
| **B** | Min | 3.373 | 4.230 | 1.074 | 1.346 | M | S |
| | Max | 3.692 | 4.630 | 1.175 | 1.474 | | |
| **C** | Min | 3.692 | 4.630 | 1.175 | 1.474 | L | M |
| | Max | 4.003 | 5.020 | 1.274 | 1.598 | | |
| **D** | Min | 4.003 | 5.020 | 1.274 | 1.598 | XL | L |
| | Max | 4.633 | 5.810 | 1.475 | 1.849 | | |

Further, Table 10 can be employed as a template for a user to select the correct exemplary constriction device based on their general penis size and the severity of their ED condition.

### Example 6: First Example of Selecting an ED Device

Sam does not have ED, but wants to purchase an ED device to enhance his erection. However, Sam does not know which size ED device is appropriate for his situation. Sam reads the sizing recommendations per Table 10 above and sees that the table provides a range of penis sizes for different categories that correspond to different sizes. Measuring the girth of his erect penis, Sam learns that his erect penis falls within the range of a Min. and Max. for a general penis size "C". Sam visits his local store and purchases a Size L device. When he goes home, he finds out that the Size L device is a perfect fit. Because of the table, Sam does not have to guess which device is appropriate for his situation.

### Example 7: Second Example of Selecting an ED Device

Joe has been experiencing mild signs of erectile dysfunction and decides to buy an ED device. Joe measures the girth of his flaccid and erect penis as 3.5 inches and 4.5 inches, respectively, using a tape measure. Joe references the sizing recommendations chart, per Table 10 above, to determine that his general penis size is size "B" and that, as his ED symptoms are mild, he should purchase a Size M ED device. Joe purchases the Size M ED device, which comes with a tension band separately so the Size M ED device and tension bands are two separate pieces. Joe fits the Size M ED device along with the tension band, which alleviates his mild ED symptoms.

As Joe ages his mild ED symptoms exacerbate and become severe. Joe references the user manual to determine that, as his symptoms have worsened, the use of a Size S ED device is now recommended. Joe purchases the Size 1 ED devices, which comes with a tension band separately. Joe opts to fit the Size S ED device without the tension band and finds that the Size 1 ED device alleviates his severe ED symptoms.

### Example 8: Third Example of Selecting an ED Device

Tom has been experiencing severe signs of erectile dysfunction and decides to buy an ED device. Tom measures the circular diameter of his flaccid and erect penis as 1.2 inches and 1.5 inches, respectively, using a ruler. Tom references the sizing recommendations chart, per Table 10 above, to determine that his general penis size is size "C" and that, as his ED symptoms are severe, he should purchase a Size M ED device. Tom purchases the Size M ED device, which comes with a tension band attached so the Size M ED device and tension band is one piece. The Size M ED device fits Tom well and alleviates his severe ED symptoms.

### Example 9: Band Dimensions

Optionally, in this or any other example, the dimensions of the primary band, per **FIG 50B****,** vary based on a tension level provided the primary band **1500.** Optionally, in this or any other example, the primary band **1500** comprises a high tension primary band **1500A.** Optionally, in this or any other example, the primary band **1500** comprises a low tension primary band **1500B.** The dimensions (in inches) of the depth **1501,** the width **1502,** the outer diameter **1503,** the tab thickness **1504,** and the hoop thickness **1505** for the high tension band **1500A** (having sizes 1 and 2) and for the low tension band **1500B** (having sizes 1-4) are shown in table 11 below.

**Table 11**

| **Tension** | **Size** | **For Device Size** | **1501** | **1502** | **1503** | **1504** | **1505** |
|---|---|---|---|---|---|---|---|
| High | 1 | S/M | 0.2-0.3 | 0.8-0.9 | 0.3-0.4 | 0.06-0.07 | 0.06-0.07 |
| | 2 | L/XL | 0.2-0.3 | 0.85-0.95 | 0.4-0.5 | 0.06-0.07 | 0.06-0.07 |
| Low | 1 | S | 0.2-0.3 | 1.0-1.2 | 0.5-0.7 | 0.06-0.07 | 0.06-0.07 |
| | 2 | M | 0.2-0.3 | 1.0-1.2 | 0.6-0.8 | 0.06-0.07 | 0.06-0.07 |
| | 3 | L | 0.2-0.3 | 1.0-1.2 | 0.6-0.8 | 0.06-0.07 | 0.06-0.07 |
| | 4 | XL | 0.2-0.3 | 1.0-1.3 | 0.6-0.8 | 0.06-0.07 | 0.06-0.07 |

Optionally, in this or any other example, as the dimensions of the high tension band **1500A** are smaller than the dimensions of the low tension band **1500B,** the high tension band **1500A** imparts a greater force when applied on the devices herein. Optionally, in this or any other example, the high tension band **1500A** high tension band **1500A** high tension band **1500A** and the low tension band **1500B** are formed of the same material. Optionally, in this or any other example, the high tension band **1500A** and the low tension band **1500B** are formed of different materials. Optionally, in this or any other example, the high tension band **1500A** are formed of a material having a higher modulus of elasticity than the material of the low tension band **1500B.**

### Example 10: Choosing a Low Tension Band

After measuring his flaccid and erect penis, Paul determines that his general penis size is size "D" and that, as his ED symptoms are mild, he should purchase a Size XL ED device. However, during intercourse, the size XL ED device rotates and translates from its initial position on his penis such that the efficacy of the XL ED device is diminished. Paul then pinches each tab of a high tension band that arrived with his XL ED device, and applies tension on the band while inserting the elbows of his XL ED band into the hoop of the band. Wearing his XL ED device with the high tension band placed thereon Paul finds that the device maintains its position and orientation during intercourse, but is uncomfortably tight. As such, Paul pinches the tabs of the high tension band and applies tension to the high tension band to remove the high tension band from his XL ED device. Paul then replaces the high tension band with a low tension band in the same way he attached the high tension band to his XL ED device. Now during intercourse, his XL ED device is comfortable and maintains its position and efficacy.

### Example 11: Choosing a High Tension Band

After measuring his flaccid and erect penis, Gary determines that his general penis size is size "D" and that, as his ED symptoms are severe, he should purchase a Size L ED device. However, during intercourse, his size L ED device rotates and translates from its initial position on his penis such that the efficacy of his size L ED device is diminished. Gary then pinches each tab of a low tension band that arrived with his size L ED device, and applies tension on the band while inserting the elbows of his size L ED band into the hoop of the band. Wearing his size L ED device with the low tension band placed thereon Gary experiences an improved efficacy but notices that his size L ED device rotates and translates slightly from its initial position on his penis. As such, Gary pinches the tabs of the low tension band and applies tension to the low tension band to remove the low tension band from his size L ED device. Gary then replaces the low tension band with a high tension band in the same way he attached the low tension band to his size L ED device. Now during intercourse, his size L ED device is maintaining its position and provides its full efficacy.

### Example 12: Symptom Improvement

In a clinical trial, 60 male participants, each at least 18 years, used the Giddy device for 16 weeks. Every four weeks, each participant provided rankings of their ED symptoms, sexual experiences, and lifestyles on a 1-10 scale old study. The results, per Table 12 below show marked improvements over a variety of categories.

**Table 12**

| **Question** | **Visit Average** | | | | **% Improved** |
|---|---|---|---|---|---|
| | **1** | **2** | **3** | **4** | |
| how often have you felt sexual desire? | 3.80 | 3.82 | 3.93 | 4.07 | 7% |
| how would you rate your level of sexual desire? | 3.27 | 3.36 | 3.54 | 3.86 | 18% |
| how satisfied have you been with your overall sex life? | 2.52 | 3.23 | 3.36 | 3.59 | 43% |
| how satisfied have you been with your sexual relationship with your partner? | 2.83 | 3.39 | 3.68 | 3.93 | 39% |
| how would you rate your confidence that you could achieve an erection? | 2.93 | 3.54 | 3.63 | 4.07 | 39% |
| how would you rate your confidence that you could keep an erection long enough to climax/orgasm? | 2.70 | 3.44 | 3.70 | 4.03 | 49% |
| how would you rate your overall satisfaction level with your erections? | 2.70 | 3.26 | 6.62 | 6.97 | 158% |
| how would you rate the quality of your erections? | 2.77 | 3.34 | 6.63 | 6.88 | 149% |
| how often have you felt the desire to masturbate? | 4.00 | 4.11 | 7.35 | 7.42 | 86% |
| how often have you felt like you were capable of masturbating? | 4.22 | 4.92 | 8.08 | 8.17 | 94% |
| how often did you attempt masturbation? | 2.68 | 3.11 | 6.35 | 6.31 | 135% |
| when you attempted masturbation, how often were you satisfied with your erection? | 3.70 | 4.69 | 7.77 | 8.12 | 119% |
| how much have you enjoyed masturbation? | 3.83 | 4.52 | 7.65 | 7.90 | 106% |
| when you attempted masturbation, how often were you able to achieve an erection? | 4.08 | 5.08 | 8.10 | 8.27 | 103% |
| when you were able to achieve an erection for masturbation, how long were you able to maintain your erection? | 2.77 | 4.03 | 7.15 | 7.24 | 162% |
| when you were able to achieve an erection for masturbation, how often were you able to maintain your erection to completion, i.e. to the point of ejaculation/climax/orgasm? | 4.15 | 5.03 | 8.00 | 8.29 | 100% |
| how often have you felt the desire to receive oral sex? | 4.10 | 4.20 | 7.45 | 7.76 | 89% |
| how often have you felt capable of receiving oral sex? | 3.87 | 4.89 | 8.17 | 8.19 | 112% |
| how often did you attempt to receive oral sex? | 1.97 | 2.31 | 5.98 | 6.00 | 205% |
| when you attempted to receive oral sex, how often were you satisfied with your erection? | 3.17 | 3.92 | 7.45 | 7.88 | 149% |
| how much have you enjoyed receiving oral sex? | 3.52 | 3.80 | 7.52 | 7.78 | 121% |
| when you attempted to receive oral sex, how often were you able to achieve an erection? | 3.62 | 4.05 | 7.80 | 8.24 | 128% |
| when you were able to achieve an erection to receive oral sex, how long were you able to maintain your erection? | 2.45 | 3.34 | 7.18 | 7.42 | 203% |
| when you were able to achieve an erection to receive oral sex, how often were you able to maintain your erection to completion, i.e. to the point of ejaculation/climax/orgasm? | 3.12 | 3.82 | 7.65 | 8.03 | 158% |
| how much have you felt the desire to have sexual intercourse? | 4.35 | 4.82 | 7.92 | 8.15 | 87% |
| how often have you felt capable of having sexual intercourse? | 3.75 | 4.95 | 8.08 | 8.42 | 125% |
| how often did you attempt sexual intercourse? | 2.23 | 2.85 | 6.28 | 6.25 | 180% |
| when you attempted sexual intercourse, how often were you satisfied with your erections? | 3.28 | 4.57 | 7.68 | 7.83 | 138% |
| how much have you enjoyed sexual intercourse? | 3.78 | 4.51 | 7.70 | 7.85 | 107% |
| when you attempted sexual intercourse, how often were you able to achieve an erection? | 3.97 | 4.77 | 7.85 | 7.98 | 101% |
| when you attempted sexual intercourse, how often were you able to penetrate (enter) your partner? | 3.88 | 4.89 | 8.60 | 8.85 | 128% |
| during sexual intercourse, how often were you able to maintain your erection after you had penetrated (entered) your partner? | 3.68 | 4.79 | 8.43 | 8.69 | 136% |
| when you were able to achieve an erection for sexual intercourse, how long were you able to maintain your erection? | 3.00 | 3.95 | 7.22 | 7.39 | 146% |
| when you were able to achieve an erection for sexual intercourse, how often were you able to maintain your erection to completion, i.e. to the point of ejaculation/climax/orgasm? | 3.62 | 4.74 | 7.67 | 8.00 | 121% |
| how would you rate your confidence in your ability to perform sexually? | 2.58 | 3.52 | 6.65 | 6.93 | 168% |
| how would you rate your confidence that you have pleased your partner sexually? | 2.82 | 3.48 | 7.40 | 7.76 | 176% |
| how would you rate your intimacy levels? | 2.68 | 3.21 | 6.68 | 6.80 | 153% |
| how would you rate the overall quality of your relationship? | 3.52 | 3.77 | 7.10 | 7.07 | 101% |
| how would you rate the communication between you and your partner? | 3.55 | 3.67 | 6.97 | 7.00 | 97% |
| how would you rate your level of output for household chores and/or errands? | 3.62 | 3.43 | 6.47 | 6.54 | 81% |
| how would you rate the level of closeness in your relationship? | 3.35 | 3.48 | 6.95 | 6.85 | 104% |
| how would you rate your level of self-esteem? | 3.10 | 3.39 | 6.57 | 6.80 | 119% |
| how would you rate your overall confidence? | 3.17 | 3.36 | 6.55 | 6.78 | 114% |
| how would you describe your overall mood? | 3.43 | 3.34 | 6.63 | 6.76 | 97% |
| how would you describe your overall quality of life? | 3.72 | 3.56 | 7.73 | 7.56 | 103% |
| how would you describe your level of physical activity? | 3.10 | 3.03 | 6.23 | 6.36 | 105% |
| how would you describe your level of hopefulness for your future sex life? | 3.52 | 3.80 | 6.83 | 7.00 | 99% |
| how would you rate your partner's sexual interest in you? | 3.12 | 3.41 | 6.67 | 6.76 | 117% |
| **Overall Avg** | **3.32** | **3.89** | **6.83** | **7.02** | **111%** |

## Claims

1. A method of selecting a penile constriction device (100) for an individual, the method comprising:
a. providing a measurement device having color coded demarcations, each demarcation indicating a size of the erectile device;
b. measuring the individual's penis using the measurement device; and
c. selecting the penile constrictive device (100) based on the demarcation.

2. A measurement device for sizing a penile constriction device (1) comprising color-coded demarcations, each demarcation indicating a size of the penile constriction device (100).

## Patentansprüche

1. Verfahren zur Auswahl einer Peniskonstriktionsvorrichtung (100) für ein Individuum, wobei das Verfahren Folgendes umfasst:
a. Bereitstellen einer Messvorrichtung mit farbcodierten Markierungen, wobei jede Markierung eine Größe der Erektionsvorrichtung anzeigt;
b. Messen des Penis des Individuums unter Verwendung der Messvorrichtung;
und
c. Auswählen der Peniskonstriktionsvorrichtung (100) beruhend auf der Markierung.

2. Messvorrichtung zum Bemessen einer Peniskonstriktionsvorrichtung (1), umfassend farbcodierte Markierungen, wobei jede Markierung eine Größe der Peniskonstriktionsvorrichtung (100) anzeigt.

## Revendications

1. Procédé de sélection d'un dispositif de constriction pénienne (100) pour un individu, le procédé comprenant les étapes consistant à :
a. fournir un dispositif de mesure présentant des démarcations codées par couleur, chaque démarcation indiquant une taille du dispositif érectile ;
b. mesurer le pénis de l'individu à l'aide du dispositif de mesure ; et
c. sélectionner le dispositif constrictif pénien (100) sur la base de la démarcation.

2. Dispositif de mesure pour dimensionner un dispositif de constriction pénienne (1) comprenant des démarcations codées par couleur, chaque démarcation indiquant une taille du dispositif de constriction pénienne (100).
